# EUROPÄISCHE PATENTANMELDUNG

(11) **EP 3 896 065 A1**
(43) Veröffentlichungstag der Anmeldung: **20.10.2021**
(21) Anmeldenummer: 21171968.7
(22) Anmeldetag: 04.08.2016
(51) Int. Cl.: C07D 401/14, C07D 401/04, C07D 235/18, C07D 403/10, C07D 409/10, C07D 409/14, A61K 31/4439, A01N 43/52, A01N 43/653, C07D 471/04, C07D 487/04, C07D 498/04

(54) **2-(HET)ARYL-SUBSTITUIERTE KONDENSIERTE HETEROCYCLEN-DERIVATE ALS SCHÄDLINGSBEKÄMPFUNGSMITTEL**

(30) Priorität: 07.08.2015 EP 15180149
(62) Teilanmeldung aus: 16750152.7
(71) Anmelder: Bayer CropScience Aktiengesellschaft, 40789 Monheim am Rhein (DE)
(72) Erfinder: FISCHER, Rüdiger, 50259 Pulheim (DE); WILCKE, David, 40237 Düsseldorf (DE); KAUSCH-BUSIES, Nina, 40227 Düsseldorf (DE); HAGER, Dominik, 40789 Monheim (DE); ILG, Kerstin, 50670 Köln (DE); HOFFMEISTER, Laura, 40593 Düsseldorf (DE); WILLOT, Matthieu, 40215 Düsseldorf (DE); PORTZ, Daniela, 52391 Vettweiß (DE); GÖRGENS, Ulrich, 40882 Ratingen (DE); TURBERG, Andreas, 42781 Haan (DE)
(74) Vertreter: BIP Patents

(57) **Zusammenfassung**

Die Erfindung betrifft neue Verbindungen der Formel (I), 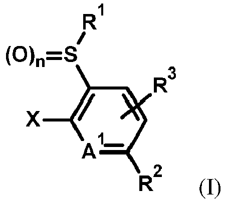 in welcher R¹, R², R³, A¹, X und n die oben genannten Bedeutungen haben,
deren Anwendung als Akarizide und/oder Insektizide zur Bekämpfung tierischer Schädlinge und Verfahren und Zwischenprodukte zu ihrer Herstellung.

## Beschreibung

Die vorliegende Erfindung betrifft neue 2-(Het)Aryl-substituierte kondensierte Heterocyclen-Derivate der Formel (I), deren Anwendung als Akarizide und/oder Insektizide zur Bekämpfung tierischer Schädlinge, vor allem von Arthropoden und insbesondere von Insekten und Spinnentieren und Verfahren und Zwischenprodukte zu ihrer Herstellung.

Kondensierte Heterocyclen-Derivate mit insektiziden Eigenschaften sind in der Literatur bereits beschrieben, z.B. in WO 2010/125985, WO 2012/074135, WO 2012/086848, WO 2013/018928, WO 2013/191113, WO 2014/142292, WO 2014/148451, WO 2015/000715, EP 15153943.4, EP 15153948.3, WO 2015/121136, WO 2015/133603, WO 2015/198859, WO 2015/002211,WO 2015/071180, WO 2015/091945, WO 2016/005263, WO 2015/198817, WO 2016/041819, WO 2016/039441, WO 2016/026848, WO 2016/023954, WO 2016/020286, WO 2016/046071.

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue 2-(Het)Aryl-substituierte kondensierte Heterocyclen-Derivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die 2-(Het)Aryl-substituierten kondensierten Heterocyclen-Derivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Verbindungen der Formel (I) 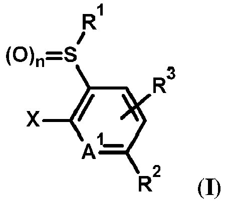 in welcher (Ausgestaltung 1-1)
- A¹: für Stickstoff, =N⁺-O⁻ oder =C-R⁴ steht,
- R¹: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Amino, (C₁-C₆)Alkylamino, Di-(C₁-C₆)alkyl-amino, (C₃-C₈)Cycloalkylamino, (C₁-C₆)Alkylcarbonyl-amino, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₁-C₆)Alkylsulfonylamino, Aminosulfonyl-(C₁-C₆)alkyl, (C₁-C₆)Alkylaminosulfonyl-(C₁-C₆)alkyl, Di-(C₁-C₆)alkyl-aminosulfonyl-(C₁-C₆)alkyl,
oder für jeweils gegebenenfalls einfach oder mehrfach, gleich oder verschieden durch Aryl, Hetaryl oder Heterocyclyl substituiertes (C₁-C₆)Alkyl, (C₁-C₆)Alkoxy, (C₂-C₆)Alkenyl, (C₂-C₆)Alkinyl, (C₃-C₈)Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl oder Benzyl substituiert sein können, oder
- R¹: für jeweils gegebenenfalls einfach oder mehrfach, gleich oder verschieden durch Halogen, Cyano, Nitro, Hydroxy, Amino, Carboxy, Carbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl, (=O) (nur im Fall von Heterocyclyl) oder (=O)₂ (nur im Fall von Heterocyclyl) substituiertes Aryl, Hetaryl oder Heterocyclyl steht,
- R²: für einen gegebenenfalls einfach oder mehrfach, gleich oder verschieden substituierten gesättigten, teilgesättigten oder heteroaromatischen Ring, in dem mindestens ein C-Atom durch ein Heteroatom ersetzt ist, steht, wobei jeweils gegebenenfalls mindestens eine Carbonylgruppe enthalten sein kann und/oder wobei als Substituenten jeweils in Frage kommen: Wasserstoff, Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
- R²: weiterhin für eine Gruppe steht, ausgewählt aus -C(=O)-R⁸ (G1), -C(=S)-R⁸ (G2), -C(=O)-NR¹¹R¹² (G3), -C(=S)-NR¹¹R¹² (G4), -S(O)ₘ-R¹³ (G5), -S=O(=NH)-R¹³ (G6), -S=O(=N-CN)-R¹³ (G7), -S(=N-CN)-R¹³ (G8), -S(O)₂-NR¹¹R¹² (G9), -NR¹¹R¹² (G10), -NR¹¹-NR¹¹R¹² (G11), -NR¹¹-C(=O)-R⁸ (G12), -NR¹¹-C(=S)-R⁸ (G13), -NR¹¹-S(O)₂-R¹³ (G14), -N(R¹¹)-O-R¹³ (G15), -N=S(=O)ₚ-R¹⁴R¹⁵ (G16), -O-R¹³ (G17) oder -CR¹¹=N-OR¹³ (G18), wobei wenn R² für G5, G6, G7, G8, G9 steht, dann steht n für 2,
- R³: für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁**-**C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino),
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁**-**C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
- R⁴: für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁**-**C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino,-NHCO-(C₁-C₆)alkyl ((C₁-C₆)Alkylcarbonylamino),
- X: für ein teilweise gesättigtes oder gesättigtes heterozyklisches oder heteroaromatisches 8-, 9-, 10-, 11- oder 12-gliedriges annelliertes bicyclisches oder tricyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert ist, und wobei die Substituenten unabhängig voneinander ausgewählt sein können aus Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Alkinyloxy-(C₁-C₄)alkyl, (C₂-C₆)Halogenalkinyl, (C₂-C₆)Cyanoalkinyl, (C₁-C₆)Alkoxy, (C₁-C₆)Halogenalkoxy, (C₁-C₆)Halogenalkoxy-(C₁-C₆)alkyl, (C₂-C₆)Alkenyloxy-(C₁**-**C₆)alkyl, (C₂-C₆)Halogenalkenyloxy-(C₁-C₆)alkyl, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁**-**C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylcarbonyl-(C₁-C₆)alkyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino),
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
wobei X nicht für 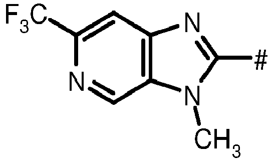 steht,
- R⁸: für Wasserstoff oder für jeweils gegebenenfalls einfach oder mehrfach, gleich oder verschieden substituiertes C₁-C₆-Alkyl, C₂-C₆-Alkenyl, C₂-C₆-Alkinyl, C₁-C₄-Alkoxy, C₃-C₁₂-Cycloalkyl, C₃-C₁₂-Cycloalkyl-C₁-C₆-Alkyl oder C₄-C₁₂-Bicycloalkyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-Alkyl, C₁-C₄-Alkylsulfimino-C₂-C₅-Alkylcarbonyl, C₁-C₄-Alkylsulfoximino, C₁-C₄-Alkylsulfoximino-C₁-C₄-alkyl, C₁-C₄-Alkylsulfoximino-C₂-C₅-Alkylcarbonyl, C₂-C₆-Alkoxycarbonyl, C₂-C₆-Alkylcarbonyl, C₃-C₆-Trialkylsilyl, Amino, C₁-C₄-Alkylamino, Di-(C₁-C₄-alkyl)amino, C₃-C₆-Cycloalkylamino, einem Phenylring oder einem 3- bis 6-gliedrigenaromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino, oder
- R⁸: für einen Phenylring oder einen 3- bis 6-gliedrigen aromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino, oder
- R¹³, R¹⁴, R¹⁵: unabhängig voneinander für jeweils gegebenenfalls einfach oder mehrfach, gleich oder verschieden substituiertes C₁-C₆-Alkyl, C₂-C₆-Alkenyl, C₂-C₆-Alkinyl, C₃-C₁₂-Cycloalkyl, C₃-C₁₂-Cycloalkyl-C₁-C₆-Alkyl oder C₄-C₁₂-Bicycloalkyl stehen, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino- C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl, C₃-C₆-Trialkylsilyl, Amino, C₁-C₄-Alkylamino, Di-(C₁-C₄-Alkyl)amino, C₃-C₆-Cycloalkylamino, einem Phenylring oder einem 3- oder 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenaₗkyl, C₂-C₆-Halogenalkenyl, C₂-C₆-Halogenalkinyl, C₃-C₆-Halogencycloalkyl, Halogen, Cyano, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
- R¹³, R¹⁴, R¹⁵: unabhängig voneinander für einen Phenylring oder einen 3- bis 6-gliedrigen aromatischen, teilgesättigten oder gesättigten Heterocyclus stehen, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogen-alkyl, C₂-C₆-Halogenalkenyl, C₂-C₆-Halogenalkinyl, C₃-C₆-Halogencycloalkyl, Halogen, Cyano, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
- R¹¹, R¹²: unabhängig voneinander für Wasserstoff oder für R¹³ stehen,
- m: für 0, 1 oder 2 steht,
- n: für 0, 1 oder 2 steht,
- p: für 0 oder 1 steht,
ausgenommen Verbindungen der Formel (I), in denen
X für 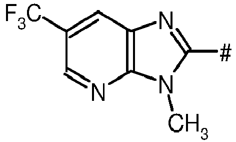 steht
und A¹ für N oder CH steht, R¹ für Ethyl steht, R² für S-Methyl steht, R³ für Wasserstoff steht und n für 0 steht
oder A¹ für N oder CH steht, R¹ für Ethyl steht, R² für O-Methyl steht, R³ für Wasserstoff steht und n für 0, 1 oder 2 steht.

### Ausgestaltung 1-2:

A¹, R¹, R³, R⁴, X, R¹¹, R¹², R¹³, R¹⁴, R¹⁵, m, n und p haben die in Ausgestaltung 1-1 angegebenen Bedeutungen und
R² steht für einen gegebenenfalls einfach oder mehrfach, gleich oder verschieden substituierten gesättigten heterocylcischen, teilgesättigten heterocyclischen oder heteroaromatischen Ring, in dem mindestens ein C-Atom durch ein Heteroatom ersetzt ist, steht, wobei jeweils gegebenenfalls mindestens eine Carbonylgruppe enthalten sein kann und/oder wobei als Substituenten jeweils in Frage kommen: Wasserstoff, Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
R² steht weiterhin für eine Gruppe, ausgewählt aus -C(=O)-R⁸ (G1), -C(=S)-R⁸ (G2), -C(=O)-NR¹¹R¹² (G3), -C(=S)-NR¹¹R¹² (G4), -S(O)ₘ-R¹³ (G5), -S=O(=NH)-R¹³ (G6), -S=O(=N-CN)-R¹³ (G7), -S(=N-CN)-R¹³ (G8), -S(O)₂-NR¹¹R¹² (G9), -NR¹¹R¹² (G10), -NR¹¹-NR¹¹R¹² (G11), -NR¹¹-C(=O)-R⁸ (G12), -NR¹¹-C(=S)-R⁸ (G13), -NR¹¹-S(O)₂-R¹³ (G14), -N(R¹¹)-O-R¹³ (G15), -N=S(=O)ₚ-R¹⁴R¹⁵ (G16), -O-R¹³ (G17), -CR¹¹=N-OR¹³ (G18) oder -CR¹¹=CR⁸R¹²G(19), wobei wenn R² für G5, G6, G7, G8, G9 steht, dann steht n für 2,
R⁸ steht für Wasserstoff, oder für jeweils gegebenenfalls einfach oder mehrfach, gleich oder verschieden substituiertes Amino, C₁-C₆-Alkyl, C₂-C₆-Alkenyl, C₂-C₆-Alkinyl, C₁-C₄-Alkoxy, C₃-C₁₂-Cycloalkyl, C₃-C₁₂-Cycloalkyl-C₁-C₆-Alkyl oder C₄-C₁₂-Bicycloalkyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-Alkyl, C₁-C₄-Alkylsulfimino-C₂-C₅-Alkylcarbonyl, C₁-C₄-Alkylsulfoximino, C₁-C₄-Alkylsulfoximino-C₁-C₄-alkyl, C₁-C₄-Alkylsulfoximino-C₂-C₅-Alkylcarbonyl, C₂-C₆-Alkoxycarbonyl, C₂-C₆-Alkylcarbonyl, C₃-C₆-Trialkylsilyl, Amino, C₁-C₄-Alkylamino, Di-(C₁-C₄-alkyl)amino, C₃-C₆-Cycloalkylamino, einem Phenylring oder einem 3- bis 6-gliedrigenaromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino, oder
R⁸ für einen Phenylring oder einen 3- bis 6-gliedrigen aromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
wobei wenn R² für gegebenenfalls substituiertes Q45 steht, dann steht X nicht für H1, H2 oder H4 und wenn R² für G10, G11, G12, G13, G14, G15 oder G16 steht und A¹ für N steht, dann steht X nicht für H1, H2, H4, H5, H7, H8 oder H14,
ausgenommen Verbindungen der Formel (I), in denen
X für 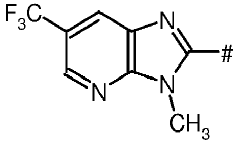 steht
und A¹ für N oder CH steht, R¹ für Ethyl steht, R² für S-Methyl steht, R³ für Wasserstoff steht und n für 0 steht
oder A¹ für N oder CH steht, R¹ für Ethyl steht, R² für O-Methyl steht, R³ für Wasserstoff steht und n für 0, 1 oder 2 steht,
ausgenommen folgende Verbindung:

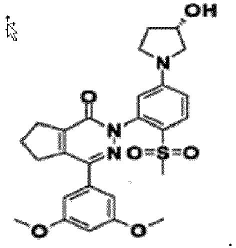

Weiterhin wurde gefunden, dass die Verbindungen der Formel (I) eine sehr gute Wirksamkeit als Schädlingsbekämpfungsmittel, vorzugsweise als Insektizide und/oder Akarizide aufweisen, darüber hinaus in der Regel insbesondere gegenüber Kulturpflanzen sehr gut pflanzenverträglich sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:

### Ausgestaltung 2-1:

- A¹: steht bevorzugt für Stickstoff, =N⁺-O⁻ oder =C-R⁴,
- R¹: steht bevorzugt für (C₁-C₄)Alkyl, (C₁-C₄)Hydroxyalkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyloxy-(C₁-C₄)alkyl, (C₂-C₄)Halogenalkinyl, (C₂-C₄)Cyanoalkinyl, (C₃-C₆)Cycloalkyl, (C₃-C₆)Cycloalkyl(C₃-C₆)cycloalkyl, (C₁-C₄)Alkyl-(C₃-C₆)cycloalkyl, Halogen(C₃-C₆)cycloalkyl, (C₁-C₄)Alkylamino, Di-(C₁-C₄)alkyl-amino, (C₃-C₆)Cycloalkylamino, (C₁-C₄)Alkylcarbonyl-amino, (C₁-C₄)Alkylthio-(C₁**-**C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Alkylcarbonyl-(C₁-C₄)alkyl, (C₁-C₄)Halogenalkylcarbonyl-(C₁-C₄)alkyl, (C₁-C₄)Alkylsulfonylamino,
oder für jeweils gegebenenfalls einfach oder zweifach, gleich oder verschieden durch Aryl, Hetaryl oder Heterocyclyl substituiertes (C₁-C₄)Alkyl, (C₁-C₄)Alkoxy, (C₂-C₄)Alkenyl, (C₂-C₄)Alkinyl, (C₃-C₆)Cycloalkyl, wobei Aryl, Hetaryl oder Heterocyclyl jeweils gegebenenfalls einfach oder zweifach gleich oder verschieden durch Halogen, Cyano, Carbamoyl, Aminosulfonyl, (C₁-C₄)Alkyl, (C₃-C₄)Cycloalkyl, (C₁-C₄)Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Alkylsulfimino substituiert sein können, oder
- R¹: steht bevorzugt für jeweils gegebenenfalls einfach oder zweifach, gleich oder verschieden durch Halogen, Cyano, Carbamoyl, (C₁-C₄)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Alkylsulfimino, (C₁-C₄)Alkylsulfoximino, (C₁-C₄)Alkylcarbonyl, (C₃-C₄)Trialkylsilyl, (=O) (nur im Fall von Heterocyclyl) oder (=O)₂ (nur im Fall von Heterocyclyl) substituiertes Aryl, Hetaryl oder Heterocyclyl,
- R²: steht bevorzugt für einen gegebenenfalls einfach oder zweifach, gleich oder verschieden substituierten gesättigten, teilgesättigten oder heteroaromatischen Ring, in dem mindestens ein C-Atom durch ein Heteroatom aus der Gruppe N, O oder S ersetzt ist, wobei jeweils gegebenenfalls mindestens eine Carbonylgruppe enthalten sein kann und/oder wobei als Substituenten jeweils in Frage kommen: Wasserstoff, Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkoxyimino, -N=C(H)-O(C₁-C₄)Alkyl, -C(H)=N-O(C₁-C₄)Alkyl, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbonylamino,
- R²: steht weiterhin bevorzugt für eine Gruppe, ausgewählt aus -C(=O)-R⁸ (G1), -C(=S)-R⁸ (G2),-C(=O)-NR¹¹R¹² (G3), -C(=S)-NR¹¹R¹² (G4), -S=O(=NH)-R¹³ (G6), -S=O(=N-CN)-R¹³ (G7), -S(=N-CN)-R¹³ (G8), -S(O)₂-NR¹¹R¹² (G9), -NR¹¹R¹² (G10), -NR¹¹-NR¹¹R¹² (G11), -NR¹¹-C(=O)-R⁸ (G12), -NR¹¹-C(=S)-R⁸ (G13), -NR¹¹-S(O)₂-R¹³ (G14), -N(R¹¹)-O-R¹³ (G15), -N=S(=O)ₚ-R¹⁴R¹⁵ (G16) oder -CR¹¹=N-OR¹³ (G18), wobei wenn R² für G6, G7, G8, G9 steht, dann steht n für 2,
- R³: steht bevorzugt für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino),
weiterhin bevorzugt für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und/oder wobei als Substituenten jeweils in Frage kommen: Cyano, Halogen, Nitro, Acetyl, Amino, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl((C₁-C₄)Alkylcarbonylamino),
- R⁴: steht bevorzugt für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino),
- X: steht bevorzugt für ein heteroaromatisches 8-, 9-, 10-, 11- oder 12-gliedriges annelliertes bicyclisches oder tricyclisches Ringsystem, wobei das Ringsystem gegebenenfalls einfach oder mehrfach, gleich oder verschieden substituiert ist, und wobei die Substituenten unabhängig voneinander ausgewählt sein können aus Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Alkinyloxy-(C₁-C₄)alkyl, (C₂-C₆)Halogenalkinyl, (C₁-C₆)Alkoxy, (C₁-C₆)Halogenalkoxy, (C₁-C₆)Halogenalkoxy-(C₁-C₆)alkyl, (C₂-C₆)Alkenyloxy-(C₁-C₆)alkyl, (C₂-C₆)Halogenalkenyloxy-(C₁-C₆)alkyl, (C₁-C₆)Cyanoalkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylcarbonyl-(C₁-C₆)alkyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl((C₁-C₆)Alkylcarbonylamino),
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, C₁-C₄-Alkoxy, C₁-C₄-Haloalkoxy substituiert sein können,
wobei X nicht für 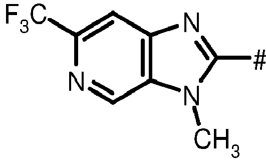 steht,
- R⁸: steht bevorzugt für Wasserstoff, für jeweils gegebenenfalls einfach oder mehrfach, gleich oder verschieden substituiertes C₁-C₄-Alkyl, C₃-C₆-Cycloalkyl, C₃-C₆-Cycloalkyl-C₁-C₄-alkyl, C₁-C₄-Alkoxy oder C₁-C₄-Alkoxy-C₁-C₄-alkyl, wobei die Substituenten unabhängig voneinander ausgewählt sein können aus Halogen, Cyano, einem Phenylring oder einem 3- bis 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen, Cyano, NO₂, C₁-C₄-Alkoxy oder C₁-C₄-Halogenalkoxy, oder
- R⁸: steht bevorzugt für einen Phenylring oder einen 3- bis 6-gliedrigenaromatischen, teilgesättigten oder gesättigten Heterocyclus, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹³, R¹⁴, R¹⁵: stehen unabhängig voneinander bevorzugt für jeweils gegebenenfalls einfach oder mehrfach, gleich oder verschieden substituiertes C₁-C₄-Alkyl oder C₃-C₆-Cycloalkyl, wobei die Substituenten unabhängig voneinander ausgewählt sein können aus Halogen, Cyano, einem Phenylring oder einem 3- bis 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen, Cyano, NO₂, C₁-C₄-Alkoxy oder C₁-C₄-Halogenalkoxy, oder
- R¹³, R¹⁴, R¹⁵: stehen unabhängig voneinader bevorzugt für einen Phenylring oder einen 3- bis 6-gliedrigen aromatischen, teilgesättigten oder gesättigten Heterocyclus, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹¹, R¹²: stehen unabhängig voneinander bevorzugt für Wasserstoff oder für R¹³,
- n: seht bevorzugt für 0, 1 oder 2,
- p: steht bevorzugt für 0 oder 1.

### Ausgestaltung 2-2:

A¹, R¹, R³, R⁴, X, R¹¹, R¹², R¹³, R¹⁴, R¹⁵, n und p haben die in Ausgestaltung 2-1 angegebenen Bedeutungen und
R² steht bevorzugt für einen gegebenenfalls einfach oder zweifach, gleich oder verschieden substituierten gesättigten heterocyclischen, teilgesättigten heterocyclischen oder heteroaromatischen Ring, in dem mindestens ein C-Atom durch ein Heteroatom aus der Gruppe N, O oder S ersetzt ist, wobei jeweils gegebenenfalls mindestens eine Carbonylgruppe enthalten sein kann und/oder wobei als Substituenten jeweils in Frage kommen: Wasserstoff, Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkoxyimino, -N=C(H)-O(C₁-C₄)Alkyl, -C(H)=N-O(C₁-C₄)Alkyl, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbonylamino,
R² steht weiterhin bevorzugt für eine Gruppe, ausgewählt aus -C(=O)-R⁸ (G1), -C(=S)-R⁸ (G2), -C(=O)-NR¹¹R¹² (G3), -C(=S)-NR¹¹R¹² (G4), -S=O(=NH)-R¹³ (G6), -S=O(=N-CN)-R¹³ (G7), -S(=N-CN)-R¹³ (G8), -S(O)₂-NR¹¹R¹² (G9), -NR¹¹R¹² (G10), -NR¹¹-NR¹¹R¹² (G11), -NR¹¹-C(=O)-R⁸ (G12), -NR¹¹-C(=S)-R⁸ (G13), -NR¹¹-S(O)₂-R¹³ (G14), -N(R¹¹)-O-R¹³ (G15), -N=S(=O)ₚ-R¹⁴R¹⁵ (G16), -CR¹¹=N-OR¹³ (G18) oder -CR¹¹=CR⁸R¹²G(19), wobei wenn R² für G6, G7, G8, G9 steht, dann steht n für 2,
R⁸ steht bevorzugt für Wasserstoff, Amino, für jeweils gegebenenfalls einfach oder mehrfach, gleich oder verschieden substituiertes C₁-C₄-Alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, C₁-C₄-Alkylamino, C₃-C₆-Cycloalkyl, C₃-C₆-Cycloalkyl-C₁-C₄-alkyl, C₁-C₄-Alkoxy oder C₁-C₄-Alkoxy-C₁-C₄-alkyl, wobei die Substituenten unabhängig voneinander ausgewählt sein können aus Halogen, Cyano, einem Phenylring oder einem 3- bis 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen, Cyano, NO₂, C₁-C₄-Alkoxy oder C₁-C₄-Halogenalkoxy, oder
R⁸ steht bevorzugt für einen Phenylring oder einen 3- bis 6-gliedrigenaromatischen, teilgesättigten oder gesättigten Heterocyclus, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
wobei wenn R² für gegebenenfalls substituiertes Q45 steht, dann steht X nicht für H1, H2 oder H4 und wenn R² für G10, G11, G12, G13, G14, G15 oder G 16 steht und A¹ für N steht, dann steht X nicht für H1, H2, H4, H5, H7, H8 oder H14,
ausgenommen folgende Verbindung:

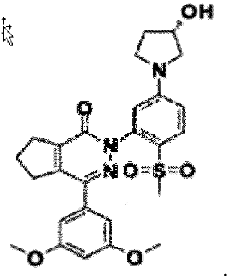

### Ausgestaltung 3-1:

- A¹: steht besonders bevorzugt für Stickstoff oder =C-R⁴,
- R¹: steht besonders bevorzugt für (C₁-C₄)Alkyl, (C₁-C₄)Hydroxyalkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl,
- R²: steht besonders bevorzugt für einen gegebenenfalls einfach oder zweifach, gleich oder verschieden substituierten 5- oder 6-gliedrigen gesättigten, teilgesättigten oder heteroaromatischen Ring, aus der Reihe Q1 bis Q99, wobei jeweils gegebenfalls mindestens eine Carbonylgruppe enthalten sein kann und/oder wobei als Substituenten jeweils in Frage kommen: Wasserstoff, Cyano, (C₁-C₄)Cyanoalkyl, (C₁-C₄)Alkoxy-(C₁-C₄)alkyl, Halogen, Amino, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, -C(H)=N-O(C₁-C₄)Alkyl, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbonylamino, 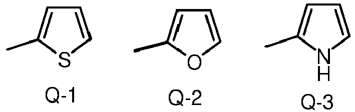 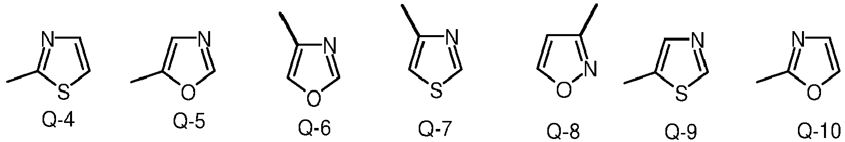 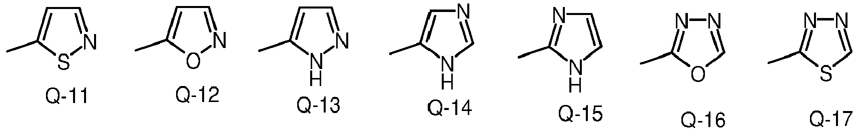 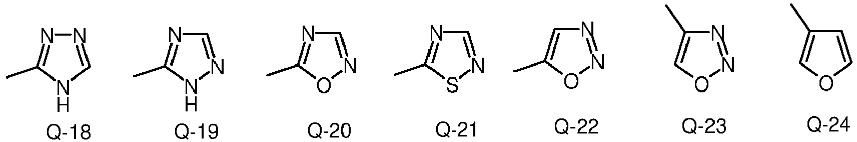 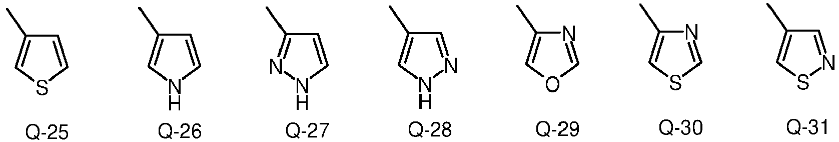 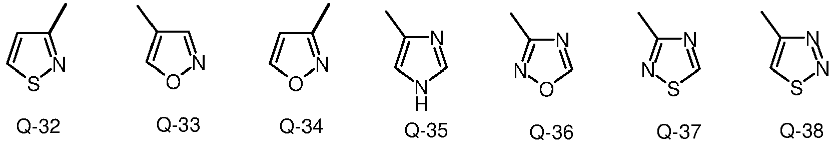 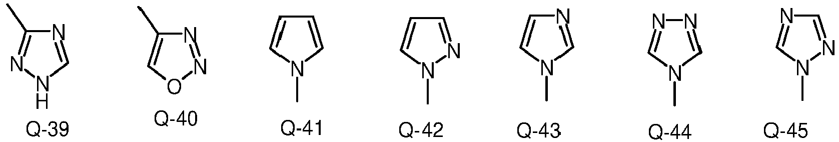 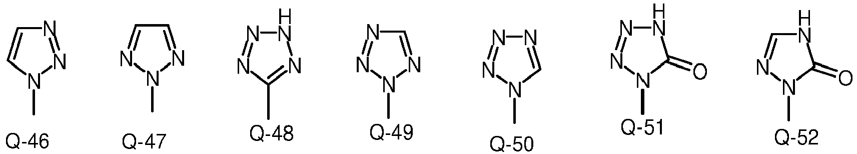 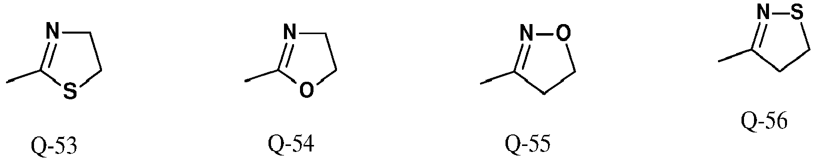 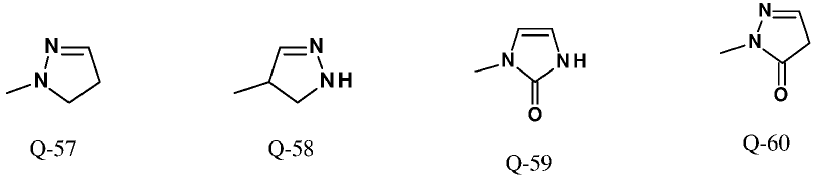 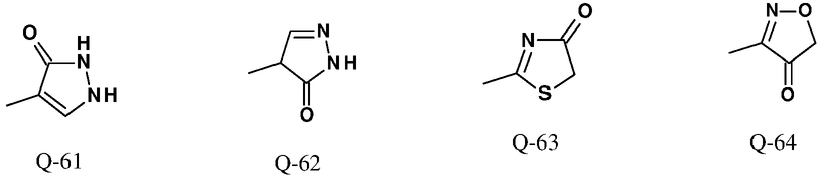 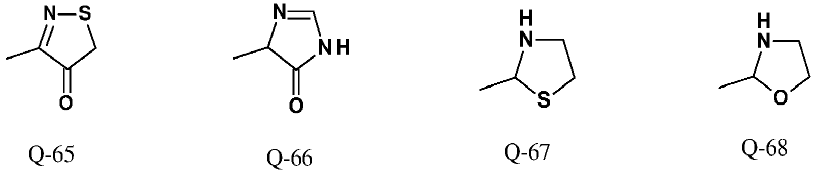 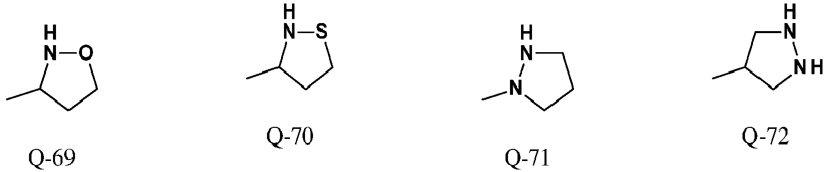 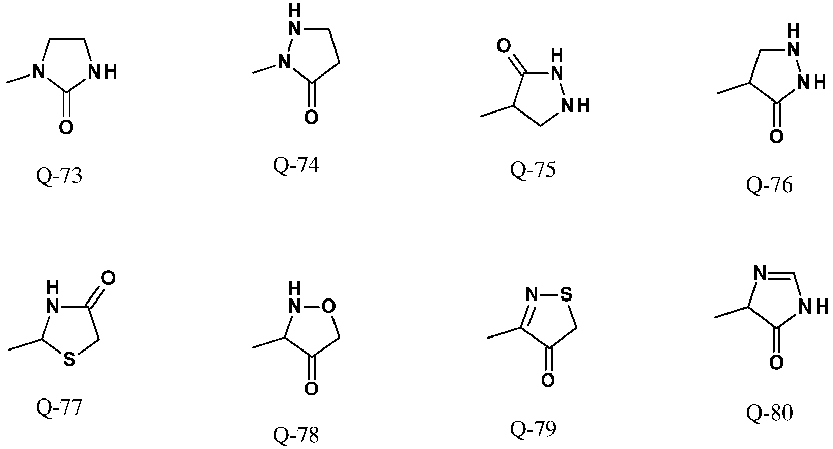 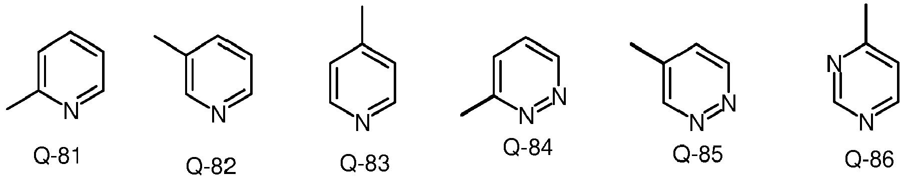 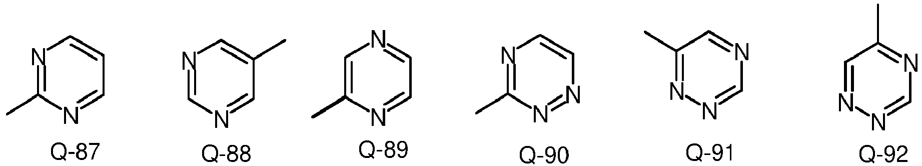 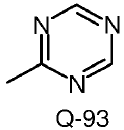 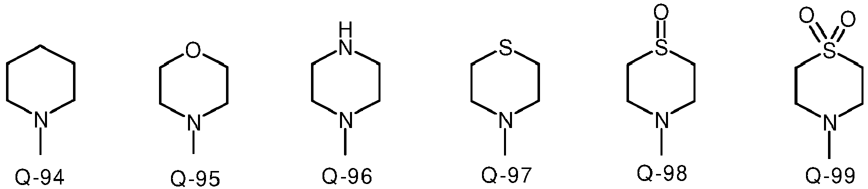
- R²: steht weiterhin besonders bevorzugt für eine Gruppe ausgewählt aus G1, G2, G3, G4, G6, G7, G8, G9, G10, G11, G12, G13, G14, G15, G16 oder G18, wobei wenn R² für G6, G7, G8, G9 steht, dann steht n für 2,
- R³: steht besonders bevorzugt für Wasserstoff, Cyano, Halogen, Nitro, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, NHCO-(C₁-C₄)alkyl ((C₁-C₄)Alkylcarbonylamino),
weiterhin besonders bevorzugt für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und/oder wobei als Substituenten jeweils in Frage kommen: Cyano, Halogen, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
- R⁴: steht besonders bevorzugt für Wasserstoff, Halogen, Cyano oder (C₁-C₄)Alkyl,
- X: steht besonders bevorzugt für ein heteroaromatisches 9-gliedriges oder 12-gliedriges annelliertes bicyclisches oder tricyclisches Ringsystem aus der Reihe H1 bis H19, 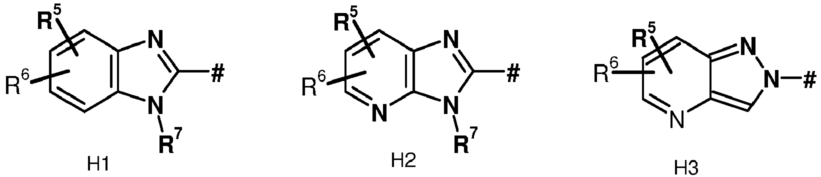 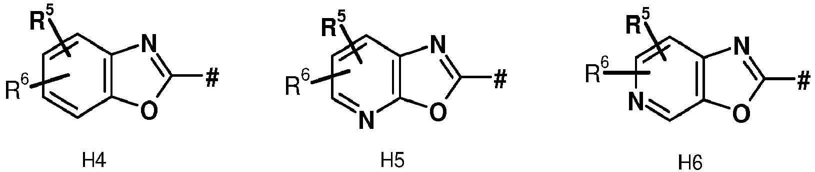 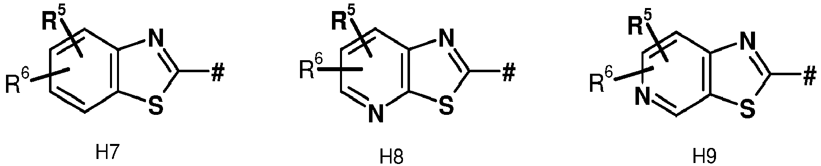 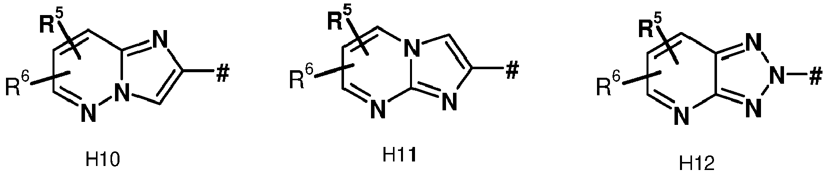 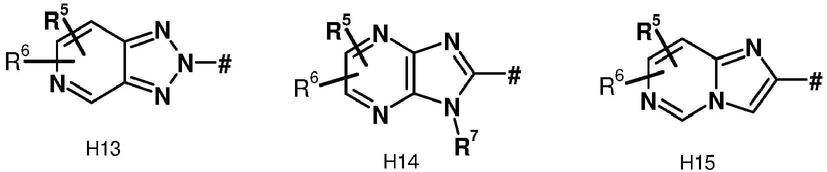 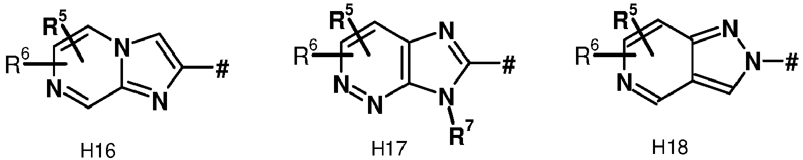 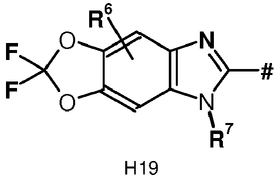
- R⁵, R⁶: stehen unabhängig voneinander besonders bevorzugt für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl,
- R⁷: steht besonders bevorzugt für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
- R⁸: steht besonders bevorzugt für Wasserstoff, (C₁-C₄)Alkoxy-(C₁-C₄)alkyl, (C₃-C₆)Cycloalkyl oder (C₃-C₆)Cycloalkyl-(C₁-C₄)alkyl, für jeweils gegebenenfalls einfach durch Halogen, Cyano, Phenyl oder Pyridyl substituiertes (C₁-C₄)Alkyl oder (C₁-C₄)Alkoxy, wobei Phenyl bzw. Pyridyl jeweils gegebenenfalls einfach oder zweifach, gleich oder verschieden durch Trifluormethyl, Cyano, Fluor, Chlor, Brom oder Trifluormethoxy substituiert sein kann, oder
- R⁸: steht besonders bevorzugt für Phenyl, Pyridyl oder einen 3- bis 6-gliedrigen gesättigten Heterocyclus, enthaltend 1-2 Heteroatome aus der Reihe N,S,O, wobei Phenyl, Pyridyl bzw. der Heterocyclus jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹³, R¹⁴, R¹⁵: stehen unabhängig voneinander besonders bevorzugt für gegebenenfalls einfach durch Halogen, Cyano, Phenyl oder Pyridyl substituiertes (C₁-C₄)Alkyl, wobei Phenyl bzw. Pyridyl jeweils gegebenenfalls einfach oder zweifach, gleich oder verschieden durch Trifluormethyl, Cyano, Fluor, Chlor oder Trifluormethoxy substituiert sein kann, oder
- R¹³, R¹⁴, R¹⁵: stehen unabhängig voneinander besonders bevorzugt für Phenyl, Pyridyl, oder einen 3-bis 6-gliedrigen gesättigten Heterocyclus, enthaltend 1-2 Heteroatome aus der Reihe N,S,O, wobei Phenyl, Pyridyl, bzw. der Heterocyclus jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹¹, R¹²: stehen unabhängig voneinander besonders bevorzugt für Wasserstoff oder für R¹³,
- n: steht besonders bevorzugt für 0, 1 oder 2,
- p: steht besonders bevorzugt für 0 oder 1.

### Ausgestaltung 3-2:

A¹, R¹, R³, R⁴, R⁵, R⁶, R⁷, n und p haben die in Ausgestaltung 3-1 angegebenen Bedeutungen und
R² steht besonders bevorzugt für einen gegebenenfalls einfach oder zweifach, gleich oder verschieden substituierten 5- oder 6-gliedrigen gesättigten, teilgesättigten oder heteroaromatischen Ring, aus der Reihe Q1 bis Q101, wobei jeweils gegebenfalls mindestens eine Carbonylgruppe enthalten sein kann und/oder wobei als Substituenten jeweils in Frage kommen: Wasserstoff, Cyano, (C₁-C₄)Cyanoalkyl, (C₁-C₄)Alkoxy-(C₁-C₄)alkyl, Halogen, Amino, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, -C(H)=N-O(C₁-C₄)Alkyl, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Alkylsulfonyl-(C₁-C₄)alkyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbonylamino, 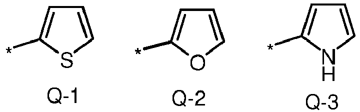 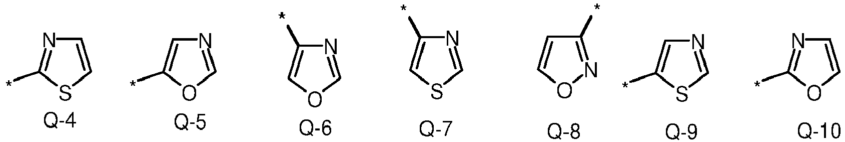 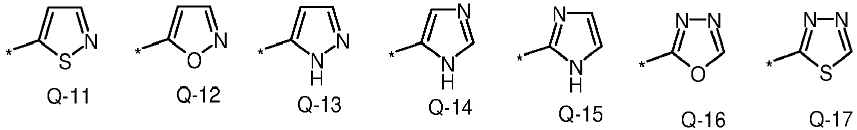 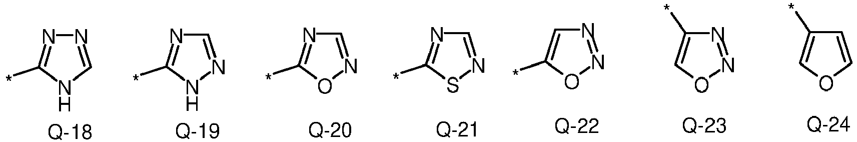 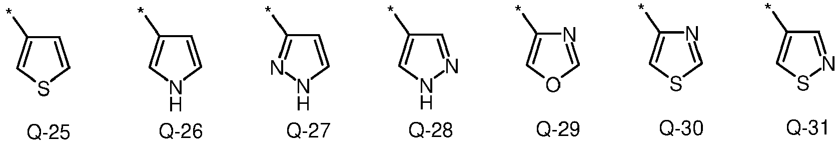 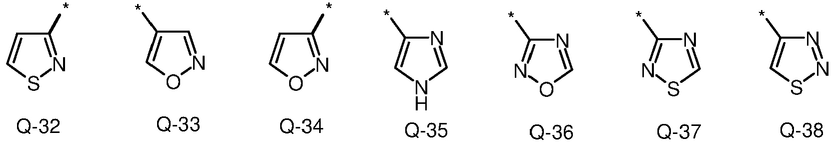 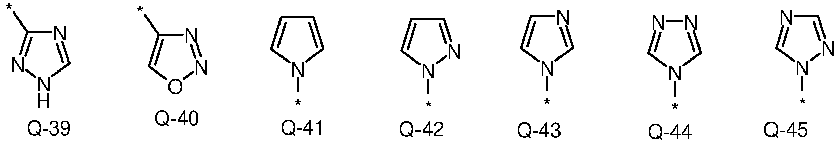 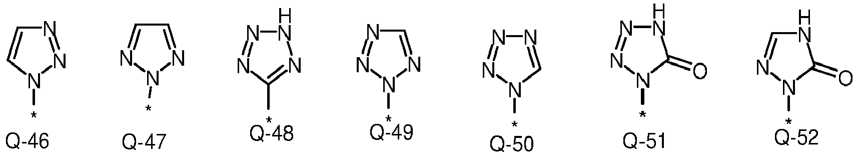 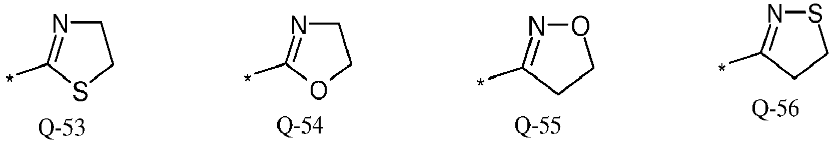 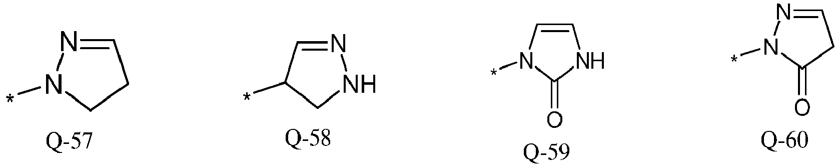 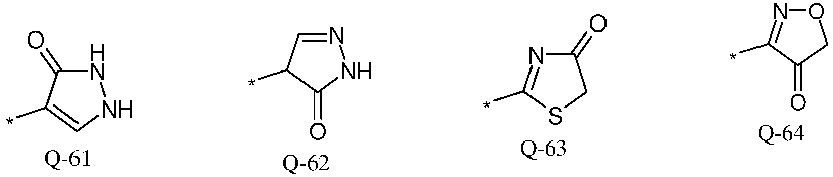 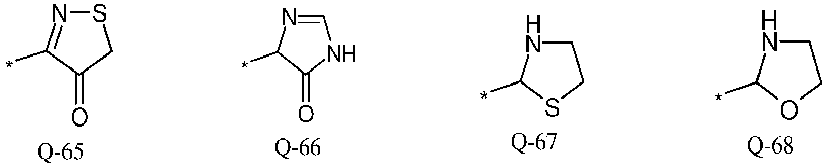 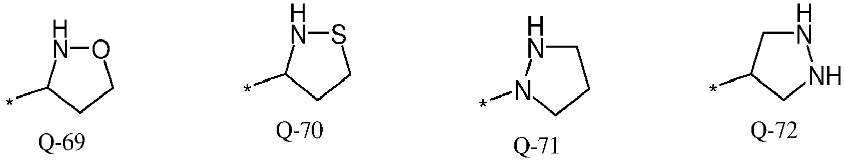 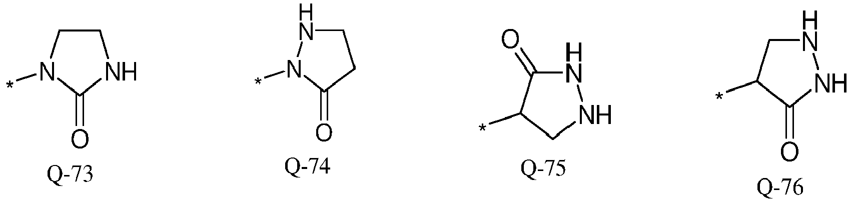 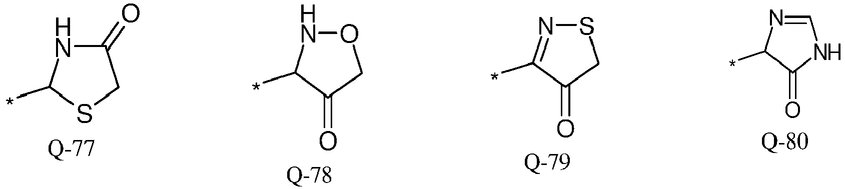 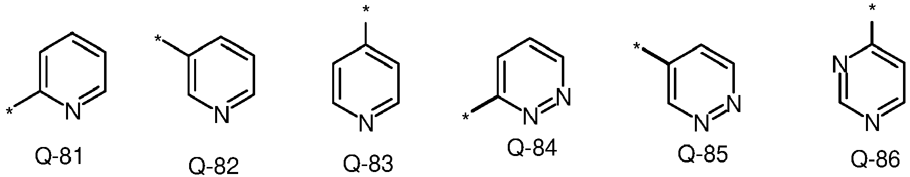 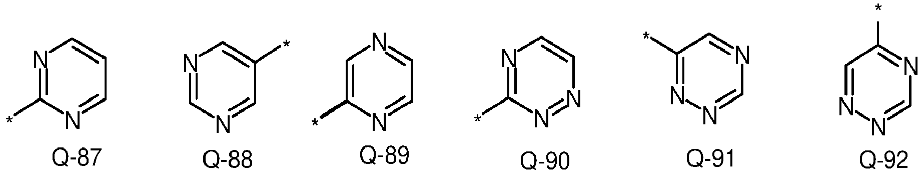 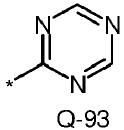 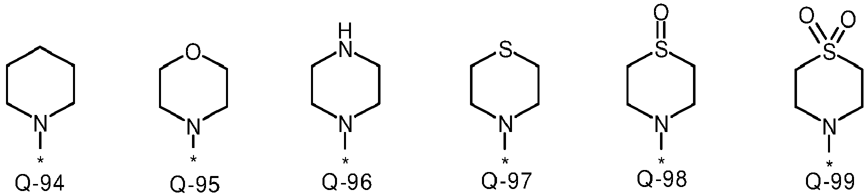 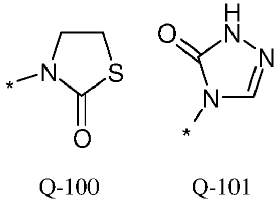
wobei die Bindung zum Restmolekül mit einem Sternchen * gekennzeichnet ist,
- R²: steht weiterhin besonders bevorzugt für eine Gruppe ausgewählt aus G1, G2, G3, G4, G6, G7, G8, G9, G10, G11, G12, G13, G14, G15, G16, G18 oder G19, wobei wenn R² für G6, G7, G8, G9 steht, dann steht n für 2,
- X: steht besonders bevorzugt für ein heteroaromatisches 9-gliedriges oder 12-gliedriges annelliertes bicyclisches oder tricyclisches Ringsystem aus der Reihe H1 bis H20, 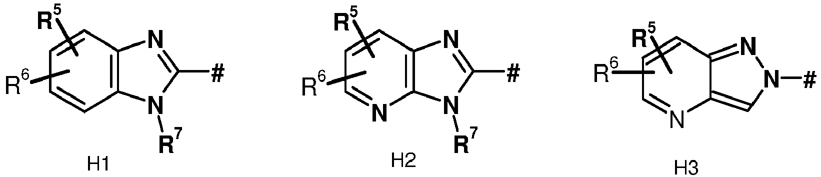 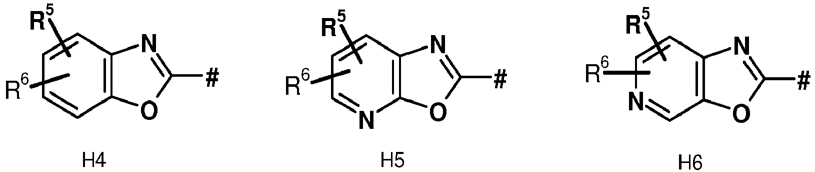 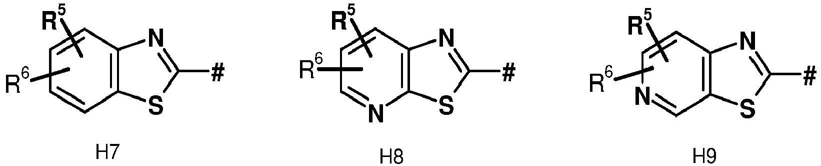 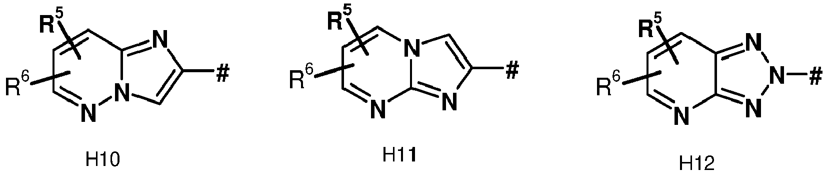 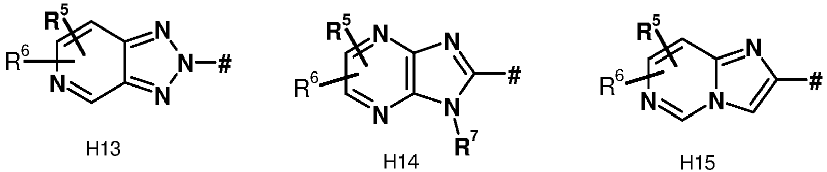 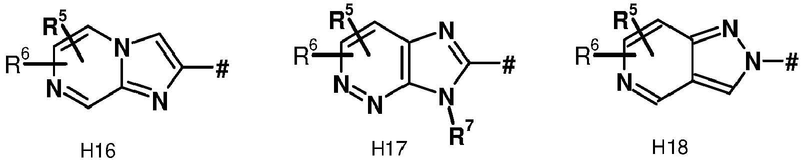 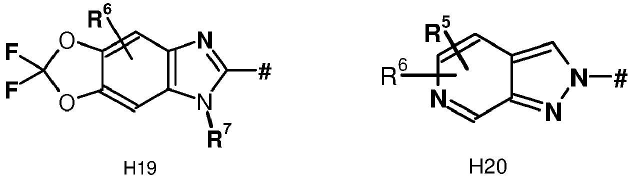
wobei die Bindung zum Restmolekül mit einer Raute ^{#} gekennzeichnet ist,
- R⁸: steht besonders bevorzugt für Wasserstoff, Amino, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, (C₁-C₄)Alkylamino, (C₁-C₄)Alkoxy-(C₁-C₄)alkyl, (C₃-C₆)Cycloalkyl oder (C₃-C₆)Cycloalkyl-(C₁-C₄)alkyl, für jeweils gegebenenfalls einfach durch Halogen, Cyano, Phenyl oder Pyridyl substituiertes (C₁-C₄)Alkyl oder (C₁-C₄)Alkoxy, wobei Phenyl bzw. Pyridyl jeweils gegebenenfalls einfach oder zweifach, gleich oder verschieden durch Trifluormethyl, Cyano, Fluor, Chlor, Brom oder Trifluormethoxy substituiert sein kann, oder
- R⁸: steht besonders bevorzugt für Phenyl, Thiazolyl, Pyrazolyl, Pyridyl oder einen 3- bis 6-gliedrigen gesättigten Heterocyclus, enthaltend 1-2 Heteroatome aus der Reihe N,S,O, wobei Phenyl, Pyridyl bzw. der Heterocyclus jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹³, R¹⁴, R¹⁵: stehen unabhängig voneinander besonders bevorzugt für jeweils gegebenenfalls einfach durch Halogen, Cyano, Phenyl oder Pyridyl substituiertes (C₁-C₄)Alkyl oder (C₃-C₆)Cycloalkyl, wobei Phenyl bzw. Pyridyl jeweils gegebenenfalls einfach oder zweifach, gleich oder verschieden durch Trifluormethyl, Cyano, Fluor, Chlor oder Trifluormethoxy substituiert sein kann, oder
- R¹³, R¹⁴, R¹⁵: stehen unabhängig voneinander besonders bevorzugt für Phenyl, Pyridyl, oder einen 3-bis 6-gliedrigen gesättigten Heterocyclus, enthaltend 1-2 Heteroatome aus der Reihe N,S,O, wobei Phenyl, Pyridyl, bzw. der Heterocyclus jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹¹, R¹²: stehen unabhängig voneinander besonders bevorzugt für Wasserstoff oder für R¹³,
wobei wenn R² für gegebenenfalls substituiertes Q45 steht, dann steht X nicht für H1, H2 oder H4 und wenn R² für G10, G11, G12, G13, G14, G15 oder G16 steht und A¹ für N steht, dann steht X nicht für H1, H2, H4, H5, H7, H8 oder H14.

Die Substitution an den Ringen Q1 bis Q101 kann durch Substitution von Wasserstoff am C-Atom und/oder am N-Atom erfolgen.

### Ausgestaltung 4-1:

- A¹: steht ganz besonders bevorzugt für Stickstoff oder =C-R⁴,
- R¹: steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,
- R²: steht ganz besonders bevorzugt für einen gegebenenfalls einfach durch Cyano, (C₁-C₄)Cyanoalkyl, (C₁-C₄)Alkoxy-(C₁-C₄)alkyl, Halogen, (C₁-C₄)Alkyl, (C₁-C₄)Halogenalkyl, (C₃-C₆)Cycloalkyl, (C₁-C₄)Alkylthio, (C₁-C₄)Halogenalkylthio, (C₁-C₄)Alkylsulfonyl, (C₁-C₄)Alkylaminocarbonyl, (C₁-C₄)Alkylcarbonylamino substituierten Ring, ausgewählt aus Q-25, Q-41, Q-42, Q-43, Q-44, Q-45, Q-46, Q-47, Q-52 oder Q-95,
- R²: steht weiterhin ganz besonders bevorzugt für eine Gruppe ausgewählt aus G1, G2, G3, G4, G6, G7, G8, G9, G10, G11, G12, G13, G14, G15, G16 oder G18, wobei wenn R² für G6, G7, G8, G9 steht, dann steht n für 2,
- R³: steht ganz besonders bevorzugt für Wasserstoff, Cyano, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl, (C₁-C₄)Halogenalkylsulfonyl oder NHCO-(C₁-C₄)alkyl ((C₁-C₄)Alkylcarbonylamino),
- R⁴: steht ganz besonders bevorzugt für Wasserstoff, Fluor, Chlor, Brom oder Cyano,
- X: steht ganz besonders bevorzugt für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18 oder H19,
- R⁵: steht ganz besonders bevorzugt für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylcarbonyl, (C₁-C₄)Halogenalkylcarbonyl, (C₁-C₄)Alkylaminocarbonyl, Di-(C₁-C₄)alkyl-aminocarbonyl, (C₁-C₄)Alkylsulfonylamino, (C₁-C₄)Alkylaminosulfonyl oder Di-(C₁-C₄)alkylaminosulfonyl,
- R⁶: steht ganz besonders bevorzugt für Wasserstoff,
- R⁷: steht ganz besonders bevorzugt für (C₁-C₄)Alkyl oder (C₁-C₄)Alkoxy-(C₁-C₄)alkyl,
- R⁸: steht ganz besonders bevorzugt für Wasserstoff, (C₁-C₄)Alkyl, (C₁-C₄)Halogenalkyl, (C₁-C₄)Alkoxy-(C₁-C₄)alkyl, (C₃-C₆)Cycloalkyl, (C₃-C₆)Cycloalkyl-(C₁-C₄)alkyl oder (C₁-C₄)Alkoxy, oder
- R⁸: steht ganz besonders bevorzugt für Phenyl oder Pyridyl, wobei Phenyl bzw. Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹³, R¹⁴, R¹⁵: stehen unabhängig voneinander ganz besonders bevorzugt für (C₁-C₄)Alkyl oder
- R¹³, R¹⁴, R¹⁵: stehen unabhängig voneinander ganz besonders bevorzugt für Phenyl oder Pyridyl, wobei Phenyl oder Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹¹, R¹²: stehen unabhängig voneinander ganz besonders bevorzugt für Wasserstoff oder für R¹³,
- n: steht ganz besonders bevorzugt für 0, 1 oder 2,
- p: steht ganz besonders bevorzugt für 0 oder 1.

### Ausgestaltung 4-2:

- A¹: steht ganz besonders bevorzugt für Stickstoff oder =C-R⁴,
- R¹: steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,
- R²: steht ganz besonders bevorzugt für einen gegebenenfalls einfach durch Cyano, (C₁-C₄)Cyanoalkyl, (C₁-C₄)Alkoxy-(C₁-C₄)alkyl, Halogen, (C₁-C₄)Alkyl, (C₁-C₄)Halogenalkyl, (C₃-C₆)Cycloalkyl, (C₁-C₄)Alkylthio, (C₁-C₄)Halogenalkylthio, (C₁-C₄)Alkylsulfonyl, (C₁-C₄)Alkylaminocarbonyl, (C₁-C₄)Alkylcarbonylamino substituierten Ring, ausgewählt aus Q-25, Q-41, Q-42, Q-43, Q-44, Q-45, Q-46, Q-47, Q-52 oder Q-95,
- R²: steht weiterhin ganz besonders bevorzugt für eine Gruppe ausgewählt aus G1, G2, G3, G4, G10, G11, G12, G13, G14, G15, G18 oder G19,
- R³: steht ganz besonders bevorzugt für Wasserstoff, Cyano, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl, (C₁-C₄)Halogenalkylsulfonyl oder NHCO-(C₁-C₄)alkyl ((C₁-C₄)Alkylcarbonylamino),
- R⁴: steht ganz besonders bevorzugt für Wasserstoff, Fluor, Chlor, Brom oder Cyano,
- X: steht ganz besonders bevorzugt für ein heteroaromatisches 9-gliedriges oder 12-gliedriges annelliertes bicyclisches oder tricyclisches Ringsystem aus der Reihe H1, H2, H3, H4, H5, H7, H8, H10, H11, H12, H13, H15, H16, H17, H18, H19 oder H20,
- R⁵: steht ganz besonders bevorzugt für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylcarbonyl, (C₁-C₄)Halogenalkylcarbonyl, (C₁-C₄)Alkylaminocarbonyl, Di-(C₁-C₄)alkyl-aminocarbonyl, (C₁-C₄)Alkylsulfonylamino, (C₁-C₄)Alkylaminosulfonyl oder Di-(C₁-C₄)alkylaminosulfonyl,
- R⁶: steht ganz besonders bevorzugt für Wasserstoff,
- R⁷: steht ganz besonders bevorzugt für (C₁-C₄)Alkyl oder (C₁-C₄)Alkoxy-(C₁-C₄)alkyl,
- R⁸: steht ganz besonders bevorzugt für Wasserstoff, (C₁-C₄)Alkyl, (C₁-C₄)Halogenalkyl, (C₁-C₄)Alkoxy-(C₁-C₄)alkyl, (C₁-C₄)Alkylthio-(C₁-C₄)alkyl, (C₁-C₄)Alkylsulfonyl-(C₁-C₄)alkyl, (C₃-C₆)Cycloalkyl, (C₃-C₆)Cycloalkyl-(C₁-C₄)alkyl oder (C₁-C₄)Alkoxy, oder
- R⁸: steht ganz besonders bevorzugt für Phenyl oder Pyridyl, wobei Phenyl bzw. Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
- R¹³, R¹⁴, R¹⁵: stehen unabhängig voneinander ganz besonders bevorzugt für (C₁-C₄)Alkyl oder (C₃-C₆)Cycloalkyl oder
- R¹³, R¹⁴, R¹⁵: stehen unabhängig voneinander ganz besonders bevorzugt für Phenyl oder Pyridyl, wobei Phenyl oder Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano, oder für Oxetanyl,
- R¹¹, R¹²: stehen unabhängig voneinander ganz besonders bevorzugt für Wasserstoff oder für R¹³,
- n: steht ganz besonders bevorzugt für 0, 1 oder 2,
- p: steht ganz besonders bevorzugt für 0 oder 1,
wobei wenn R² für gegebenenfalls substituiertes Q45 steht, dann steht X nicht für H1, H2 oder H4 und wenn R² für G10, G11, G12, G13, G14 oder G15 steht und A¹ für N steht, dann steht X nicht für H1, H2, H4, H5, H7 oder H8.

### Ausgestaltung 5-1:

- A¹: steht hervorgeoben für Stickstoff oder =C-R⁴,
- R¹: steht hervorgehoben für Methyl, Ethyl, n-Propyl, i-Propyl oder cyclo-Propyl,
- R²: steht hervorgehoben für einen gegebenenfalls einfach durch Fluor, Chlor, Brom, Iod, Cyano, Cyanomethyl, Methyl, Methoxymethyl, Trifluormethyl, Trifluormethylthio, Difluormethyl, Pentafluorethyl, Cyclopropyl, Methylaminocarbonyl, Methylcarbonylamino, Methylsulfonyl oder Methylthio substituierten Ring aus der Reihe Q-25, Q-41, Q-42, Q-43, Q-44, Q-45, Q-46, Q-47, Q-52 oder Q-95, (besonders hervorgehoben für Q-42, Q-43, Q-44, Q-45, Q46, Q-47 oder Q-52)
- R²: steht weiterhin hervorgehoben für eine Gruppe ausgewählt aus G1, G10, G12, G13 oder G14 (besonders hervorgehoben für G12 oder G13),
- R³: steht hervorgehoben für Wasserstoff, Cyano, Methyl, Ethyl, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl, Trifluormethylsulfinyl, Fluor, Brom oder Chlor, (besonders hervorgehoben für Wasserstoff),
- R⁴: steht hervorgehoben für Wasserstoff,
- X: steht hervorgehoben für ein heteroaromatisches 9-gliedriges oder 12-gliedriges annelliertes bicyclisches oder tricyclisches Ringsystem aus der Reihe H1, H2, H3, H4, H5, H7, H8, H10, H11, H15, H16, H17 oder H19,
- R⁵: steht hervorgehoben für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl, (besonders hervorgehoben für Trifluormethyl),
- R⁶: steht hervorgehoben für Wasserstoff,
- R⁷: steht hervorgehoben für Methyl, Ethyl, i-Propyl, Methoxymethyl oder Methoxyethyl, (besonders hervorgehoben für Methyl),
- R⁸: steht hervorgehoben für Wasserstoff, Methyl, Ethyl, iso-Propyl, tert-Butyl, Trifluormethyl, Difluormethyl, Tetrafluorethyl, Pentafluorethyl, Methoxymethyl, Cyclopropyl, Cyclopropyl-methyl, Methoxy oder Ethoxy, oder für Phenyl,
- R¹³: steht hervorgehoben für Methyl, Ethyl, iso-Propyl, tert-Butyl, Benzyl oder Methylsulfonyl,
- R¹¹, R¹²: stehen unabhängig voneinander hervorgehoben für Wasserstoff oder für R¹³,
- n: steht hervorgehoben für 0, 1 oder 2.

### Ausgestaltung 5-2:

- A¹: steht hervorgeoben für Stickstoff,
- R¹: steht hervorgehoben für Methyl, Ethyl, n-Propyl, i-Propyl oder cyclo-Propyl,
- R²: steht hervorgehoben für einen gegebenenfalls einfach durch Fluor, Chlor, Brom, Iod, Trifluormethyl, Difluormethyl oder Pentafluorethyl substituierten Ring aus der Reihe Q-42, Q-43, Q-45 oder Q-47,
- R²: steht weiterhin hervorgehoben für eine Gruppe ausgewählt aus G10, wobei R¹¹ für Wasserstoff steht und R¹² für Wasserstoff oder Oxetanyl steht
oder G12, wobei R¹¹ für Wasserstoff steht und R⁸ für Wasserstoff, Methyl, Ethyl, iso-Propyl, tert-Butyl, Trifluormethyl, Difluormethyl, Tetrafluorethyl, Pentafluorethyl, Cyclopropyl, Cyclopropyl-methyl, Methylsulfanylmethyl ( 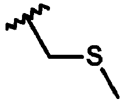 ), Methylsulfonylpropan-2-yl ( 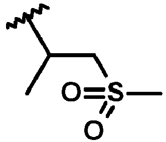 ), 1-(Methylsulfanyl)propan-2-yl ( 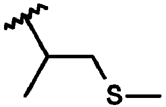 ) steht
oder G3, wobei R¹¹ für Wasserstoff steht und R¹² für Methyl, Ethyl, iso-Propyl, Cyclopropyl oder tert-Butyl steht,
- R³: steht hervorgehoben für Wasserstoff,
- X: steht hervorgehoben für ein heteroaromatisches 9-gliedriges oder 12-gliedriges annelliertes bicyclisches oder tricyclisches Ringsystem aus der Reihe H1, H2, H5, H15, H16, H17 oder H19,
- R⁵: steht hervorgehoben für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl,
- R⁶: steht hervorgehoben für Wasserstoff,
- R⁷: steht hervorgehoben für Methyl, Ethyl, i-Propyl, Methoxymethyl oder Methoxyethyl,
- n: steht hervorgehoben für 0, 1 oder 2,
wobei wenn R² für gegebenenfalls substituiertes Q45 steht, dann steht X nicht für H1 oder H2 und wenn R² für G10 oder G12 steht, dann steht X nicht für H1, H2 oder H5.

### Ausgestaltung 6-1:

- A¹: steht insbesondere für Stickstoff,
- R¹: steht insbesondere für Ethyl,
- R²: steht insbesondere für einen gegebenenfalls einfach durch Chlor oder Trifluormethyl substituierten Ring aus der Reihe Q-42,
- R²: steht weiterhin insbesondere für eine Gruppe ausgewählt aus G10 oder G12,
- R³: steht insbesondere für Wasserstoff,
- X: steht insbesondere für ein heteroaromatisches 9-gliedriges oder 12-gliedriges annelliertes bicyclisches oder tricyclisches Ringsystem aus der Reihe H1, H2 oder H19,
- R⁵: steht insbesondere für Trifluormethyl,
- R⁶: steht insbesondere für Wasserstoff,
- R⁷: steht insbesondere für Methyl,
- R⁸: steht insbesondere für Methyl, Trifluormethyl oder Cyclopropyl,
- R¹¹, R¹²: stehen unabhängig voneinander insbesondere für Wasserstoff,
- n: steht insbesondere für 1 oder 2.

### Ausgestaltung 6-2a:

- A¹: steht insbesondere für Stickstoff,
- R¹: steht insbesondere für Ethyl,
- R²: steht insbesondere für einen gegebenenfalls einfach durch Fluor, Chlor oder Trifluormethyl substituierten Ring aus der Reihe Q-42, Q-43, Q-45 oder Q-47,
- R²: steht weiterhin insbesondere für eine Gruppe ausgewählt aus G10, wobei R¹¹ für Wasserstoff steht und R¹² für Wasserstoff oder 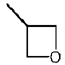 steht
oder G12, wobei R¹¹ für Wasserstoff steht und R⁸ für Methyl, Trifluormethyl, Cyclopropyl, -CH₂SCH₃, -CH(CH₃)CH₂SCH₃ oder -CH(CH₃)CH₂SO₂CH₃ steht
oder G3, wobei R¹¹ für Wasserstoff steht und R¹² für Methyl steht,
- R³: steht insbesondere für Wasserstoff,
- X: steht insbesondere für ein heteroaromatisches 9-gliedriges oder 12-gliedriges annelliertes bicyclisches oder tricyclisches Ringsystem aus der Reihe H1, H2, H5, H15, H16, H17 oder H19,
- R⁵: steht insbesondere für Trifluormethyl,
- R⁶: steht insbesondere für Wasserstoff,
- R⁷: steht insbesondere für Methyl,
- n: steht insbesondere für 0, 1 oder 2,
wobei wenn R² für gegebenenfalls substituiertes Q45 steht, dann steht X nicht für H1 oder H2 und wenn R² für G10 oder G12 steht, dann steht X nicht für H1, H2 oder H5.

### Ausgestaltung 6-2b:

A¹, R¹, R³, R⁵, R⁶, R⁷, X und n haben die in Ausgestaltung 6-2a angegebenen Bedeutungen und
R² steht insbesonders für einen Rest aus der Reihe 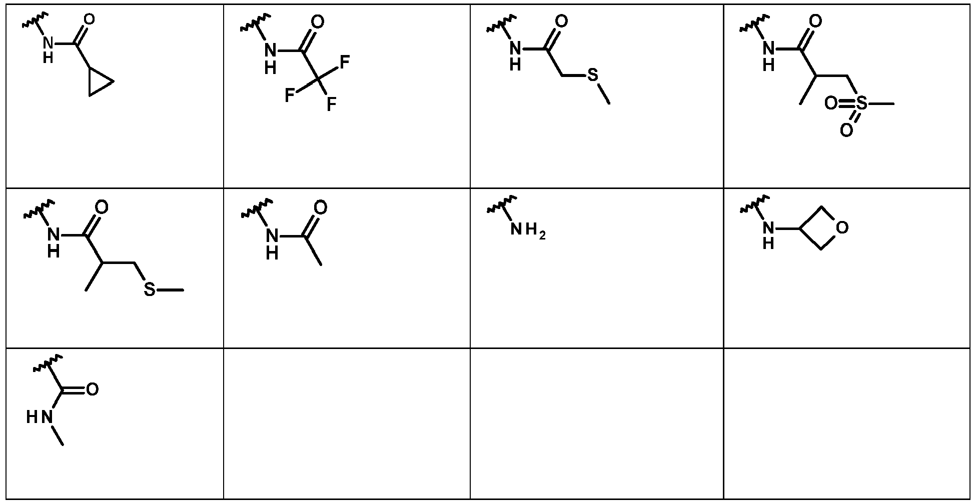
R² steht weiterhin insbesonders für einen Rest aus der Reihe 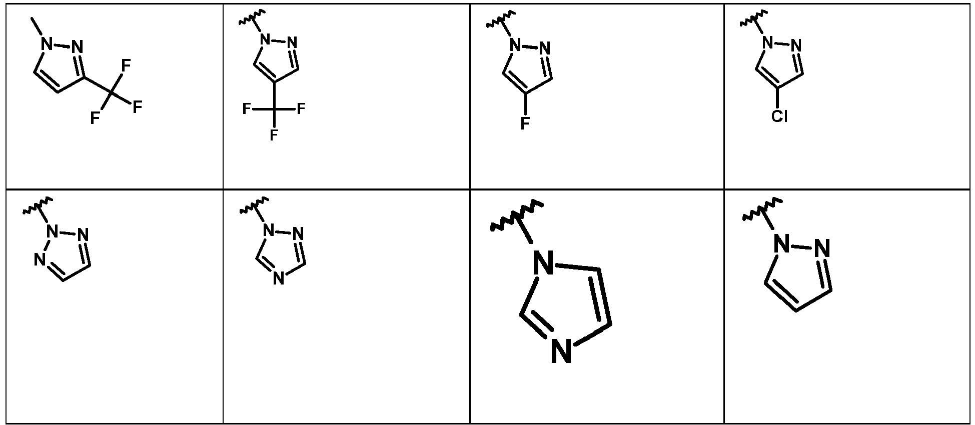
wobei wenn R² für gegebenenfalls substituiertes Q45 steht, dann steht X nicht für H1 oder H2 und wenn R² für G10 oder G12 steht, dann steht X nicht für H1, H2 oder H5.

### Ausgestaltung 6-2c:

A¹, R¹, R², R³, R⁵, R⁶, R⁷ und n haben die in Ausgestaltung 6-2a angegebenen Bedeutungen und
X steht insbesonders für einen Rest aus der Reihe 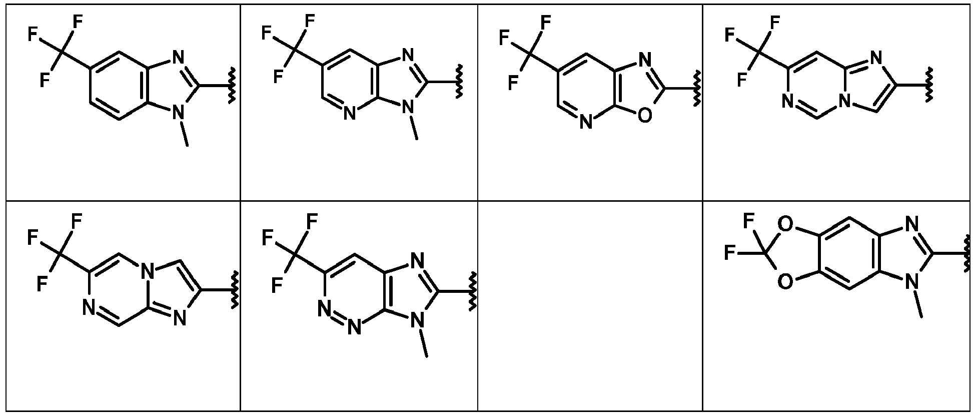
wobei wenn R² für gegebenenfalls substituiertes Q45 steht, dann steht X nicht für H1 oder H2 und wenn R² für G10 oder G12 steht, dann steht X nicht für H1, H2 oder H5.

A¹ steht für =N⁺-O⁻ bedeutet =N⁺(O⁻)-; A¹ steht für =C-R⁴ bedeutet =C(R⁴)- (identisch mit CR⁴).

Hervorgehoben sind Verbindungen der Formel (I), in welcher A¹, R¹, R³, R⁵, R⁶, R⁷, X und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben und R² für einen gegebenenfalls einfach durch Fluor, Chlor oder Trifluormethyl substituierten Ring aus der Reihe Q-42 oder Q-47 steht.

Hervorgehoben sind auch Verbindungen der Formel (I), in welcher A¹, R¹, R³, R⁵, R⁶, R⁷, X und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben und R² für einen gegebenenfalls einfach durch Fluor, Chlor, Brom, Iod, Trifluormethyl, Difluormethyl oder Pentafluorethyl substituieren Ring aus der Reihe Q-42 oder Q-47 steht.

Hervorgehoben sind Verbindungen der Formel (I), in welcher A¹, R¹, R³, R⁵, R⁶, R⁷, X und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben und R² für einen gegebenenfalls einfach durch Fluor, Chlor oder Trifluormethyl substituierten Ring aus der Reihe Q-45 steht, wobei X nicht für H1, H2 oder H4 stehen kann.

Hervorgehoben sind Verbindungen der Formel (I), in welcher A¹, R¹, R³, R⁵, R⁶, R⁷, R¹¹, R¹², X und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben und R² für G3 steht.

Hervorgehoben sind Verbindungen der Formel (I), in welcher A¹, R¹, R³, R⁵, R⁶, R⁷, R⁸, R¹¹, R¹², X und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben und R² für G10 oder G12 steht, wobei X nicht für H1, H2, H4, H5, H7, H8 oder H14 stehen kann.

In einer bevorzugten Ausführungsform betrifft die Erfindung Verbindungen der Formel (I), wobei X für H1 steht und R¹, R², R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für G12 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für G13 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht und R¹, R², R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für G12 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für G13 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H2 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht und R¹, R², R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für G12 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für G13 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht und R¹, R², R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für G12 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für G13 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht und R¹, R², R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für G12 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für G13 steht und R¹, R³, R⁵, R⁶, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht und R¹, R², R³, R⁷, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) haben.

In einer bevorzugten Ausführungsform betrifft die Erfindung Verbindungen der Formel (I), wobei X für H19 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für G12 steht und R¹, R³, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für G13 steht und R¹, R³, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht und R¹, R², R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für G10 steht und R¹, R³, R⁵, R⁶, R⁷, R¹¹, R¹², A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für G12 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für G13 steht und R¹, R³, R⁵, R⁶, R⁷, R⁸, R¹¹, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q42 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q43 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q44 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q45 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q46 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q47 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² für Q52 (gegebenenfalls einfach durch Halogen, Cyano, Trifluormethyl, Difluormethyl, Pentafluorethyl, Methylsulfonyl, Methylthio, Cyanomethyl, Methyl, Methoxymethyl, Trifluormethylthio, Cyclopropyl, Methylaminocarbonyl oder Methylcarbonylamino substituiert) steht und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H1 steht, R² die in der Tabelle 1 angegebenen Bedeutungen hat und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

**Tabelle 1:**

| R² |
|---|
| acetyl amino |
| propanoylamino |
| cyclopropancarbonylamino |
| (2-cyclopropylacetyl)amino |
| 2- methylpropanoylamino |
| (2-methoxyacetyl) amino |
| benzamido |
| (2,2-difluoroacetyl)amino |
| (2,2,2-trifluoracetyl)amino |
| 2,2,3,3,3-pentafluorpropanoylamino |
| 2,2,3,3-tetrafluoropropanoylamino |
| methoxycarbonyl |
| amino |
| morpholin-4-yl |
| 3-[(trifluormethyl)sulfanyl]-1H-pyrrol-1-yl |
| 4-(methylsulfonyl)-1H-pyrazol-1-yl |
| 3-(methylcarbamoyl)-1H-pyrazol-1-yl |
| 4-(methylcarbamoyl)-1H-pyrazol-1-yl |
| 3-(methylsulfonyl)-1H-1,2,4-triazol-1-yl |
| 4-chlor-1H-pyrazol-1-yl |
| 3-chlor-1H-pyrazol-1-yl |
| 4-iod-1H-pyrazol-1-yl |
| 3-iod-1H-pyrazol-1-yl |
| 3-fluor-1H-pyrazol-1-yl |
| 4-brom-1H-pyrazol-1-yl |
| 3-brom-1H-pyrazol-1-yl |
| 3-(cyanomethyl)-1H-pyrazol-1-yl |
| 3-(difluormethyl)-1H-pyrazol-1-yl |
| 3-(methoxymethyl)-1H-pyrazol-1-yl |
| 3-acetamido-1H-1,2,4-triazol-1-yl |
| 4-cyclopropyl-1H-pyrazol-1-yl |
| 4-iodo-1H-imidazol-1-yl |
| 3-(methylsulfanyl)-1H-1,2,4-triazol-1-yl |
| 4-(methylsulfanyl)-1H-pyrazol-1-yl |
| 2H-1,2,3-triazol-2-yl |
| 1H-1,2,4-triazol-1-yl |
| 3-chlor-1H-1,2,4-triazol-1-yl |
| 4-cyano-1H-pyrazol-1-yl |
| 3-thienyl |
| 1H-pyrazol-1-yl |
| 3-cyano-1H-pyrazol-1-yl |
| 3-acetamido-1H-pyrazol-1-yl |
| 4-(trifluormethyl)-1H-1,2,3-triazol-1-yl |
| 3-(trifluormethyl)-1H-pyrazol-1-yl |
| 4-(trifluormethyl)-1H-pyrazol-1-yl |
| 4-(trifluormethyl)-1H-imidazol-1-yl |
| 4-fluor-1H-pyrazol-1-yl |
| 3-(methylsulfanyl)-1H-pyrazol-1-yl |
| 4-(trifluormethyl)-2H-1,2,3-triazol-2-yl |
| 4-methyl-1H-pyrazol-1-yl |
| 1-imidazol-1yl |
| 3-methyl-1H-pyrazol-1-yl |
| 3-(methylsulfonyl)-1H-pyrazol-1-yl |
| (Pyridin-2-ylcarbonyl)amino |
| [2-Methyl-3-(methylsulfinyl)propanoyl]amino |
| [(Methylsulfanyl)acetyl] amino |
| [2-Methyl-3-(methylsulfonyl)propanoyl]amino |
| [(Methylsulfonyl) acetyl] amino |
| [2-Methyl-3-(methylsulfanyl)propanoyl]amino |
| [(2-Methyl-1,3-thiazol-4-yl)carbonyl]amino |
| [(1-Methyl-1H-pyrazol-3-yl)carbonyl]amino |
| (1,3-Thiazol-2-ylcarbonyl)amino |
| (1,3-Thiazol-4-ylcarbonyl)amino |
| {[2-(Trifluormethyl)-1,3-thiazol-4-yl]carbonyl}amino |
| Ethanethioylamino |
| Methylamino |
| Dimethylamino |
| Ethylamino |
| 1 -Methylhydrazino |
| Hydrazino |
| 1,2-Dimethylhydrazino |
| (Methoxycarbonyl)amino |
| Carbamoylamino |
| (Ethylcarbamoyl)amino |
| Methyloxy |
| Ethyloxy |
| Carbamoyl |
| Methylcarbamoyl |
| Ethylcarbamoyl |
| (Methylamino)carbonothioyl |
| Prop-1-en-1-yl |
| 2-Cyclopropylvinyl |
| 2-Phenylvinyl |
| 2-(4-Chlorphenyl)vinyl |
| 4-Chlor-3-(pentafluorethyl)-1H-pyrazol-1-yl |
| 3-Cyclopropyl-1H-pyrazol-1-yl |
| 3-[(Methylsulfonyl)methyl]-1H-pyrazol-1-yl |
| 3-[(Methylsulfinyl)methyl]-1H-pyrazol-1-yl |
| 4-(Methylsulfanyl)-1H-pyrazol-1-yl |
| 3-(Methylsulfanyl)-1H-1,2,4-triazol-1-yl |
| 1,3-Dimethyl-5-oxo-1,5-dihydro-4H-1,2,4-triazol-4-yl |
| 4-Methyl-5-oxo-4,5-dihydro-1 H-tetrazol-1-yl |
| 5-Oxo-4-(2,2,2-trifluorethyl)-4,5-dihydro-1H-tetrazol-1-yl |
| 5-Oxo-4-(2,2,3,3,3-pentafluorpropyl)-4,5-dihydro-1-tetrazol-1yl |
| 2-Oxo-1,3-thiazolidin-3-yl |
| 5-Chlorpyridin-2-yl |
| 2-Chlor-1-methyl-1H-imidazol-5-yl |
| 1H-Pyrrol-3-yl |
| 1-Methyl-1H-pyrazol-4-yl |
| Oxetan-3-ylamino |

In einer bevorzugten Ausführungsform betrifft die Erfindung Verbindungen der Formel (I), wobei X für H2 steht, R² die in der Tabelle 1 angegebenen Bedeutungen hat und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H3 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H4 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H5 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H6 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H7 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H8 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H9 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H10 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H11 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H12 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H13 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H14 steht, R² die in der Tabelle 1 angegebenen Bedeutungen hat und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H15 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H16 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H17 steht, R² die in der Tabelle 1 angegebenen Bedeutungen hat und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H18 steht, R² die in der Tabelle 1 angegebenen Bedeutungen hat und R¹, R³, R⁵, R⁶, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H19 steht, R² die in der Tabelle 1 angegebenen Bedeutungen hat und R¹, R³, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

In einer bevorzugten Ausführungsform betrifft die Erfindung Verbindungen der Formel (I), wobei X für H20 steht, R² die in der Tabelle 1 angegebenen Bedeutungen hat und R¹, R³, R⁵, R⁶, R⁷, A¹ und n die in Ausgestaltung (3-1) oder Ausgestaltung (3-2) oder Ausgestaltung (4-1) oder Ausgestaltung (4-2) oder Ausgestaltung (5-1) oder Ausgestaltung (5-2) oder Ausgestaltung (6-1) oder Ausgestaltung (6-2a) oder Ausgestaltung (6-2b) angegebenen Bedeutungen haben.

Im einzelnen seien außerdem die folgenden Verbindungen der Formel (I-A) genannt: 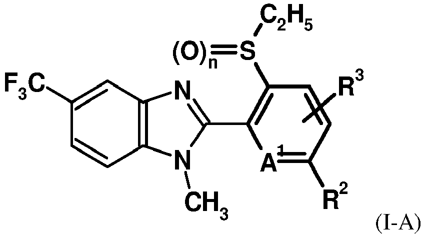 A¹, R², R³ und n wie in Tabelle 2 genannt.

**Tabelle 2:**

| A¹ | R² | R³ | n |
|---|---|---|---|
| N | acetyl amino | H | 0 |
| N | propanoylamino | H | 0 |
| N | cyclopropancarbonylamino | H | 0 |
| N | (2-cyclopropylacetyl)amino | H | 0 |
| N | 2-methylpropanoylamino | H | 0 |
| N | (2-methoxyacetyl) amino | H | 0 |
| N | benzamido | H | 0 |
| N | (2,2-difluoroacetyl)amino | H | 0 |
| N | (2,2,2-trifluoracetyl)amino | H | 0 |
| N | 2,2,3,3,3-pentafluorpropanoylamino | H | 0 |
| N | 2,2,3,3-tetrafluoropropanoylamino | H | 0 |
| N | methoxycarbonyl | H | 0 |
| N | amino | H | 0 |
| N | morpholin-4-yl | H | 0 |
| N | 3-[(trifluormethyl)sulfanyl]-1-pyrrol-1-yl | H | 0 |
| N | 4-(methylsulfonyl)-1H-pyrazol-1-yl | H | 0 |
| N | 3-(methylcarbamoyl)-1H-pyrazol-1-yl | H | 0 |
| N | 4-(methylcarbamoyl)-1H-pyrazol-1-yl | H | 0 |
| N | 3-(methylsulfonyl)-1H-1,2,4-triazol-1-yl | H | 0 |
| N | 4-chlor-1H-pyrazol-1-yl | H | 0 |
| N | 3-chlor-1H-pyrazol-1-yl | H | 0 |
| N | 4-iod-1H-pyrazol-1-yl | H | 0 |
| N | 3-iod-1H-pyrazol-1-yl | H | 0 |
| N | 3-fluor-1-pyrazol-1yl | H | 0 |
| N | 4-brom-1H-pyrazol-1-yl | H | 0 |
| N | 3-brom-1H-pyrazol-1-yl | H | 0 |
| N | 3-(cyanomethyl)-1H-pyrazol-1-yl | H | 0 |
| N | 3-(difluormethyl)-1H-pyrazol-1-yl | H | 0 |
| N | 3-(methoxymethyl)-1H-pyrazol-1-yl | H | 0 |
| N | 3-acetamido-1H-1,2,4-triazol-1-yl | H | 0 |
| N | 4-cyclopropyl-1H-pyrazol-1-yl | H | 0 |
| N | 4-iodo-1H-imidazol-1-yl | H | 0 |
| N | 3-(methylsulfanyl)-1H-1,2,4-triazol-1-yl | H | 0 |
| N | 4-(methylsulfanyl)-1H-pyrazol-1-yl | H | 0 |
| N | 2H-1,2,3-triazol-2-yl | H | 0 |
| N | 1H-1,2,4-triazol-1-yl | H | 0 |
| N | 3-chlor-1H-1,2,4-triazol-1-yl | H | 0 |
| N | 4-cyano-1H-pyrazol-1-yl | H | 0 |
| N | 3-thienyl | H | 0 |
| N | 1H-pyrazol-1-yl | H | 0 |
| N | 3-cyano-1H-pyrazol-1-yl | H | 0 |
| N | 3-acetamido-1H-pyrazol-1-yl | H | 0 |
| N | 4-(trifluormethyl)-1H-1,2,3-triazol-1-yl | H | 0 |
| N | 3-(trifluormethyl)-1H-pyrazol-1-yl | H | 0 |
| N | 4-(trifluormethyl)-1H-pyrazol-1-yl | H | 0 |
| N | 4-(trifluormethyl)-1H-imidazol-1-yl | H | 0 |
| N | 4-fluor-1H-pyrazol-1-yl | H | 0 |
| N | 3-(methylsulfanyl)-1H-pyrazol-1-yl | H | 0 |
| N | 4-(trifluormethyl)-2H-1,2,3-triazol-2-yl | H | 0 |
| N | 4-methyl-1H-pyrazol-1-yl | H | 0 |
| N | 1H-imidazol-1-yl | H | 0 |
| N | 3-methyl-1H-pyrazol-1-yl | H | 0 |
| N | 3-(methylsulfonyl)-1H-pyrazol-1-yl | H | 0 |
| N | (Pyridin-2-ylcarbonyl)amino | H | 0 |
| N | [2-Methyl-3-(methylsulfinyl)propanoyl]amino | H | 0 |
| N | [(Methylsulfanyl)acetyl] amino | H | 0 |
| N | [2-Methyl-3-(methylsulfonyl)propanoyl]amino | H | 0 |
| N | [(Methylsulfonyl) acetyl] amino | H | 0 |
| N | [2-Methyl-3-(methylsulfanyl)propanoyl]amino | H | 0 |
| N | [(2-Methyl-1,3-thiazol-4-yl)carbonyl]amino | H | 0 |
| N | [(1-Methyl-1H-pyrazol-3-yl)carbonyl]amino | H | 0 |
| N | (1,3-Thiazol-2-ylcarbonyl)amino | H | 0 |
| N | (1,3-Thiazol-4-ylcarbonyl)amino | H | 0 |
| N | {[2-(Trifluormethyl)-1,3-thiazol-4-yl]carbonyl}amino | H | 0 |
| N | Ethanethioylamino | H | 0 |
| N | Methylamino | H | 0 |
| N | Dimethylamino | H | 0 |
| N | Ethylamino | H | 0 |
| N | 1-Methylhydrazino | H | 0 |
| N | Hydrazino | H | 0 |
| N | 1,2-Dimethylhydrazino | H | 0 |
| N | (Methoxycarbonyl)amino | H | 0 |
| N | Carbamoylamino | H | 0 |
| N | (Ethylcarbamoyl)amino | H | 0 |
| N | Methyloxy | H | 0 |
| N | Ethyloxy | H | 0 |
| N | Carbamoyl | H | 0 |
| N | Methylcarbamoyl | H | 0 |
| N | Ethylcarbamoyl | H | 0 |
| N | (Methylamino)carbonothioyl | H | 0 |
| N | Prop-1-en-1-yl | H | 0 |
| N | 2-Cyclopropylvinyl | H | 0 |
| N | 2-Phenylvinyl | H | 0 |
| N | 2-(4-Chlorphenyl)vinyl | H | 0 |
| N | 4-Chlor-3-(pentafluorethyl)-1H-pyrazol-1-yl | H | 0 |
| N | 3-Cyclopropyl-1H-pyrazol-1-yl | H | 0 |
| N | 3-[(Methylsulfonyl)methyl]-1H-pyrazol-1-yl | H | 0 |
| N | 3-[(Methylsulfinyl)methyl]-1H-pyrazol-1-yl | H | 0 |
| N | 4-(Methylsulfanyl)-1H-pyrazol-1-yl | H | 0 |
| N | 3-(Methylsulfanyl)-1H-1,2,4-triazol-1-yl | H | 0 |
| N | 1,3-Dimethyl-5-oxo-1,5-dihydro-4H-1,2,4-triazol-4-yl | H | 0 |
| N | 4-Methyl-5-oxo-4,5-dihydro-1 H-tetrazol-1-yl | H | 0 |
| N | 5-Oxo-4-(2,2,2-trifluorethyl)-4,5-dihydro-1H-tetrazol-1-yl | H | 0 |
| N | 5-Oxo-4-(2,2,3,3,3-pentafluorpropyl)-4,5-dihydro-1H-tetrazol-1-yl | H | 0 |
| N | 2-Oxo-1,3-thiazolidin-3-yl | H | 0 |
| N | 5-Chlorpyridin-2-yl | H | 0 |
| N | 2-Chlor-1-methyl-1H-imidazol-5-yl | H | 0 |
| N | 1H-Pyrrol-3-yl | H | 0 |
| N | 1-Methyl-1H-pyrazol-4-yl | H | 0 |
| N | Oxetan-3-ylamino | H | 0 |
| | | | |
| N | acetyl amino | H | 1 |
| N | propanoylamino | H | 1 |
| N | cyclopropancarbonylamino | H | 1 |
| N | (2-cyclopropylacetyl)amino | H | 1 |
| N | 2-methylpropanoylamino | H | 1 |
| N | (2-methoxyacetyl)amino | H | 1 |
| N | benzamido | H | 1 |
| N | (2,2-difluoroacetyl)amino | H | 1 |
| N | (2,2,2-trifluoracetyl)amino | H | 1 |
| N | 2,2,3,3,3-pentafluorpropanoylamino | H | 1 |
| N | 2,2,3,3-tetrafluoropropanoylamino | H | 1 |
| N | methoxycarbonyl | H | 1 |
| N | amino | H | 1 |
| N | morpholin-4-yl | H | 1 |
| N | 3-[(trifluormethyl)sulfanyl]-1H-pyrrol-1-yl | H | 1 |
| N | 4-(methylsulfonyl)-1H-pyrazol-1-yl | H | 1 |
| N | 3-(methylcarbamoyl)-1H-pyrazol-1-yl | H | 1 |
| N | 4-(methylcarbamoyl)-1H-pyrazol-1-yl | H | 1 |
| N | 3-(methylsulfonyl)-1H-1,2,4-triazol-1-yl | H | 1 |
| N | 4-chlor-1H-pyrazol-1-yl | H | 1 |
| N | 3-chlor-1H-pyrazol-1-yl | H | 1 |
| N | 4-iod-1H-pyrazol-1-yl | H | 1 |
| N | 3-iod-1H-pyrazol-1-yl | H | 1 |
| N | 3-fluor-1H-pyrazol-1-yl | H | 1 |
| N | 4-brom-1H-pyrazol-1-yl | H | 1 |
| N | 3-brom-1H-pyrazol-1-yl | H | 1 |
| N | 3-(cyanomethyl)-1H-pyrazol-1-yl | H | 1 |
| N | 3-(difluormethyl)-1H-pyrazol-1-yl | H | 1 |
| N | 3-(methoxymethyl)-1H-pyrazol- 1 -yl | H | 1 |
| N | 3-acetamido-1H-1,2,4-triazol-1-yl | H | 1 |
| N | 4-cyclopropyl-1H-pyrazol-1-yl | H | 1 |
| N | 4-iodo-1H-imidazol-1-yl | H | 1 |
| N | 3-(methylsulfanyl)-1H-1,2,4-triazol-1-yl | H | 1 |
| N | 4-(methylsulfanyl)-1H-pyrazol-1-yl | H | 1 |
| N | 2H-1,2,3-triazol-2-yl | H | 1 |
| N | 1H-1,2,4-triazol-1-yl | H | 1 |
| N | 3-chlor-1H-1,2,4-triazol-1-yl | H | 1 |
| N | 4-cyano-1H-pyrazol-1-yl | H | 1 |
| N | 3-thienyl | H | 1 |
| N | 1H-pyrazol-1-yl | H | 1 |
| N | 3-cyano-1H-pyrazol-1-yl | H | 1 |
| N | 3-acetamido-1H-pyrazol-1-yl | H | 1 |
| N | 4-(trifluormethyl)-1H-1,2,3-triazol-1-yl | H | 1 |
| N | 3-(trifluormethyl)-1H-pyrazol-1-yl | H | 1 |
| N | 4-(trifluormethyl)-1H-pyrazol-1-yl | H | 1 |
| N | 4-(trifluormethyl)-1H-imidazol-1-yl | H | 1 |
| N | 4-fluor-1H-pyrazol-1-yl | H | 1 |
| N | 3-(methylsulfanyl)-1H-pyrazol-1-yl | H | 1 |
| N | 4-(trifluormethyl)-2H-1,2,3-triazol-2-yl | H | 1 |
| N | 4-methyl-1H-pyrazol-1-yl | H | 1 |
| N | 1H-imidazol-1-yl | H | 1 |
| N | 3-methyl-1H-pyrazol-1-yl | H | 1 |
| N | 3-(methylsulfonyl)-1H-pyrazol-1-yl | H | 1 |
| N | (Pyridin-2-ylcarbonyl)amino | H | 1 |
| N | [2-Methyl-3-(methylsulfinyl)propanoyl]amino | H | 1 |
| N | [(Methylsulfanyl)acetyl] amino | H | 1 |
| N | [2-Methyl-3-(methylsulfonyl)propanoyl]amino | H | 1 |
| N | [(Methylsulfonyl) acetyl] amino | H | 1 |
| N | [2-Methyl-3-(methylsulfanyl)propanoyl]amino | H | 1 |
| N | [(2-Methyl-1,3-thiazol-4-yl)carbonyl]amino | H | 1 |
| N | [(1-Methyl-1H-pyrazol-3-yl)carbonyl]amino | H | 1 |
| N | (1,3-Thiazol-2-ylcarbonyl)amino | H | 1 |
| N | (1,3-Thiazol-4-ylcarbonyl)amino | H | 1 |
| N | {[2-(Trifluormethyl)-1,3-thiazol-4-yl]carbonyl}amino | H | 1 |
| N | Ethanethioylamino | H | 1 |
| N | Methylamino | H | 1 |
| N | Dimethylamino | H | 1 |
| N | Ethylamino | H | 1 |
| N | 1-Methylhydrazino | H | 1 |
| N | Hydrazino | H | 1 |
| N | 1,2-Dimethylhydrazino | H | 1 |
| N | (Methoxycarbonyl)amino | H | 1 |
| N | Carbamoylamino | H | 1 |
| N | (Ethylcarbamoyl)amino | H | 1 |
| N | Methyloxy | H | 1 |
| N | Ethyloxy | H | 1 |
| N | Carbamoyl | H | 1 |
| N | Methylcarbamoyl | H | 1 |
| N | Ethylcarbamoyl | H | 1 |
| N | (Methylamino)carbonothioyl | H | 1 |
| N | Prop-1-en-1-yl | H | 1 |
| N | 2-Cyclopropylvinyl | H | 1 |
| N | 2-Phenylvinyl | H | 1 |
| N | 2-(4-Chlorphenyl)vinyl | H | 1 |
| N | 4-Chlor-3-(pentafluorethyl)-1H-pyrazol-1-yl | H | 1 |
| N | 3-Cyclopropyl-1H-pyrazol-1-yl | H | 1 |
| N | 3-[(Methylsulfonyl)methyl]-1H-pyrazol-1-yl | H | 1 |
| N | 3-[(Methylsulfinyl)methyl]-1H-pyrazol-1-yl | H | 1 |
| N | 4-(Methylsulfanyl)-1H-pyrazol-1-yl | H | 1 |
| N | 3-(Methylsulfanyl)-1H-1,2,4-triazol-1-yl | H | 1 |
| N | 1,3-Dimethyl-5-oxo-1,5-dihydro-4H-1,2,4-triazol-4-yl | H | 1 |
| N | 4-Methyl-5-oxo-4,5-dihydro-1 H-tetrazol- 1 -yl | H | 1 |
| N | 5-Oxo-4-(2,2,2-trifluorethyl)-4,5-dihydro-1H-tetrazol-1-yl | H | 1 |
| N | 5-Oxo-4-(2,2,3,3,3-pentafluorpropyl)-4,5-dihydro-1H-tetrazol-1-yl | H | 1 |
| N | 2-Oxo-1,3-thiazolidin-3-yl | H | 1 |
| N | 5-Chlorpyridin-2-yl | H | 1 |
| N | 2-Chlor-1-methyl-1H-imidazol-5-yl | H | 1 |
| N | 1H-Pyrrol-3-yl | H | 1 |
| N | 1-Methyl-1H-pyrazol-4-yl | H | 1 |
| N | Oxetan-3-ylamino | H | 1 |
| | | | |
| N | acetyl amino | H | 2 |
| N | propanoylamino | H | 2 |
| N | cyclopropancarbonylamino | H | 2 |
| N | (2-cyclopropylacetyl)amino | H | 2 |
| N | 2-methylpropanoylamino | H | 2 |
| N | (2-methoxyacetyl)amino | H | 2 |
| N | benzamido | H | 2 |
| N | (2,2-difluoroacetyl)amino | H | 2 |
| N | (2,2,2-trifluoracetyl)amino | H | 2 |
| N | 2,2,3,3,3-pentafluorpropanoylamino | H | 2 |
| N | 2,2,3,3-tetrafluoropropanoylamino | H | 2 |
| N | methoxycarbonyl | H | 2 |
| N | amino | H | 2 |
| N | morpholin-4-yl | H | 2 |
| N | 3-[(trifluormethyl)sulfanyl]-1H-pyrrol-1-yl | H | 2 |
| N | 4-(methylsulfonyl)-1H-pyrazol-1-yl | H | 2 |
| N | 3-(methylcarbamoyl)-1H-pyrazol-1-yl | H | 2 |
| N | 4-(methylcarbamoyl)-1H-pyrazol-1-yl | H | 2 |
| N | 3-(methylsulfonyl)-1H-1,2,4-triazol-1-yl | H | 2 |
| N | 4-chlor-1H-pyrazol-1-yl | H | 2 |
| N | 3-chlor-1H-pyrazol-1-yl | H | 2 |
| N | 4-iod-1H-pyrazol-1-yl | H | 2 |
| N | 3-iod-1H-pyrazol-1-yl | H | 2 |
| N | 3-fluor-1H-pyrazol-1-yl | H | 2 |
| N | 4-brom-1H-pyrazol-1-yl | H | 2 |
| N | 3-brom-1H-pyrazol-1-yl | H | 2 |
| N | 3-(cyanomethyl)-1H-pyrazol-1-yl | H | 2 |
| N | 3-(difluormethyl)-1H-pyrazol-1-yl | H | 2 |
| N | 3-(methoxymethyl)-1H-pyrazol-1-yl | H | 2 |
| N | 3-acetamido-1H-1,2,4-triazol-1-yl | H | 2 |
| N | 4-cyclopropyl-1H-pyrazol-1-yl | H | 2 |
| N | 4-iodo-1H-imidazol-1-yl | H | 2 |
| N | 3-(methylsulfanyl)-1H-1,2,4-triazol-1-yl | H | 2 |
| N | 4-(methylsulfanyl)-1H-pyrazol-1-yl | H | 2 |
| N | 2H-1,2,3-triazol-2-yl | H | 2 |
| N | 1H-1,2,4-triazol-1-yl | H | 2 |
| N | 3-chlor-1H-1,2,4-triazol-1-yl | H | 2 |
| N | 4-cyano-1H-pyrazol-1-yl | H | 2 |
| N | 3-thienyl | H | 2 |
| N | 1H-pyrazol-1-yl | H | 2 |
| N | 3-cyano-1H-pyrazol-1-yl | H | 2 |
| N | 3-acetamido-1H-pyrazol-1-yl | H | 2 |
| N | 4-(trifluormethyl)-1H-1,2,3-triazol-1-yl | H | 2 |
| N | 3-(trifluormethyl)-1H-pyrazol-1-yl | H | 2 |
| N | 4-(trifluormethyl)-1H-pyrazol-1-yl | H | 2 |
| N | 4-(trifluormethyl)-1H-imidazol-1-yl | H | 2 |
| N | 4-fluor-1H-pyrazol-1-yl | H | 2 |
| N | 3-(methylsulfanyl)-1H-pyrazol-1-yl | H | 2 |
| N | 4-(trifluormethyl)-2H-1,2,3-triazol-2-yl | H | 2 |
| N | 4-methyl-1H-pyrazol-1-yl | H | 2 |
| N | 1H-imidazol-1-yl | H | 2 |
| N | 3-methyl-1H-pyrazol-1-yl | H | 2 |
| N | 3-(methylsulfonyl)-1H-pyrazol-1-yl | H | 2 |
| N | (Pyridin-2-ylcarbonyl)amino | H | 2 |
| N | [2-Methyl-3-(methylsulfinyl)propanoyl]amino | H | 2 |
| N | [(Methylsulfanyl)acetyl] amino | H | 2 |
| N | [2-Methyl-3-(methylsulfonyl)propanoyl]amino | H | 2 |
| N | [(Methylsulfonyl) acetyl]amino | H | 2 |
| N | [2-Methyl-3-(methylsulfanyl)propanoyl]amino | H | 2 |
| N | [(2-Methyl-1,3-thiazol-4-yl)carbonyl]amino | H | 2 |
| N | [(1-Methyl-1H-pyrazol-3-yl)carbonyl]amino | H | 2 |
| N | (1,3-Thiazol-2-ylcarbonyl)amino | H | 2 |
| N | (1,3-Thiazol-4-ylcarbonyl)amino | H | 2 |
| N | {[2-(Trifluormethyl)-1,3-thiazol-4-yl]carbonyl}amino | H | 2 |
| N | Ethanethioylamino | H | 2 |
| N | Methylamino | H | 2 |
| N | Dimethylamino | H | 2 |
| N | Ethylamino | H | 2 |
| N | 1-Methylhydrazino | H | 2 |
| N | Hydrazino | H | 2 |
| N | 1,2-Dimethylhydrazino | H | 2 |
| N | (Methoxycarbonyl)amino | H | 2 |
| N | Carbamoylamino | H | 2 |
| N | (Ethylcarbamoyl)amino | H | 2 |
| N | Methyloxy | H | 2 |
| N | Ethyloxy | H | 2 |
| N | Carbamoyl | H | 2 |
| N | Methylcarbamoyl | H | 2 |
| N | Ethylcarbamoyl | H | 2 |
| N | (Methylamino)carbonothioyl | H | 2 |
| N | Prop-1-en-1-yl | H | 2 |
| N | 2-Cyclopropylvinyl | H | 2 |
| N | 2-Phenylvinyl | H | 2 |
| N | 2-(4-Chlorphenyl)vinyl | H | 2 |
| N | 4-Chlor-3-(pentafluorethyl)-1H-pyrazol-1-yl | H | 2 |
| N | 3-Cyclopropyl-1H-pyrazol-1-yl | H | 2 |
| N | 3-[(Methylsulfonyl)methyl]-1H-pyrazol-1-yl | H | 2 |
| N | 3-[(Methylsulfinyl)methyl]-1H-pyrazol-1-yl | H | 2 |
| N | 4-(Methylsulfanyl)-1H-pyrazol-1-yl | H | 2 |
| N | 3-(Methylsulfanyl)-1H-1,2,4-triazol-1-yl | H | 2 |
| N | 1,3-Dimethyl-5-oxo-1,5-dihydro-4H-1,2,4-triazol-4-yl | H | 2 |
| N | 4-Methyl-5-oxo-4,5-dihydro-1 H-tetrazol-1-yl | H | 2 |
| N | 5-Oxo-4-(2,2,2-trifluorethyl)-4,5-dihydro-1H-tetrazol-1-yl | H | 2 |
| N | 5-Oxo-4-(2,2,3,3,3-pentafluorpropyl)-4,5-dihydro-1H-tetrazol-1-yl | H | 2 |
| N | 2-Oxo-1,3-thiazolidin-3-yl | H | 2 |
| N | 5-Chlorpyridin-2-yl | H | 2 |
| N | 2-Chlor-1-methyl-1H-imidazol-5-yl | H | 2 |
| N | 1H-Pyrrol-3-yl | H | 2 |
| N | 1-Methyl-1H-pyrazol-4-yl | H | 2 |
| N | Oxetan-3-ylamino | H | 2 |
| | | | |
| C-H | acetyl amino | H | 0 |
| C-H | propanoylamino | H | 0 |
| C-H | cyclopropancarbonylamino | H | 0 |
| C-H | (2-cyclopropylacetyl)amino | H | 0 |
| C-H | 2-methylpropanoylamino | H | 0 |
| C-H | (2-methoxyacetyl) amino | H | 0 |
| C-H | benzamido | H | 0 |
| C-H | (2,2-difluoroacetyl)amino | H | 0 |
| C-H | (2,2,2-trifluoracetyl)amino | H | 0 |
| C-H | 2,2,3,3,3-pentafluorpropanoylamino | H | 0 |
| C-H | 2,2,3,3-tetrafluoropropanoylamino | H | 0 |
| C-H | methoxycarbonyl | H | 0 |
| C-H | amino | H | 0 |
| C-H | morpholin-4-yl | H | 0 |
| C-H | 3-[(trifluormethyl)sulfanyl]-1H-pyrrol-1-yl | H | 0 |
| C-H | 4-(methylsulfonyl)-1H-pyrazol-1-yl | H | 0 |
| C-H | 3-(methylcarbamoyl)-1H-pyrazol-1-yl | H | 0 |
| C-H | 4-(methylcarbamoyl)-1H-pyrazol-1-yl | H | 0 |
| C-H | 3-(methylsulfonyl)-1H-1,2,4-triazol-1-yl | H | 0 |
| C-H | 4-chlor-1H-pyrazol-1-yl | H | 0 |
| C-H | 3-chlor-1H-pyrazol-1-yl | H | 0 |
| C-H | 4-iod-1H-pyrazol-1-yl | H | 0 |
| C-H | 3-iod-1H-pyrazol-1-yl | H | 0 |
| C-H | 3-fluor-1H-pyrazol-1-yl | H | 0 |
| C-H | 4-brom-1H-pyrazol-1-yl | H | 0 |
| C-H | 3-brom-1H-pyrazol-1-yl | H | 0 |
| C-H | 3-(cyanomethyl)-1H-pyrazol-1-yl | H | 0 |
| C-H | 3-(difluormethyl)-1H-pyrazol-1-yl | H | 0 |
| C-H | 3-(methoxymethyl)-1H-pyrazol-1-yl | H | 0 |
| C-H | 3-acetamido-1H-1,2,4-triazol-1-yl | H | 0 |
| C-H | 4-cyclopropyl-1H-pyrazol-1-yl | H | 0 |
| C-H | 4-iodo-1H-imidazol-1-yl | H | 0 |
| C-H | 3-(methylsulfanyl)-1H-1,2,4-triazol-1-yl | H | 0 |
| C-H | 4-(methylsulfanyl)-1H-pyrazol-1-yl | H | 0 |
| C-H | 2H-1,2,3-triazol-2-yl | H | 0 |
| C-H | 1H-1,2,4-triazol-1-yl | H | 0 |
| C-H | 3-chlor-1H-1,2,4-triazol-1-yl | H | 0 |
| C-H | 4-cyano-1H-pyrazol-1-yl | H | 0 |
| C-H | 3-thienyl | H | 0 |
| C-H | 1H-pyrazol-1-yl | H | 0 |
| C-H | 3-cyano-1H-pyrazol-1-yl | H | 0 |
| C-H | 3-acetamido-1H-pyrazol-1-yl | H | 0 |
| C-H | 4-(trifluormethyl)-1H-1,2,3-triazol-1-yl | H | 0 |
| C-H | 3-(trifluormethyl)-1H-pyrazol-1-yl | H | 0 |
| C-H | 4-(trifluormethyl)-1H-pyrazol-1-yl | H | 0 |
| C-H | 4-(trifluormethyl)-1H-imidazol-1-yl | H | 0 |
| C-H | 4-fluor-1H-pyrazol-1-yl | H | 0 |
| C-H | 3-(methylsulfanyl)-1H-pyrazol-1-yl | H | 0 |
| C-H | 4-(trifluormethyl)-2H-1,2,3-triazol-2-yl | H | 0 |
| C-H | 4-methyl-1H-pyrazol-1-yl | H | 0 |
| C-H | 1H-imidazol-1-yl | H | 0 |
| C-H | 3-methyl-1H-pyrazol-1-yl | H | 0 |
| C-H | 3-(methylsulfonyl)-1H-pyrazol-1-yl | H | 0 |
| C-H | (Pyridin-2-ylcarbonyl)amino | H | 0 |
| C-H | [2-Methyl-3-(methylsulfinyl)propanoyl]amino | H | 0 |
| C-H | [(Methylsulfanyl)acetyl]amino | H | 0 |
| C-H | [2-Methyl-3-(methylsulfonyl)propanoyl]amino | H | 0 |
| C-H | [(Methylsulfonyl)acetyl]amino | H | 0 |
| C-H | [2-Methyl-3-(methylsulfanyl)propanoyl]amino | H | 0 |
| C-H | [(2-Methyl-1,3-thiazol-4-yl)carbonyl]amino | H | 0 |
| C-H | [(1-Methyl-1H-pyrazol-3-yl)carbonyl]amino | H | 0 |
| C-H | (1,3-Thiazol-2-ylcarbonyl)amino | H | 0 |
| C-H | (1,3-Thiazol-4-ylcarbonyl)amino | H | 0 |
| C-H | {[2-(Trifluormethyl)-1,3-thiazol-4-yl]carbonyl}amino | H | 0 |
| C-H | Ethanethioylamino | H | 0 |
| C-H | Methylamino | H | 0 |
| C-H | Dimethylamino | H | 0 |
| C-H | Ethylamino | H | 0 |
| C-H | 1-Methylhydrazino | H | 0 |
| C-H | Hydrazino | H | 0 |
| C-H | 1,2-Dimethylhydrazino | H | 0 |
| C-H | (Methoxycarbonyl)amino | H | 0 |
| C-H | Carbamoylamino | H | 0 |
| C-H | (Ethylcarbamoyl)amino | H | 0 |
| C-H | Methyloxy | H | 0 |
| C-H | Ethyloxy | H | 0 |
| C-H | Carbamoyl | H | 0 |
| C-H | Methylcarbamoyl | H | 0 |
| C-H | Ethylcarbamoyl | H | 0 |
| C-H | (Methylamino)carbonothioyl | H | 0 |
| C-H | Prop-1-en-1-yl | H | 0 |
| C-H | 2-Cyclopropylvinyl | H | 0 |
| C-H | 2-Phenylvinyl | H | 0 |
| C-H | 2-(4-Chlorphenyl)vinyl | H | 0 |
| C-H | 4-Chlor-3-(pentafluorethyl)-1H-pyrazol-1-yl | H | 0 |
| C-H | 3-Cyclopropyl-1H-pyrazol-1-yl | H | 0 |
| C-H | 3-[(Methylsulfonyl)methyl]-1H-pyrazol-1-yl | H | 0 |
| C-H | 3-[(Methylsulfinyl)methyl]-1H-pyrazol-1-yl | H | 0 |
| C-H | 4-(Methylsulfanyl)-1H-pyrazol-1-yl | H | 0 |
| C-H | 3-(Methylsulfanyl)-1H-1,2,4-triazol-1-yl | H | 0 |
| C-H | 1,3-Dimethyl-5-oxo-1,5-dihydro-4H-1,2,4-triazol-4-yl | H | 0 |
| C-H | 4-Methyl-5-oxo-4,5-dihydro-1 H-tetrazol-1-yl | H | 0 |
| C-H | 5-Oxo-4-(2,2,2-trifluorethyl)-4,5-dihydro-1H-tetrazol-1-yl | H | 0 |
| C-H | 5-Oxo-4-(2,2,3,3,3-pentafluorpropyl)-4,5-dihydro-1H-tetrazol-1-yl | H | 0 |
| C-H | 2-Oxo-1,3-thiazolidin-3-yl | H | 0 |
| C-H | 5-Chlorpyridin-2-yl | H | 0 |
| C-H | 2-Chlor-1-methyl-1H-imidazol-5-yl | H | 0 |
| C-H | 1H-Pyrrol-3-yl | H | 0 |
| C-H | 1-Methyl-1H-pyrazol-4-yl | H | 0 |
| C-H | Oxetan-3-ylamino | H | 0 |
| | | | |
| C-H | acetyl amino | H | 1 |
| C-H | propanoylamino | H | 1 |
| C-H | cyclopropancarbonylamino | H | 1 |
| C-H | (2-cyclopropylacetyl)amino | H | 1 |
| C-H | 2-methylpropanoylamino | H | 1 |
| C-H | (2-methoxyacetyl) amino | H | 1 |
| C-H | benzamido | H | 1 |
| C-H | (2,2-difluoroacetyl)amino | H | 1 |
| C-H | (2,2,2-trifluoracetyl)amino | H | 1 |
| C-H | 2,2,3,3,3-pentafluorpropanoylamino | H | 1 |
| C-H | 2,2,3,3-tetrafluoropropanoylamino | H | 1 |
| C-H | methoxycarbonyl | H | 1 |
| C-H | amino | H | 1 |
| C-H | morpholin-4-yl | H | 1 |
| C-H | 3-[(trifluormethyl)sulfanyl]-1H-pyrrol-1-yl | H | 1 |
| C-H | 4-(methylsulfonyl)-1H-pyrazol-1-yl | H | 1 |
| C-H | 3-(methylcarbamoyl)-1H-pyrazol-1-yl | H | 1 |
| C-H | 4-(methylcarbamoyl)-1H-pyrazol-1-yl | H | 1 |
| C-H | 3-(methylsulfonyl)-1H-1,2,4-triazol-1-yl | H | 1 |
| C-H | 4-chlor-1H-pyrazol-1-yl | H | 1 |
| C-H | 3-chlor-1H-pyrazol-1-yl | H | 1 |
| C-H | 4-iod-1H-pyrazol-1-yl | H | 1 |
| C-H | 3-iod-1H-pyrazol-1-yl | H | 1 |
| C-H | 3-fluor-1H-pyrazol-1-yl | H | 1 |
| C-H | 4-brom-1H-pyrazol-1-yl | H | 1 |
| C-H | 3-brom-1H-pyrazol-1-yl | H | 1 |
| C-H | 3-(cyanomethyl)-1H-pyrazol-1-yl | H | 1 |
| C-H | 3-(difluormethyl)-1H-pyrazol-1-yl | H | 1 |
| C-H | 3-(methoxymethyl)-1H-pyrazol-1-yl | H | 1 |
| C-H | 3-acetamido-1H-1,2,4-triazol-1-yl | H | 1 |
| C-H | 4-cyclopropyl-1H-pyrazol-1-yl | H | 1 |
| C-H | 4-iodo-1H-imidazol-1-yl | H | 1 |
| C-H | 3-(methylsulfanyl)-1H-1,2,4-triazol-1-yl | H | 1 |
| C-H | 4-(methylsulfanyl)-1H-pyrazol-1-yl | H | 1 |
| C-H | 2H-1,2,3-triazol-2-yl | H | 1 |
| C-H | 1H-1,2,4-triazol-1-yl | H | 1 |
| C-H | 3-chlor-1H-1,2,4-triazol-1-yl | H | 1 |
| C-H | 4-cyano-1H-pyrazol-1-yl | H | 1 |
| C-H | 3-thienyl | H | 1 |
| C-H | 1H-pyrazol-1-yl | H | 1 |
| C-H | 3-cyano-1H-pyrazol-1-yl | H | 1 |
| C-H | 3-acetamido-1H-pyrazol-1-yl | H | 1 |
| C-H | 4-(trifluormethyl)-1H-1,2,3-triazol-1-yl | H | 1 |
| C-H | 3-(trifluormethyl)-1H-pyrazol-1-yl | H | 1 |
| C-H | 4-(trifluormethyl)-1H-pyrazol-1-yl | H | 1 |
| C-H | 4-(trifluormethyl)-1H-imidazol-1-yl | H | 1 |
| C-H | 4-fluor-1H-pyrazol-1-yl | H | 1 |
| C-H | 3-(methylsulfanyl)-1H-pyrazol-1-yl | H | 1 |
| C-H | 4-(trifluormethyl)-2H-1,2,3-triazol-2-yl | H | 1 |
| C-H | 4-methyl-1H-pyrazol-1-yl | H | 1 |
| C-H | 1H-imidazol-1-yl | H | 1 |
| C-H | 3-methyl-1H-pyrazol-1-yl | H | 1 |
| C-H | 3-(methylsulfonyl)-1H-pyrazol-1-yl | H | 1 |
| C-H | (Pyridin-2-ylcarbonyl)amino | H | 1 |
| C-H | [2-Methyl-3-(methylsulfinyl)propanoyl]amino | H | 1 |
| C-H | [(Methylsulfanyl)acetyl] amino | H | 1 |
| C-H | [2-Methyl-3-(methylsulfonyl)propanoyl]amino | H | 1 |
| C-H | [(Methylsulfonyl)acetyl]amino | H | 1 |
| C-H | [2-Methyl-3-(methylsulfanyl)propanoyl]amino | H | 1 |
| C-H | [(2-Methyl-1,3-thiazol-4-yl)carbonyl]amino | H | 1 |
| C-H | [(1-Methyl-1H-pyrazol-3-yl)carbonyl]amino | H | 1 |
| C-H | (1,3-Thiazol-2-ylcarbonyl)amino | H | 1 |
| C-H | (1,3-Thiazol-4-ylcarbonyl)amino | H | 1 |
| C-H | {[2-(Trifluormethyl)-1,3-thiazol-4-yl]carbonyl}amino | H | 1 |
| C-H | Ethanethioylamino | H | 1 |
| C-H | Methylamino | H | 1 |
| C-H | Dimethylamino | H | 1 |
| C-H | Ethylamino | H | 1 |
| C-H | 1-Methylhydrazino | H | 1 |
| C-H | Hydrazino | H | 1 |
| C-H | 1,2-Dimethylhydrazino | H | 1 |
| C-H | (Methoxycarbonyl)amino | H | 1 |
| C-H | Carbamoylamino | H | 1 |
| C-H | (Ethylcarbamoyl)amino | H | 1 |
| C-H | Methyloxy | H | 1 |
| C-H | Ethyloxy | H | 1 |
| C-H | Carbamoyl | H | 1 |
| C-H | Methylcarbamoyl | H | 1 |
| C-H | Ethylcarbamoyl | H | 1 |
| C-H | (Methylamino)carbonothioyl | H | 1 |
| C-H | Prop-1-en-1-yl | H | 1 |
| C-H | 2-Cyclopropylvinyl | H | 1 |
| C-H | 2-Phenylvinyl | H | 1 |
| C-H | 2-(4-Chlorphenyl)vinyl | H | 1 |
| C-H | 4-Chlor-3-(pentafluorethyl)-1H-pyrazol-1-yl | H | 1 |
| C-H | 3-Cyclopropyl-1H-pyrazol-1-yl | H | 1 |
| C-H | 3-[(Methylsulfonyl)methyl]-1H-pyrazol-1-yl | H | 1 |
| C-H | 3-[(Methylsulfinyl)methyl]-1H-pyrazol-1-yl | H | 1 |
| C-H | 4-(Methylsulfanyl)-1H-pyrazol-1-yl | H | 1 |
| C-H | 3-(Methylsulfanyl)-1H-1,2,4-triazol-1-yl | H | 1 |
| C-H | 1,3-Dimethyl-5-oxo-1,5-dihydro-4H-1,2,4-triazol-4-yl | H | 1 |
| C-H | 4-Methyl-5-oxo-4,5-dihydro-1 H-tetrazol-1-yl | H | 1 |
| C-H | 5-Oxo-4-(2,2,2-trifluorethyl)-4,5-dihydro-1H-tetrazol-1-yl | H | 1 |
| C-H | 5-Oxo-4-(2,2,3,3,3-pentafluorpropyl)-4,5-dihydro-1H-tetrazol-1-yl | H | 1 |
| C-H | 2-Oxo-1,3-thiazolidin-3-yl | H | 1 |
| C-H | 5-Chlorpyridin-2-yl | H | 1 |
| C-H | 2-Chlor-1-methyl-1H-imidazol-5-yl | H | 1 |
| C-H | 1H-Pyrrol-3-yl | H | 1 |
| C-H | 1-Methyl-1H-pyrazol-4-yl | H | 1 |
| C-H | Oxetan-3-ylamino | H | 1 |
| | | | |
| C-H | acetyl amino | H | 2 |
| C-H | propanoylamino | H | 2 |
| C-H | cyclopropancarbonylamino | H | 2 |
| C-H | (2-cyclopropylacetyl)amino | H | 2 |
| C-H | 2- methylpropanoylamino | H | 2 |
| C-H | (2-methoxyacetyl) amino | H | 2 |
| C-H | benzamido | H | 2 |
| C-H | (2,2-difluoroacetyl)amino | H | 2 |
| C-H | (2,2,2-trifluoracetyl)amino | H | 2 |
| C-H | 2,2,3,3,3-pentafluorpropanoylamino | H | 2 |
| C-H | 2,2,3,3-tetrafluoropropanoylamino | H | 2 |
| C-H | methoxycarbonyl | H | 2 |
| C-H | amino | H | 2 |
| C-H | morpholin-4-yl | H | 2 |
| C-H | 3-[(trifluormethyl)sulfanyl]-1H-pyrrol-1-yl | H | 2 |
| C-H | 4-(methylsulfonyl)-1H-pyrazol-1-yl | H | 2 |
| C-H | 3-(methylcarbamoyl)-1H-pyrazol-1-yl | H | 2 |
| C-H | 4-(methylcarbamoyl)-1H-pyrazol-1-yl | H | 2 |
| C-H | 3-(methylsulfonyl)-1H-1,2,4-triazol-1-yl | H | 2 |
| C-H | 4-chlor-1H-pyrazol-1-yl | H | 2 |
| C-H | 3-chlor-1H-pyrazol-1-yl | H | 2 |
| C-H | 4-iod-1H-pyrazol-1-yl | H | 2 |
| C-H | 3-iod-1H-pyrazol-1-yl | H | 2 |
| C-H | 3-fluor-1H-pyrazol-1-yl | H | 2 |
| C-H | 4-brom-1H-pyrazol-1-yl | H | 2 |
| C-H | 3-brom-1H-pyrazol-1-yl | H | 2 |
| C-H | 3-(cyanomethyl)-1H-pyrazol-1-yl | H | 2 |
| C-H | 3-(difluormethyl)-1H-pyrazol-1-yl | H | 2 |
| C-H | 3-(methoxymethyl)-1H-pyrazol-1-yl | H | 2 |
| C-H | 3-acetamido-1H-1,2,4-triazol-1-yl | H | 2 |
| C-H | 4-cyclopropyl-1H-pyrazol-1-yl | H | 2 |
| C-H | 4-iodo-1H-imidazol-1-yl | H | 2 |
| C-H | 3-(methylsulfanyl)-1H-1,2,4-triazol-1-yl | H | 2 |
| C-H | 4-(methylsulfanyl)-1H-pyrazol-1-yl | H | 2 |
| C-H | 2H-1,2,3-triazol-2-yl | H | 2 |
| C-H | 1H-1,2,4-triazol-1-yl | H | 2 |
| C-H | 3-chlor-1H-1,2,4-triazol-1-yl | H | 2 |
| C-H | 4-cyano-1H-pyrazol-1-yl | H | 2 |
| C-H | 3-thienyl | H | 2 |
| C-H | 1H-pyrazol-1-yl | H | 2 |
| C-H | 3-cyano-1H-pyrazol-1-yl | H | 2 |
| C-H | 3-acetamido-1H-pyrazol-1-yl | H | 2 |
| C-H | 4-(trifluormethyl)-1H-1,2,3-triazol-1-yl | H | 2 |
| C-H | 3-(trifluormethyl)-1H-pyrazol-1-yl | H | 2 |
| C-H | 4-(trifluormethyl)-1H-pyrazol-1-yl | H | 2 |
| C-H | 4-(trifluormethyl)-1H-imidazol-1-yl | H | 2 |
| C-H | 4-fluor-1H-pyrazol-1-yl | H | 2 |
| C-H | 3-(methylsulfanyl)-1H-pyrazol-1-yl | H | 2 |
| C-H | 4-(trifluormethyl)-2H-1,2,3-triazol-2-yl | H | 2 |
| C-H | 4-methyl-1H-pyrazol-1-yl | H | 2 |
| C-H | 1H-imidazol-1-yl | H | 2 |
| C-H | 3-methyl-1H-pyrazol-1-yl | H | 2 |
| C-H | 3-(methylsulfonyl)-1H-pyrazol-1-yl | H | 2 |
| C-H | (Pyridin-2-ylcarbonyl)amino | H | 2 |
| C-H | [2-Methyl-3-(methylsulfinyl)propanoyl]amino | H | 2 |
| C-H | [(Methylsulfanyl)acetyl] amino | H | 2 |
| C-H | [2-Methyl-3-(methylsulfonyl)propanoyl]amino | H | 2 |
| C-H | [(Methylsulfonyl)acetyl]amino | H | 2 |
| C-H | [2-Methyl-3-(methylsulfanyl)propanoyl]amino | H | 2 |
| C-H | [(2-Methyl-1,3-thiazol-4-yl)carbonyl]amino | H | 2 |
| C-H | [(1-Methyl-1H-pyrazol-3-yl)carbonyl]amino | H | 2 |
| C-H | (1,3-Thiazol-2-ylcarbonyl)amino | H | 2 |
| C-H | (1,3-Thiazol-4-ylcarbonyl)amino | H | 2 |
| C-H | {[2-(Trifluormethyl)-1,3-thiazol-4-yl]carbonyl}amino | H | 2 |
| C-H | Ethanethioylamino | H | 2 |
| C-H | Methylamino | H | 2 |
| C-H | Dimethylamino | H | 2 |
| C-H | Ethylamino | H | 2 |
| C-H | 1-Methylhydrazino | H | 2 |
| C-H | Hydrazino | H | 2 |
| C-H | 1,2-Dimethylhydrazino | H | 2 |
| C-H | (Methoxycarbonyl)amino | H | 2 |
| C-H | Carbamoylamino | H | 2 |
| C-H | (Ethylcarbamoyl)amino | H | 2 |
| C-H | Methyloxy | H | 2 |
| C-H | Ethyloxy | H | 2 |
| C-H | Carbamoyl | H | 2 |
| C-H | Methylcarbamoyl | H | 2 |
| C-H | Ethylcarbamoyl | H | 2 |
| C-H | (Methylamino)carbonothioyl | H | 2 |
| C-H | Prop-1-en-1-yl | H | 2 |
| C-H | 2-Cyclopropylvinyl | H | 2 |
| C-H | 2-Phenylvinyl | H | 2 |
| C-H | 2-(4-Chlorphenyl)vinyl | H | 2 |
| C-H | 4-Chlor-3-(pentafluorethyl)-1H-pyrazol-1-yl | H | 2 |
| C-H | 3-Cyclopropyl-1H-pyrazol-1-yl | H | 2 |
| C-H | 3-[(Methylsulfonyl)methyl]-1H-pyrazol-1-yl | H | 2 |
| C-H | 3-[(Methylsulfinyl)methyl]-1H-pyrazol-1-yl | H | 2 |
| C-H | 4-(Methylsulfanyl)-1H-pyrazol-1-yl | H | 2 |
| C-H | 3-(Methylsulfanyl)-1H-1,2,4-triazol-1-yl | H | 2 |
| C-H | 1,3-Dimethyl-5-oxo-1,5-dihydro-4H-1,2,4-triazol-4-yl | H | 2 |
| C-H | 4-Methyl-5-oxo-4,5-dihydro-1 H-tetrazol-1-yl | H | 2 |
| C-H | 5-Oxo-4-(2,2,2-trifluorethyl)-4,5-dihydro-1H-tetrazol-1-yl | H | 2 |
| C-H | 5-Oxo-4-(2,2,3,3,3-pentafluorpropyl)-4,5-dihydro-1H-tetrazol-1-yl | H | 2 |
| C-H | 2-Oxo-1,3-thiazolidin-3-yl | H | 2 |
| C-H | 5-Chlorpyridin-2-yl | H | 2 |
| C-H | 2-Chlor-1-methyl-1H-imidazol-5-yl | H | 2 |
| C-H | 1H-Pyrrol-3-yl | H | 2 |
| C-H | 1-Methyl-1H-pyrazol-4-yl | H | 2 |
| C-H | Oxetan-3-ylamino | H | 2 |

Im einzelnen seien außerdem die folgenden Verbindungen der Formel (I-B) genannt: 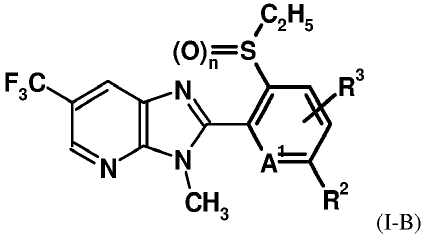 A¹, R², R³ und n wie in Tabelle 2 genannt.

Im einzelnen seien außerdem die folgenden Verbindungen der Formel (I-C) genannt: 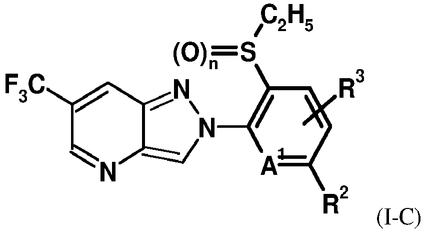 A¹, R², R³ und n wie in Tabelle 2 genannt.

Im einzelnen seien außerdem die folgenden Verbindungen der Formel (I-D) genannt: 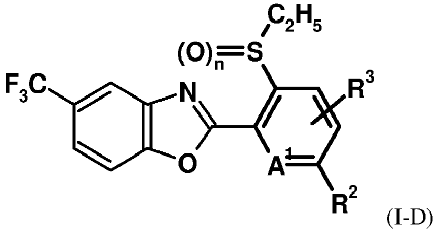 A¹, R², R³ und n wie in Tabelle 2 genannt.

Im einzelnen seien außerdem die folgenden Verbindungen der Formel (I-E) genannt: 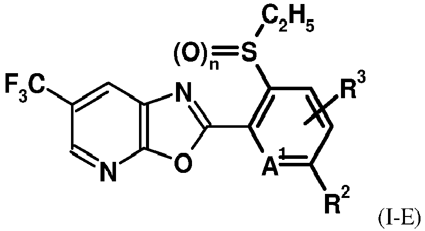 A¹, R², R³ und n wie in Tabelle 2 genannt.

Im einzelnen seien außerdem die folgenden Verbindungen der Formel (I-F) genannt: 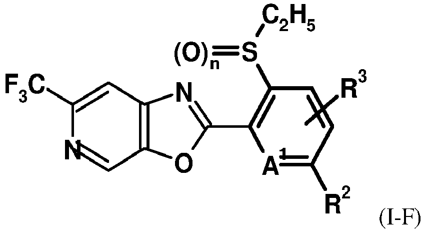 A¹, R², R³ und n wie in Tabelle 2 genannt.

Im einzelnen seien außerdem die folgenden Verbindungen der Formel (I-G) genannt: 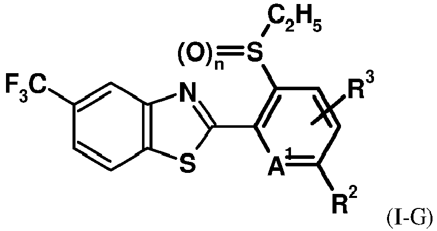 A¹, R², R³ und n wie in Tabelle 2 genannt.

Im einzelnen seien außerdem die folgenden Verbindungen der Formel (I-H) genannt: 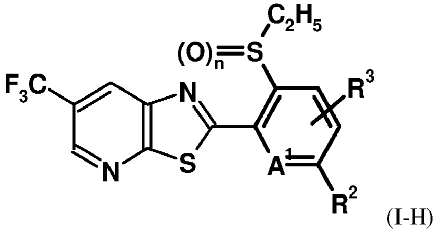 A¹, R², R³ und n wie in Tabelle 2 genannt.

Im einzelnen seien außerdem die folgenden Verbindungen der Formel (I-I) genannt: 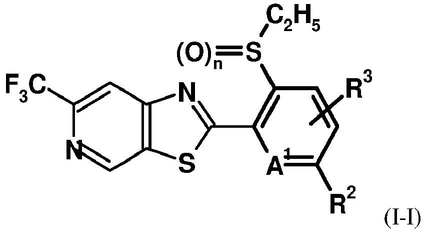 A¹, R², R³ und n wie in Tabelle 2 genannt.

Im einzelnen seien außerdem die folgenden Verbindungen der Formel (I-J) genannt: 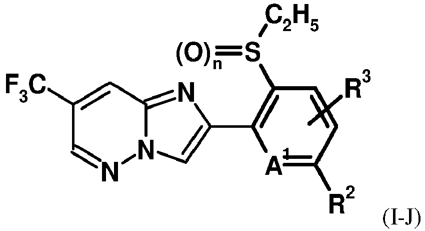 A¹, R², R³ und n wie in Tabelle 2 genannt.

Im einzelnen seien außerdem die folgenden Verbindungen der Formel (I-K) genannt: 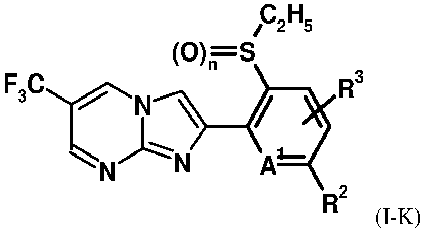 A¹, R², R³ und n wie in Tabelle 2 genannt.

Im einzelnen seien außerdem die folgenden Verbindungen der Formel (I-L) genannt: 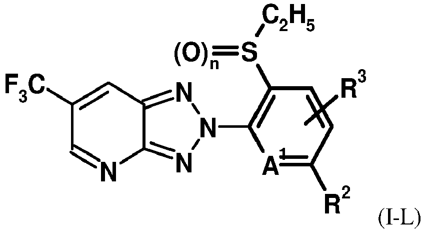 A¹, R², R³ und n wie in Tabelle 2 genannt.

Im einzelnen seien außerdem die folgenden Verbindungen der Formel (I-M) genannt: 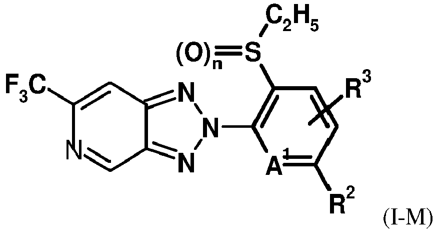 A¹, R², R³ und n wie in Tabelle 2 genannt.

Im einzelnen seien außerdem die folgenden Verbindungen der Formel (I-N) genannt: 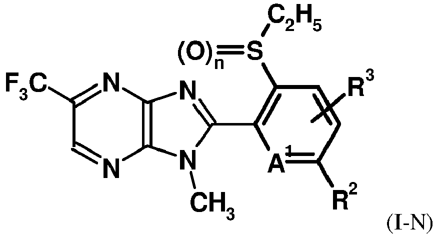 A¹, R², R³ und n wie in Tabelle 2 genannt.

Im einzelnen seien außerdem die folgenden Verbindungen der Formel (I-O) genannt: 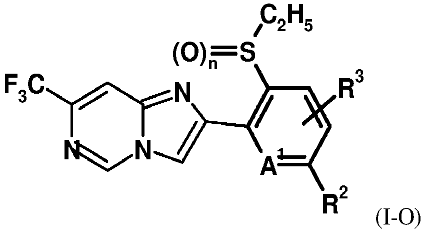 A¹, R², R³ und n wie in Tabelle 2 genannt.

Im einzelnen seien außerdem die folgenden Verbindungen der Formel (I-P) genannt: 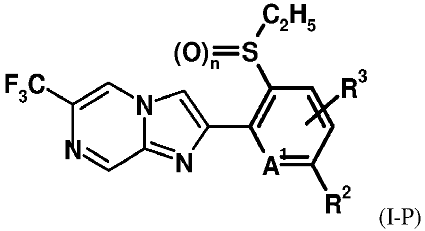 A¹, R², R³ und n wie in Tabelle 2 genannt.

Im einzelnen seien außerdem die folgenden Verbindungen der Formel (I-Q) genannt: 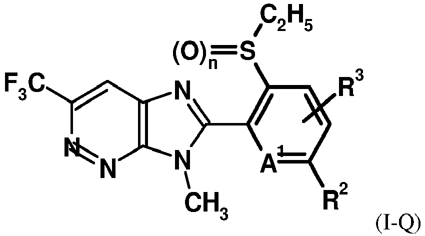 A¹, R², R³ und n wie in Tabelle 2 genannt.

Im einzelnen seien außerdem die folgenden Verbindungen der Formel (I-R) genannt: 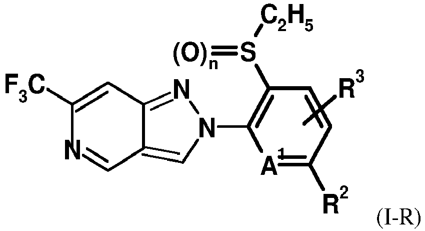 A¹, R², R³ und n wie in Tabelle 2 genannt.

Im einzelnen seien außerdem die folgenden Verbindungen der Formel (I-S) genannt: 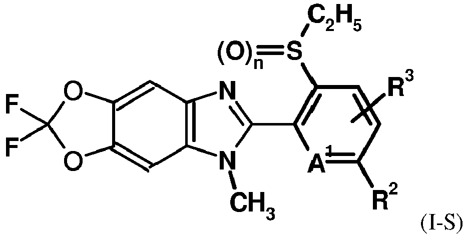 A¹, R², R³ und n wie in Tabelle 2 genannt.

Im einzelnen seien außerdem die folgenden Verbindungen der Formel (I-T) genannt: 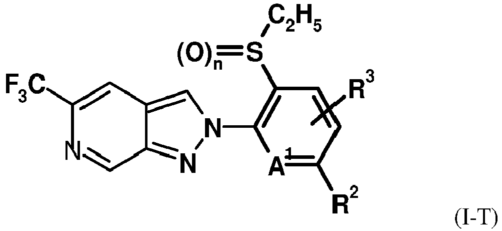 A¹, R², R³ und n wie in Tabelle 2 genannt.

In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, Pyridyl, Pyrimidyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 4-, 5- oder 6-Ring, der 1 oder 2 Stickstoffatome und/oder ein Sauerstoffatom und/oder ein Schwefelatom enthält, beispielsweise für Azetidinyl, Pyrrolidinyl, Piperidinyl, Oxetanyl, Tetrahydrofuranyl, Tetrahydropyranyl, Dioxanyl, Thietanyl, Tetrahydrothiophenyl, Tetrahydrothiopyranyl, Piperazinyl, Morpholinyl und Thiomorpholinyl.

In den besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (auch als Teil einer größeren Einheit, wie beispielsweise Hetarylalkyl) ausgewählt aus der Reihe Pyridyl, Pyrimidyl, Pyrazinyl, Pyridazinyl, Pyrazolyl, Imidazolyl, Triazolyl, Thiazolyl und Tetrazol,
Heterocyclyl ausgewählt aus der Reihe Oxetanyl, Tetrahydrofuryl und Piperazinyl.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

Erfindungsgemäß hervorgehoben verwendet werden Verbindungen der Formel (I), in welchen eine Kombination der vorstehend als hervorgehoben aufgeführten Bedeutungen vorliegt.

Erfindungsgemäß insbesondere verwendet werden Verbindungen der Formel (I), in welchen eine Kombination der vorstehend als besonders hervorgehoben aufgeführten Bedeutungen vorliegt.

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen der Formel (I) können durch die in den folgenden Schemata dargestellten Verfahren erhalten werden:

### Verfahren A

Die Verbindungen der Formel (I), bei denen X für H1, H2, H4, H5, H6, H7, H8, H9, H14 oder H19 steht, können nach bekannten Methoden hergestellt werden, beispielsweise analog der in WO2009/131237, WO2010/125985, WO2011/043404, WO2011/040629, WO2012/086848, WO2013/018928 oder WO2015/000715 beschriebenen Verfahren.

Das allgemeine Verfahren für die Herstellung von Verbindungen der Formel (I), bei denen X für H1, H2, H4, H5, H6, H7, H8, H9, H14 oder H19 steht, wird nachfolgend anhand von Beispiel H2, H5 bzw. H8 beschrieben.

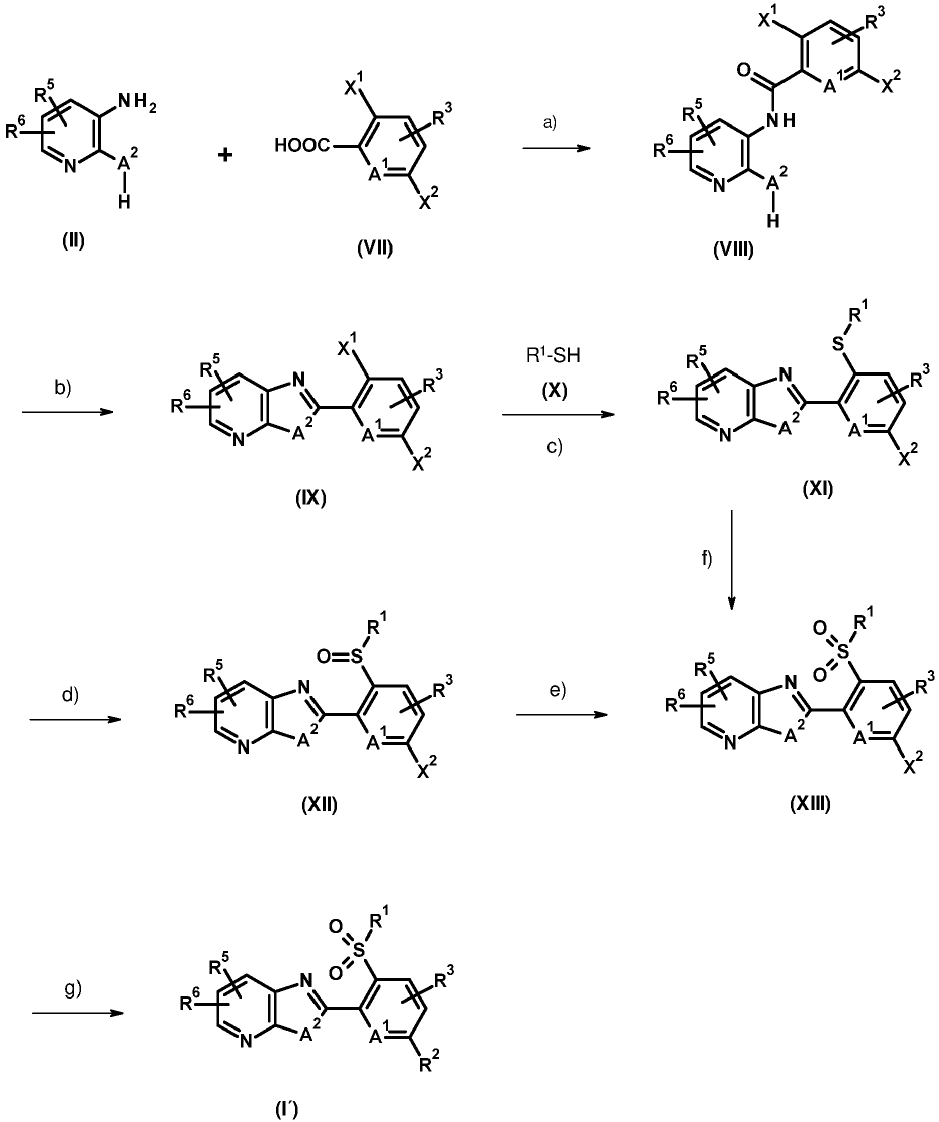

Die Reste R¹, R², R³, R⁵, R⁶ und A¹ haben die oben beschriebenen Bedeutungen, A² steht für -N-R⁷, O oder S, wobei R⁷ die oben beschriebene Bedeutung hat und X¹ bzw. X² stehen für Halogen.

### Schritt a)

Die Verbindungen der Formel (VIII) können in Analogie zu dem in US5576335 beschriebenen Verfahren durch die Umsetzung von Verbindungen der Formel (II) mit einer Carbonsäure der Formel (VII) in Gegenwart eines Kondensationsmittels bzw. einer Base hergestellt werden.

Verbindungen der Formel (II) sind entweder kommerziell erhältlich oder können nach bekannten Methoden hergestellt werden, beispielsweise analog der in US2003/69257 oder WO2006/65703 beschriebenen Verfahren.

Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.

Die Umsetzung der Verbindungen der Formel (II) mit Carbonsäuren der Formel (VII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI) oder 1,3-Dicyclohexylcarbodiimid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

### Schritt b)

Die Verbindungen der Formel (IX) lassen sich herstellen durch Kondensation der Verbindungen der Formel (VIII) z.B. analog der in WO2012/86848 beschriebenen Verfahren.

Die Umsetzung zu Verbindungen der Formel (IX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

### Schritt c)

Die Verbindungen der Formel (XI), lassen sich herstellen durch Umsetzung der Verbindungen der Formel (IX) mit den Verbindungen der Formel (X) in Gegenwart einer Base.

Mercaptanderivate der Formel (X) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindungen der Formel (XI) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

### Schritt d)

Die Verbindungen der Formel (XII) lassen sich herstellen durch Oxidation der Verbindungen der Formel (XI). Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von -20°C bis 120 °C durchgeführt werden.

### Schritt e)

Die Verbindungen der Formel (XIII) lassen sich herstellen durch Oxidation der Verbindungen der Formel (XII). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C bis 120 °C durchgeführt werden.

### Schritt f)

Die Verbindungen der Formel (XIII) lassen sich auch in einem einstufigen Prozess herstellen durch Oxidation der Verbindungen der Formel (XI). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C bis 120 °C durchgeführt werden.

### Schritt g)

Die Herstellung von Verbindungen der Formel (I') in denen R² für einen N-verknüpften Ring steht, kann nach literaturbekannten Methoden (siehe z.B. Journal of Organic Chemistry (2010), 69, 5578) z.B. in Gegenwart von Kupfer(I)-iodid und basischen Reaktionshilfsmitteln, wie beispielsweise *trans*-N,N'-Dimethylcyclohexan-1,2-diamin und Kaliumcarbonat, in einem geeigneten Lösungs- oder Verdünnungsmittel erfolgen. Als Lösungs- oder Verdünnungsmittel kommen alle inerten organischen Lösungsmittel in Betracht, beispielsweise aliphatische oder aromatische Kohlenwasserstoffe. Bevorzugt wird dabei Toluol eingesetzt. Weiterhin kann die Kupplung ohne Metallkatalyse in Gegenwart einer geeigneten Base wie beispielsweise Kaliumcarbonat oder Cäsiumcarbonat in einem geeigneten Lösungs- oder Verdünnungsmittel erfolgen. Als Lösungs- oder Verdünnungsmittel kommen alle inerten organischen Lösungsmittel in Betracht, beispielsweise aliphatische oder aromatische Kohlenwasserstoffe. Bevorzugt wird dabei Acetonitril oder Dimethylformamid eingesetzt.

Verbindungen der Formel (I') für die R² für einen C-verknüpften Ring steht, können beispielsweise aus Verbindungen der Formel (XIII), für die X bevorzugt für Halogen aus der Reihe Chlor oder Brom steht, nach allgemein bekannten Methoden (vgl. Chem. Rev. 1995, 95, 2457-2483; Tetrahedron 2002, 58, 9633-9695; Metal-Catalyzed Cross-Coupling Reactions (Eds.: A. de Meijere, F. Diederich), 2nd ed., Wiley-VCH, Weinheim, 2004) hergestellt werden.

Beispielsweise können Verbindungen der Formel (XIII), in denen X² bevorzugt für Chlor oder Brom steht, mit geeigneten Arylboronsäuren oder deren Estern nach bekannten Methoden (vgl. WO2010071819) in Gegenwart geeigneter Katalysatoren aus der Reihe der Übergangsmetallsalze zu Verbindungen der Formel (I'), umgesetzt werden. Als Kupplungskatalysatoren kommen beispielsweise bevorzugt Palladiumkatalysatoren wie [1,1'-Bis (diphenylphosphino)ferrocen]dichlorpalladium (II) oder Tetrakis(triphenylphosphin)palladium in Betracht. Als geeignete basische Reaktionshilfsmittel zur Durchführung der Verfahren finden bevorzugt Carbonate des Natriums oder Kaliums Verwendung.

Die benötigten (Hetero)arylboronsäuren oder (Hetero)arylboronsäureester sind teilweise bekannt und/oder kommerziell erhältlich bzw. können nach allgemein bekannten Methoden hergestellt werden (vgl. Boronic Acids (Eds.: D. G. Hall), 2nd ed., Wiley-VCH, Weinheim, 2011).

Die Umsetzung gemäß Schritt g) kann auch ausgehend von Verbindungen der Formeln (XI) oder (XII) erfolgen.

### Verfahren B

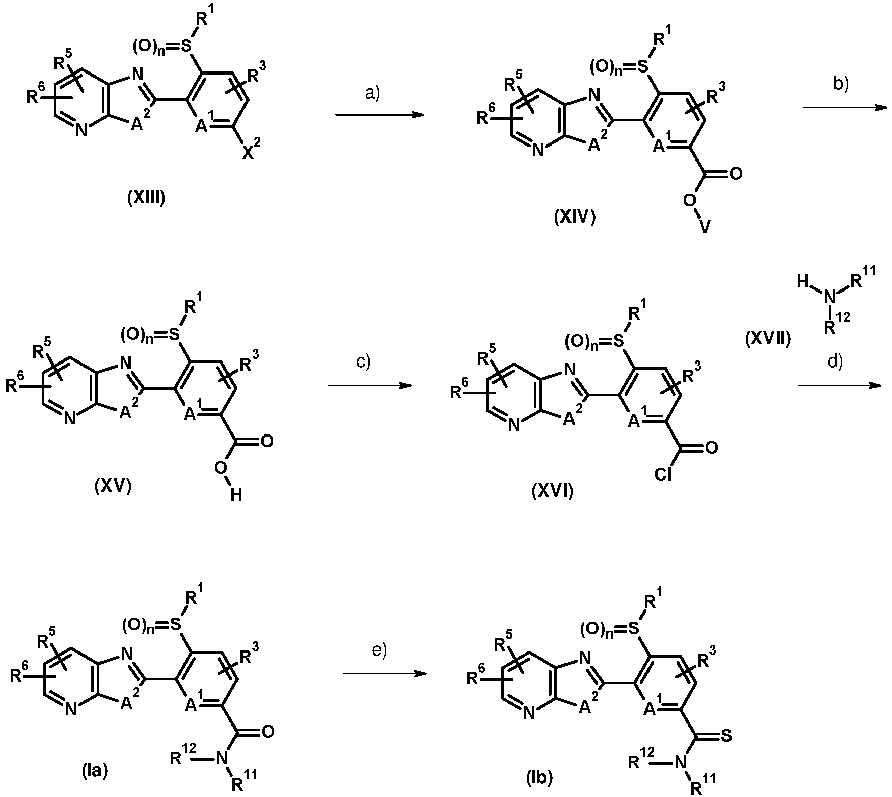

Die Reste R¹, R³, R⁵, R⁶, R¹¹, R¹², A¹ und n haben die oben beschriebenen Bedeutungen, A² steht für -N-R⁷, O oder S, wobei R⁷ die oben beschriebene Bedeutung hat, X² steht für Halogen und V für (C₁-C₄)Alkyl.

### Schritt a)

Verbindungen der Formel (Ia bzw. Ib) für die R² für einen C-verknüpftes Carbonamid oder Thioamid steht, können beispielsweise durch Carbonylierung der Verbindungen der Formel (XIII) analog S.A.Vinogradov, D.F.Wilson, Tetrahedron Letters 39 (1998), 8935-8938 hergestellt werden. Der Rest V steht bevorzugt für Methyl, Ethyl, n-Propyl oder n-Butyl. Als Katalysator für die Umsetzung lassen sich Palladiumphosphankomplexe einsetzen, beispielsweise ein Katalysator aus Palladiumchlorid, Triphenylphosphan und DPPP (1,3-Bis(diphenylphosphino)propan) (1:1:1). Bevorzugte Basen sind beispielsweise Hünigs Base (Diisopropylethylamin) oder DBU (1,8-Diazabicyclo(5.4.0)undec-7-en).

### Schritt b, c, d)

Der Ester der Formel (XIV) kann unter Verwendung von Standardmethoden, vgl. DE 2221647, zunächst in die Säure der Formel (XV) überführt werden, beispielsweise mit einem Alkalihydroxid als Base wie Natriumhydroxid oder Lithiumhydroxid, in einem Alkohol als Lösungsmittel wie z.B. Ethanol oder einem Gemisch aus Tetrahydrofuran und Wasser.

Anschließend wird die Säure der Formel (XV) nach Standardverfahren in das Säurechlorid der Formel (XVI) überführt, beispielsweise mit einem Chlorierungsreagenz wie Thionylchlorid oder Oxalylchlorid.

Die weitere Umsetzung mit dem Amin der Formel (XVII), in einem Verdünnungsmittel wie beispielsweise Dichlormethan oder Tetrahydrofuran und in Gegenwart einer Base wie beispielsweise Triethylamin oder Diisopropylethylamin, führt zur erfindungsgemäßen Verbindungen der Formel (Ia) (entspricht Formel I(G3)).

Verbindungen der Formel (XVII) sind entweder kommerziell erhältlich oder können nach bekannten Methoden hergestellt werden.

### Schritt e)

Thioamide der Formel (Ib) (entspricht Formel I(G4)) lassen sich aus den Carbonamiden der Formel (Ia), durch Umsetzung mit einem Schwefelungsreagenz, beispielsweise Lawessons Reagenz oder P₄S₁₀, herstellen.

### Verfahren C

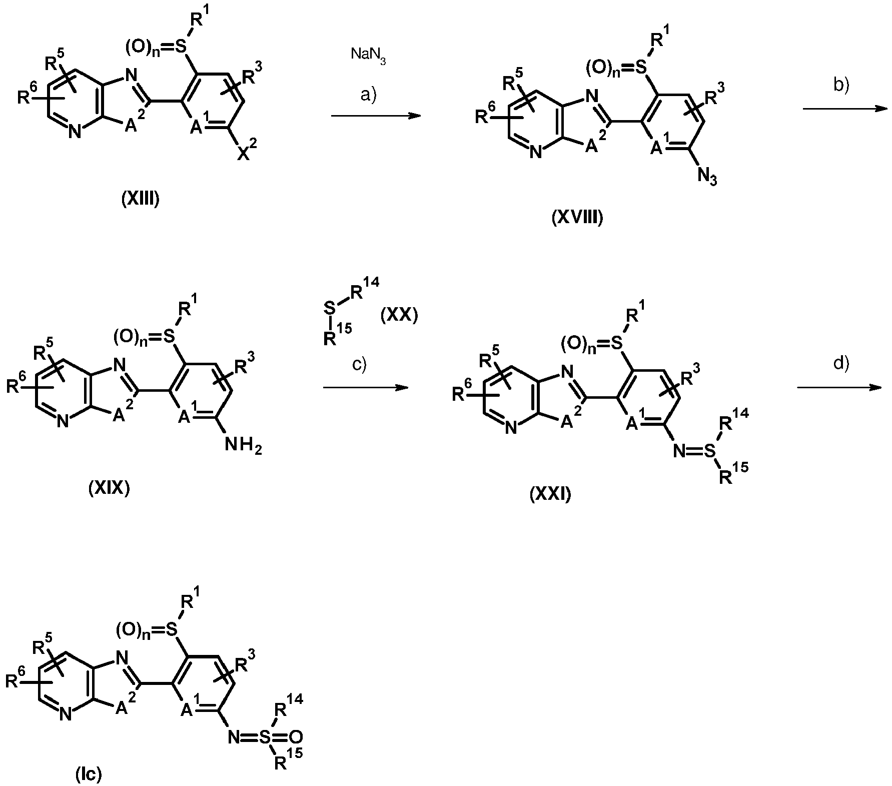

Die Reste R¹, R³, R⁵, R⁶, R¹⁴, R¹⁵, A¹ und n haben die oben beschriebenen Bedeutungen, A² steht für -N-R⁷, O oder S, wobei R⁷ die oben beschriebene Bedeutung hat und X² steht für Halogen.

### Schritt a)

Die Verbindungen der Formel (XVIII) können in Analogie zu WO2015/002211 durch Umsetzung der Verbindungen der Formel (XIII) mit Natruimazid hergestellt werden.

Die Umsetzung zu Verbindungen der Formel (XVIII) erfolgt in der Regel in einem Lösungsmittel, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Tetrahydrofuran, Ethylenglycoldimethylether, Dioxan, aprotische polare Lösungsmittel wie beispielsweise N,N-Dimethylformamid, N-Methylpyrrolidon, Dimethylsulfoxid oder Alkohole wie Methanol oder Ethanol.

### Schritt b)

Die Verbindungen der Formel (XIX) lassen sich herstellen durch Reduktion der Zwischenverbindungen der Formel (XVIII) z.B. analog der in WO2015/002211 beschriebenen Verfahren.

Beispiele für geeignete Reduktionsmittel sind Triphenylphospin, Tributylphoshin, Zinn(III)chlorid oder Zink.

Die Umsetzung zu Verbindungen der Formel (XIX) erfolgt in der Regel in einem Lösungsmittel. Bevorzugt werden Ether wie beispielsweise Tetrahydrofuran, Ethylenglycoldimethylether, Dioxan, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, aprotische polare Lösungsmittel wie beispielsweise N,N-Dimethylformamid, N-Methylpyrrolidon, Dimethylsulfoxid oder Alkohole wie Methanol oder Ethanol.

Wenn nötig kann eine Säure zur Reaktion gegeben werden wie beispielsweise Salzsäure oder Essigsäure.

### Schritt c)

Die Verbindungen der Formel (XXI) (entspricht Formel I(G16) mit p=0) lassen sich herstellen durch Umsetzung der Verbindungen der Formel (XIX) mit einer Verbindung der Formel (XX) in Gegenwart eines Oxidationsmittels.

Verbindungen der Formel (XX) sind entweder kommerziell erhältlich oder können nach bekannten Methoden hergestellt werden.

Die Umsetzung zu Verbindungen der Formel (XXI) erfolgt in der Regel in einem Lösungsmittel. Bevorzugt werden Ether wie beispielsweise Tetrahydrofuran, Methyl tert.-butylether, Dioxan, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, aprotische polare Lösungsmittel wie beispielsweise N,N-Dimethylformamid, N-Methylpyrrolidon, Dimethylsulfoxid oder Alkohole wie Methanol oder Ethanol.

Beispiele für Oxidationsmittel sind Halogenierungsmittel wie N-Chlorsuccinimid oder hypervalente Iodverbindungen wie Bis(acetato-O)phenyliodid.

### Schritt d)

Die Verbindungen der Formel (Ic) (entspricht Formel I(G16) mit p=1). können in Analogie zu dem in WO2015/002211 beschriebenen Verfahren durch die Umsetzung von Verbindungen der Formel (XXI) mit einem Oxidationsmittel hergestellt werden.

Beispiele für ein geeignetes Oxidationsmittel sind Natriumperiodat oder meta-Chlorperbenzoesäure.

Geeignete Lösungsmittel für die Oxidation sind beispielsweise halogenierte aliphatische Kohlenwasserstoffe wie beispielsweise Dichlormethan oder Chloroform Alkohole wie Methanol oder Ethanol oder Essigsäure.

### Verfahren D

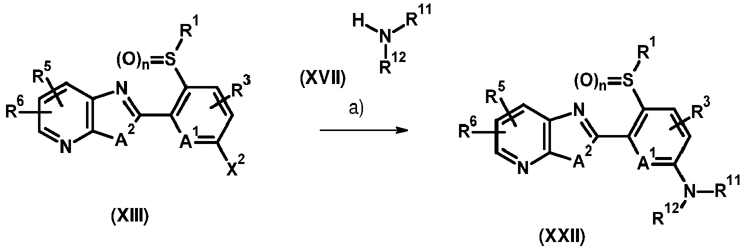

Die Reste R¹, R³, R⁵, R⁶, R¹¹, R¹², A¹ und n haben die oben beschriebenen Bedeutungen, A² steht für -N-R⁷, O oder S, wobei R⁷ die oben beschriebene Bedeutung hat und X² steht für Halogen.

### Schritt a)

Die Verbindungen der Formel (XXII) (entspricht Formel I(G10)) können in Analogie zu WO2015/002211 durch Umsetzung der Verbindungen der Formel (XIII) mit Verbindungen der Formel (XVII) hergestellt werden.

Verbindungen der Formel (XVII) sind entweder kommerziell erhältlich oder können nach bekannten Methoden hergestellt werden.

Die Umsetzung zu Verbindungen der Formel (XXII) erfolgt in der Regel in einem Lösungsmittel. Bevorzugt werden Ether wie beispielsweise Tetrahydrofuran, Methyl tert.-butylether, Dioxan, Ethylenglycoldimethylether, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, aprotische polare Lösungsmittel wie beispielsweise N,N-Dimethylformamid, N-Methylpyrrolidon, Dimethylsulfoxid, Ester wie beispielsweise Essigsäureethylester oder Nitrile wie Acetonitril.

Die Reaktion lässt sich durchführen in Gegenwart einer Base. Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin oder anorganische Basen wie Kaliumphosphat, Kaliumcarbonat und Natriumhydrid.

### Verfahren E

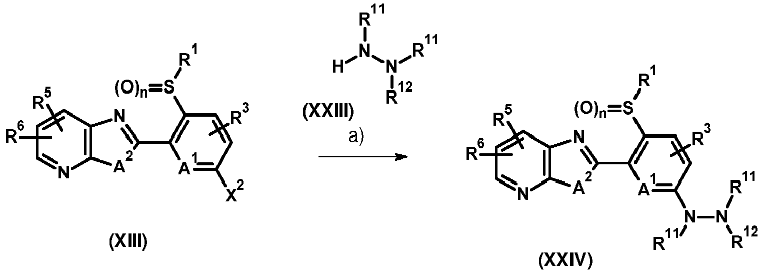

Die Reste R¹, R³, R⁵, R⁶, R¹¹, R¹², A¹ und n haben die oben beschriebenen Bedeutungen, A² steht für -N-R⁷, O oder S, wobei R⁷ die oben beschriebene Bedeutung hat und X² steht für Halogen.

### Schritt a)

Die Verbindungen der Formel (XXIV) (entspricht Formel I(G11)) lassen sich herstellen durch Umsetzung der Verbindungen der Formel (XIII) mit Verbindungen der Formel (XXIII).

Verbindungen der Formel (XXIII) sind entweder kommerziell erhältlich oder können nach bekannten Methoden hergestellt werden.

Die Umsetzung verläuft unter analogen Reaktionsbedingungen wie im Verfahren D beschrieben.

### Verfahren F

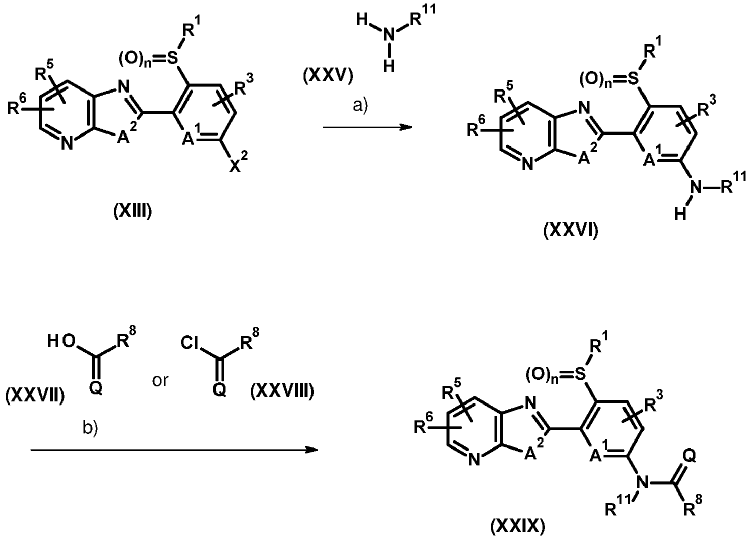

Die Reste R¹, R³, R⁵, R⁶, R⁸, R¹¹ A¹ und n haben die oben beschriebenen Bedeutungen, A² steht für -N-R⁷, O oder S, wobei R⁷ die oben beschriebene Bedeutung hat und X² steht für Halogen und Q steht für O oder S.

### Schritt a)

Die Verbindungen der Formel (XXVI) lassen sich herstellen durch Umsetzung der Verbindungen der Formel (XIII) mit Verbindungen der Formel (XXV).

Verbindungen der Formel (XXV) sind entweder kommerziell erhältlich oder können nach bekannten Methoden hergestellt werden.

Die Umsetzung verläuft unter analogen Reaktionsbedingungen wie im Verfahren D beschrieben.

### Schritt b)

Die Verbindungen der Formel (XXIX) (entspricht Formel I(G12) mit Q=O und Formel I(G13) mit Q=S)) lassen sich herstellen durch Umsetzung der Verbindungen der Formel (XXVI) mit Verbindungen der Formel (XXVII).

Verbindungen der Formeln (XXVII) sind entweder kommerziell erhältlich oder können nach bekannten Methoden hergestellt werden.

Die Umsetzung zu Verbindungen der Formel (XXIX) erfolgt in der Regel in einem Lösungsmittel. Bevorzugt werden Ether wie beispielsweise Tetrahydrofuran, Methyl tert.-butylether, Dioxan, Ethylenglycoldimethylether, halogenierte Kohlenwasserstoffe wie beispielsweise Dichlormethan, Chloroform, Tetrachlorkohlenstoff, 1,2-Dichlorethan oder Chlorbenzol, aromatische Kohlenwasserstoffe wie Toluol, Xylol, Ester wie beispielsweise Essigsäureethylester, Nitrile wie Acetonitril, aprotische polare Lösungsmittel wie beispielsweise N,N-Dimethylformamid, N-Methylpyrrolidon, Dimethylsulfoxid oder stickstoffhaltige Heterocyclen wie Pyridin oder Chinolin.

Die Reaktion lässt sich durchführen in Gegenwart eines Kondensationsmittels. Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid.

Die Reaktion lässt sich durchführen in Gegenwart eines geeigneten Katalysators. Ein Beispiel für einen geeigneten Katalysator ist 1-Hydroxybenzotriazol.

Die Verbindungen der Formel (XXIX) lassen sich auch herstellen durch Umsetzung der Verbindungen der Formel (XXVI) mit Verbindungen der Formel (XXVIII).

Verbindungen der Formel (XXVIII) sind entweder kommerziell erhältlich oder können nach bekannten Methoden hergestellt werden.

Die Umsetzung zu Verbindungen der Formel (XXIX) erfolgt in der Regel in einem Lösungsmittel.

Bevorzugt werden Ether wie beispielsweise Tetrahydrofuran, Methyl tert.-butylether, Dioxan, Ethylenglycoldimethylether, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, aprotische polare Lösungsmittel wie beispielsweise N,N-Dimethylformamid, N-Methylpyrrolidon, Dimethylsulfoxid, Ester wie beispielsweise Essigsäureethylester oder Nitrile wie Acetonitril.

Die Reaktion lässt sich durchführen in Gegenwart einer Base. Beispiele für geeignete Basen sind stickstoffhaltige Heterocyclen wie Pyridin, Dimethylaminopyridin, Picolin, 2,6-Lutidin, 1,8-Diazabicyclo[5.4.0]-7-undecen (DBU); tertiäre Amine wie Triethylamin und N,N-Diisopropylethylamin oder anorganische Basen wie Kaliumcarbonat und Natriumhydrid.

### Verfahren G

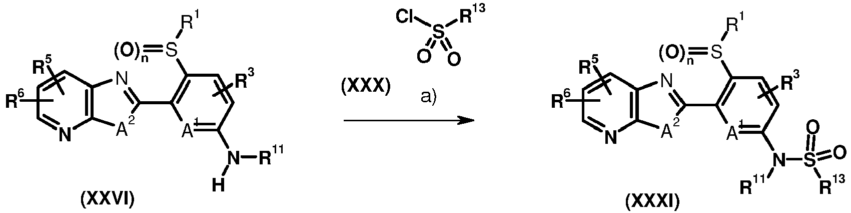

Die Reste R¹, R³, R⁵, R⁶, R¹¹, R¹³, A¹ und n haben die oben beschriebenen Bedeutungen, A² steht für -N-R⁷, O oder S, wobei R⁷ die oben beschriebene Bedeutung hat.

### Schritt a)

Die Verbindungen der Formel (XXXI) (entspricht Formel I(G14)) lassen sich herstellen durch Umsetzung der Verbindungen der Formel (XXVI) mit Verbindungen der Formel (XXX).

Verbindungen der Formel (XXX) sind entweder kommerziell erhältlich oder können nach bekannten Methoden hergestellt werden.

Die Umsetzung zu Verbindungen der Formel (XXXI) erfolgt in der Regel in einem Lösungsmittel. Bevorzugt werden Ether wie beispielsweise Tetrahydrofuran, Methyl tert.-butylether, Dioxan, Ethylenglycoldimethylether, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, Ester wie beispielsweise Essigsäureethylester, Nitrile wie Acetonitril oder aprotische polare Lösungsmittel wie beispielsweise N,N-Dimethylformamid, N-Methylpyrrolidon, Dimethylsulfoxid.

Die Reaktion lässt sich durchführen in Gegenwart einer Base. Beispiele für geeignete Basen sind stickstoffhaltige Heterocyclen wie Pyridin, Dimethylaminopyridin, Picolin, 2,6-Lutidin, 1,8-Diazabicyclo[5.4.0]-7-undecen (DBU); tertiäre Amine wie Triethylamin und N,N-Diisopropylethylamin oder anorganische Basen wie Kaliumcarbonat und Natriumhydrid.

### Verfahren H

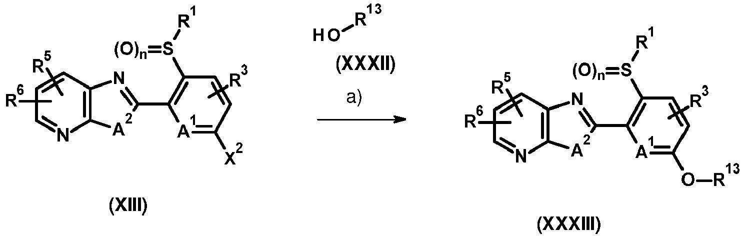

Die Reste R¹, R³, R⁵, R⁶, R¹³, A¹ und n haben die oben beschriebenen Bedeutungen, A² steht für -N-R⁷, O oder S, wobei R⁷ die oben beschriebene Bedeutung hat und X² steht für Halogen.

### Schritt a)

Die Verbindungen der Formel (XXXIII) (entspricht Formel I(G17)) lassen sich herstellen durch Umsetzung der Verbindungen der Formel (XIII) mit Verbindungen der Formel (XXXII), z.B. analog der in US4558134 oder US2014/275026 beschriebenen Verfahren.

Verbindungen der Formel (XXXII) sind entweder kommerziell erhältlich oder können nach bekannten Methoden hergestellt werden.

Die Umsetzung zu Verbindungen der Formel (XXXIII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat, Kaliumcarbonat und Kalium-tert-Butanolat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

### Verfahren I

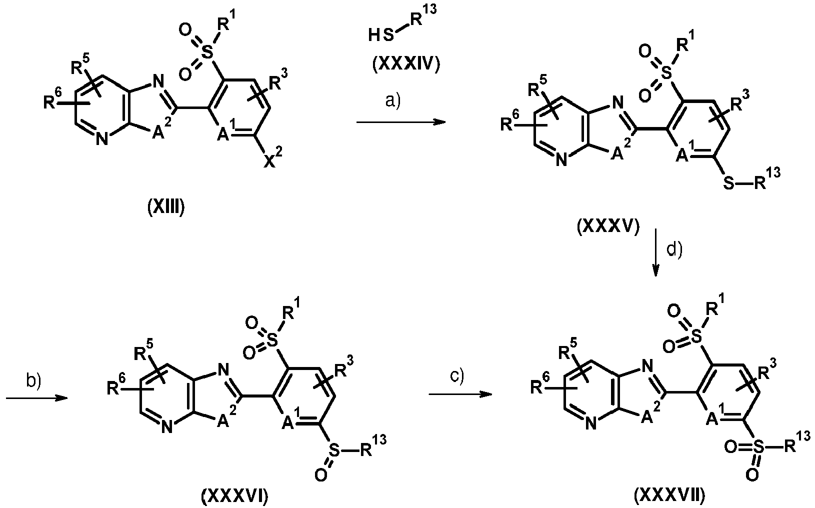

Die Reste R¹, R³, R⁵, R⁶, R¹³ und A¹ haben die oben beschriebenen Bedeutungen, A² steht für -N-R⁷, O oder S, wobei R⁷ die oben beschriebene Bedeutung hat und X² steht für Halogen.

### Schritt a)

Die Verbindungen der Formel (XXXV) (entspricht Formel I(G5) mit m=0) lassen sich herstellen durch Umsetzung der Verbindungen der Formel (XIII) mit Verbindungen der Formel (XXXIV).

Verbindungen der Formel (XXXIV) sind entweder kommerziell erhältlich oder können nach bekannten Methoden hergestellt werden.

Die Umsetzung zu Verbindungen der Formel (XXXV) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat, Kaliumcarbonat und Kalium-tert-Butanolat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

### Schritt b)

Die Verbindungen der Formel (XXXVI) (entspricht Formel I(G5) mit m=1) lassen sich herstellen durch Oxidation der Verbindungen der Formel (XXXV). Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von -20°C bis 120 °C durchgeführt werden.

### Schritt c)

Die Verbindungen der Formel (XXXVII) (entspricht Formel I(G5) mit m=2) lassen sich herstellen durch Oxidation der Verbindungen der Formel (XXXVI). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C bis 120 °C durchgeführt werden.

### Schritt d)

Die Verbindungen der Formel (XXXVII) (entspricht Formel I(G5) mit m=2) lassen sich auch in einem einstufigen Prozess herstellen durch Oxidation der Verbindungen der Formel (XXXV). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C bis 120 °C durchgeführt werden.

### Verfahren J

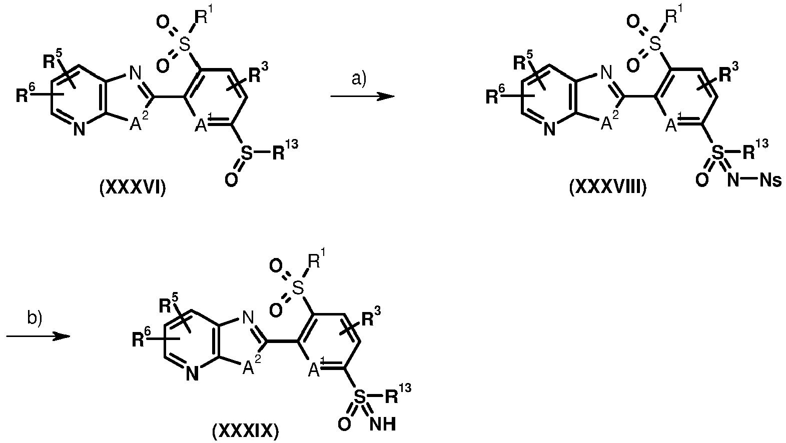

Die Reste R¹, R³, R⁵, R⁶, R¹³ und A¹ haben die oben beschriebenen Bedeutungen, A² steht für -N-R⁷, O oder S, wobei R⁷ die oben beschriebene Bedeutung hat. Ns = Nosyl/4-Nitrobenzylsulfonyl.

### Schritt a)

Die Verbindungen der Formel (XXXVIII) lassen sich herstellen durch Iminierung der Verbindungen der Formel (XXXVI) mit 4-Nitrobenzylsulfonylamid (NsNH2) und einer hypervalenten Iodverbindung unter Metallkatalyse, z.B. analog der in Organic Letters 2006, 8, 2349 und Chemistry - A European Journal 2007, 13, 6674 beschriebenen Verfahren.

Die Umsetzung zu Verbindungen der Formel (XXXVIII) wird vorzugsweise in einem Lösungsmittel durchgeführt, welches ausgewählt ist aus üblichen, bei den vorherrschenden Reaktionsbedingungen inerten Lösungsmitteln. Bevorzugt werden Nitrile, wie beispielsweise Acetonitril oder halogenierte Alkane wie beispielsweise Dichlormethan.

Beispiele für Metallkatalysatoren sind Eisen-, Kupfer-, Silber- oder Rhodiumverbindungen, beispielsweise Eisen(II)-acetylacetonat, Eisen(III)-acetylacetonat und Rhodium(II)-acetat. Als hypervalente Iodverbindung kommen häufig Diacetoxyiodbenzol oder Iodosylbenzol zum Einsatz.

Die Reaktion kann im Vakuum, bei Normaldruck oder unter Überdruck und bei Raumtemperatur durchgeführt werden.

### Schritt b)

Die Verbindungen der Formel (XXXIX) (entspricht Formel I(G6)) lassen sich herstellen aus Verbindungen der Formel (XXXVIII) durch Umsetzung mit einem Thiol unter basischen Bedingungen, z.B. analog der in Organic Letters 2006, 8, 2349; Chemistry - A European Journal 2007, 13, 6674 und WO2006/101860 beschriebenen Verfahren.

Die Umsetzung zu Verbindungen der Formel (XXXIX) erfolgt in der Regel in einem Lösungsmittel. Bevorzugt werden Ether wie beispielsweise Tetrahydrofuran, oder Nitrile wie Acetonitril.

Als Thiol wird beispielsweise Thiophenol in Kombination mit einer anorganischen Base aus der Gruppe bestehend aus Carbonaten von Alkali- oder Erdalkalimetallen wie beispielsweise Caesiumcarbonat verwendet. Auch die Verwendung einer Lösung von Natriumthiomethanolat in Methanol ist beschrieben.

Die Reaktion kann im Vakuum, bei Normaldruck oder unter Überdruck und bei Temperaturen von -78 °C bis 30 °C durchgeführt werden.

### Verfahren K

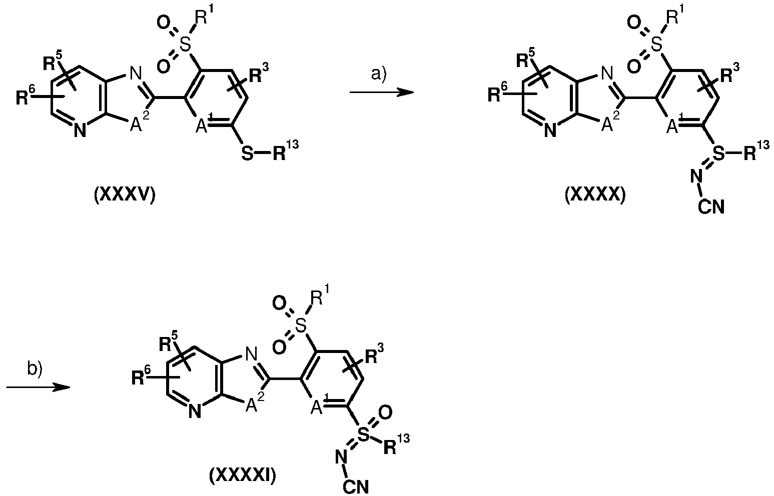

Die Reste R¹, R³, R⁵, R⁶, R¹³ und A¹ haben die oben beschriebenen Bedeutungen und A² steht für -N-R⁷, O oder S, wobei R⁷ die oben beschriebene Bedeutung hat.

### Schritt a)

Die Verbindungen der Formel (XXXX) (entspricht Formel I(G8)) lassen sich herstellen aus Verbindungen der Formel (XXXV) durch Iminierung mit Cyanamid in Anwesenheit einer hypervalenten Iodverbindung z.B. analog der in Organic Letters 2007, 9, 2951 beschriebenen Verfahren. Die Umsetzung zu Verbindungen der Formel (XXXX) erfolgt in der Regel in Acetonitril als Lösungsmittel. Als hypervalente Iodverbindung kommen häufig Diacetoxyiodbenzol oder Iodosylbenzol zum Einsatz. Die Reaktion kann im Vakuum, bei Normaldruck oder unter Überdruck und bei Temperaturen von 0 °C bis 30 °C durchgeführt werden.

Die Verbindungen der Formel (XXXX) lassen sich desweiteren herstellen aus Verbindungen der Formel (XXXV) durch Iminierung mit Cyanamid in Anwesenheit von *N*-Bromsuccinimid (NBS) oder Iod unter basischen Bedingungen, z.B. analog der in Organic Letters 2007, 9, 3809 oder WO2014/29830 beschriebenen Verfahren. Die Umsetzung zu Verbindungen der Formel (XXXX) erfolgt in diesem Fall in einem Lösungsmittel. Bevorzugt werden Alkohole wie beispielsweise Methanol, Ether wie beispielsweise Tetrahydrofuran oder Nitrile wie Acetonitril verwendet. Beispiele für geeignete Basen sind Alkoholate wie beispielsweise Kalium-*tert*-Butanolat. Weitere geeignete Basen sind Alkalimetallhydride wie z.B. Natriumhydrid. Die Reaktion kann im Vakuum, bei Normaldruck oder unter Überdruck und bei Raumtemperatur durchgeführt werden.

### Schritt b)

Die Verbindungen der Formel (XXXXI) (entspricht Formel I(G7)) lassen sich herstellen aus Verbindungen der Formel (XXXX) durch Oxidation im Basischen, z.B. analog der in Organic Letters 2007, 9, 2951; Organic Letters 2007, 9, 3809 oder WO2014/140075 beschriebenen Verfahren. Die Oxidation wird generell in einem Lösungsmittel durchgeführt. Bevorzugt werden Alkohole wie Ethanol. Als Oxidationsmittel wird beispielsweise meta-Chlorperbenzoesäure verwendet. Beispiele für geeignete Basen sind Carbonate wie beispielsweise Kaliumcarbonat. Die Reaktion kann im Vakuum, bei Normaldruck oder unter Überdruck und bei Temperaturen von 0 °C bis 30 °C durchgeführt werden.

### Verfahren L

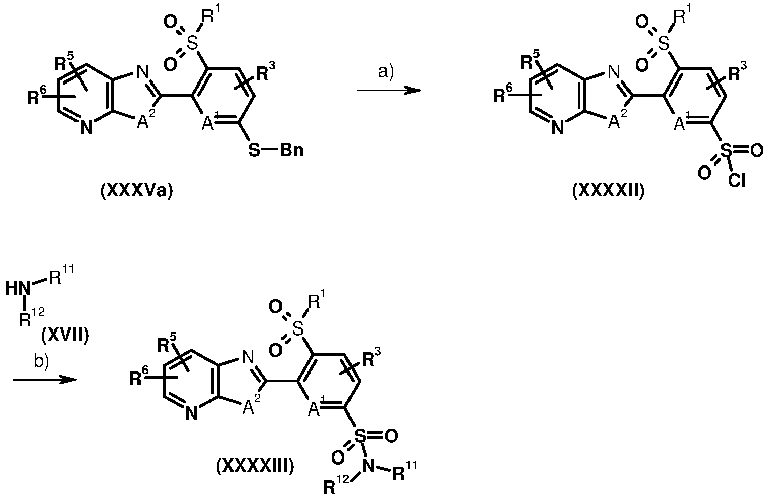

Die Reste R¹, R³, R⁵, R⁶, R¹¹, R¹² und A¹ haben die oben beschriebenen Bedeutungen und A² steht für -N-R⁷, O oder S, wobei R⁷ die oben beschriebene Bedeutung hat. Bn = Benzyl/CH₂Ph.

### Schritt a)

Die Verbindungen der Formel (XXXXII) lassen sich herstellen aus Zwischenverbindungen der Formel (XXXVa) durch Oxidation unter Anwesenheit einer Chlorquelle, z.B. analog der in WO2008/2244 und WO2010/24451 beschriebenen Verfahren.

Die Umsetzung zu Verbindungen der Formel (XXXXII) erfolgt in der Regel in einem Lösungsmittel. Bevorzugt werden halogenierte Alkane wie beispielsweise Dichlormethan oder Tetrachlormethan. Häufig werden dabei Mischungen mit Wasser verwendet.

Als Chlorquelle und Oxidationsmittel werden beispielsweise Chlorgas oder *N*-Chlorsuccinimid verwendet.

Die Reaktionen werden häufig im Sauren durchgeführt. Als Säuren finden beispielsweise Carbonsäuren wie Ameisensäure oder Essigsäure Verwendung.

Die Reaktion kann im Vakuum, bei Normaldruck oder unter Überdruck und bei Temperaturen von 0 °C bis 30 °C durchgeführt werden.

### Schritt b)

Die Verbindungen der Formel (XXXXIII) (entspricht Formel I(G9)) lassen sich herstellen aus Verbindungen der Formel (XXXXII) mit Verbindungen der Formel (XVII) unter Standardbedingungen.

Die Umsetzung zu Verbindungen der Formel (XXXXIII) erfolgt in der Regel in einem Lösungsmittel. Bevorzugt werden Ether wie beispielsweise Tetrahydrofuran, Methyl tert.-butylether, Dioxan, Ethylenglycoldimethylether, aliphatische Kohlenwasserstoffe wie Hexan, Heptan, aromatische Kohlenwasserstoffe wie Toluol, Xylol, halogenierte Kohlenwasserstoffe wie beispielsweise Dichlormethan, Chloroform, Tetrachlorkohlenstoff, 1,2-Dichlorethan oder Chlorbenzol, Ester wie beispielsweise Essigsäureethylester, Nitrile wie Acetonitril oder aprotische polare Lösungsmittel wie beispielsweise N,N-Dimethylformamid, N-Methylpyrrolidon, Dimethylsulfoxid.

Die Reaktion lässt sich durchführen in Gegenwart einer Base. Beispiele für geeignete Basen sind stickstoffhaltige Heterocyclen wie Pyridin, Dimethylaminopyridin, Picolin, 2,6-Lutidin, 1,8-Diazabicyclo[5.4.0]-7-undecen (DBU); tertiäre Amine wie Triethylamin und N,N-Diisopropylethylamin oder anorganische Basen wie Kaliumcarbonat und Natriumhydrid.

### Verfahren M

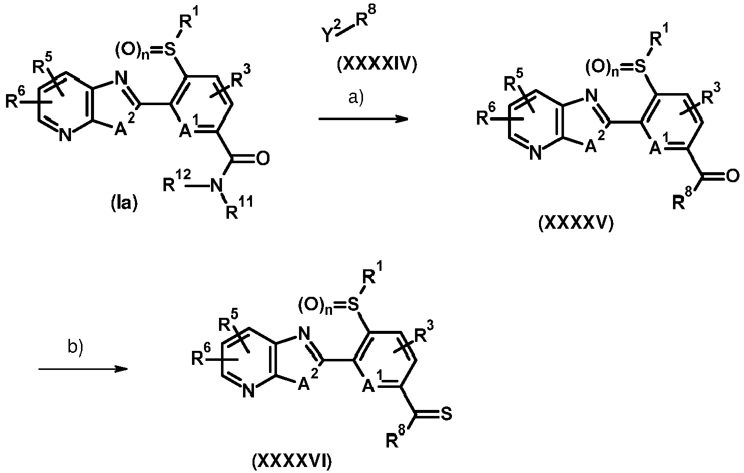

Die Reste R¹, R³, R⁵, R⁶, R⁸, R¹¹, R¹², A¹ und n haben die oben beschriebenen Bedeutungen und A² steht für -N-R⁷, O oder S, wobei R⁷ die oben beschriebene Bedeutung hat. Y² steht für Cl, Br oder I.

### Schritt a)

Die Verbindungen der Formel (XXXXV) (entspricht Formel I(G1)) lassen sich herstellen durch Überführung von Verbindungen der Formel (XXXXIV) in eine Organometallverbindung und nachfolgende Umsetzung mit Verbindungen der Formel (Ia), z.B. analog der in US5821246 und European Journal of Medicinal Chemistry 2012, 58, p. 396 beschriebenen Verfahren.

Verbindungen der Formel (XXXXIV) sind entweder kommerziell erhältlich oder können nach bekannten Methoden hergestellt werden.

Die Organometallverbindungen lassen sich beispielsweise aus Verbindungen der Formel (XXXXIV) durch Umsetzung mit Magnesium oder Lithiumalkylen erzeugen.

Die Umsetzung zu Verbindungen der Formel (XXXXV) erfolgt in der Regel in einem Lösungsmittel. Bevorzugt werden Ether wie Tetrahydrofuran oder Diethylether.

Die Reaktion kann im Vakuum, bei Normaldruck oder unter Überdruck und bei Temperaturen von -78 °C bis 45 °C durchgeführt werden.

### Schritt b)

Thioketone der Formel (XXXXVI) (entspricht Formel I(G2)) lassen sich aus Verbindungen der Formel (XXXXV) durch Umsetzung mit einem Schwefelungsreagenz, beispielsweise Lawessons Reagenz oder P4S10, herstellen.

### Verfahren N

Die Verbindungen der Formel (I), bei denen X für H10, H11, H15 oder H16 steht, können nach bekannten Methoden hergestellt werden, beispielsweise analog der in US2009/203705, US2012/258951, WO2013/3298 oder J. Med. Chem. 31, (1988) 1590-1595 beschriebenen Verfahren.

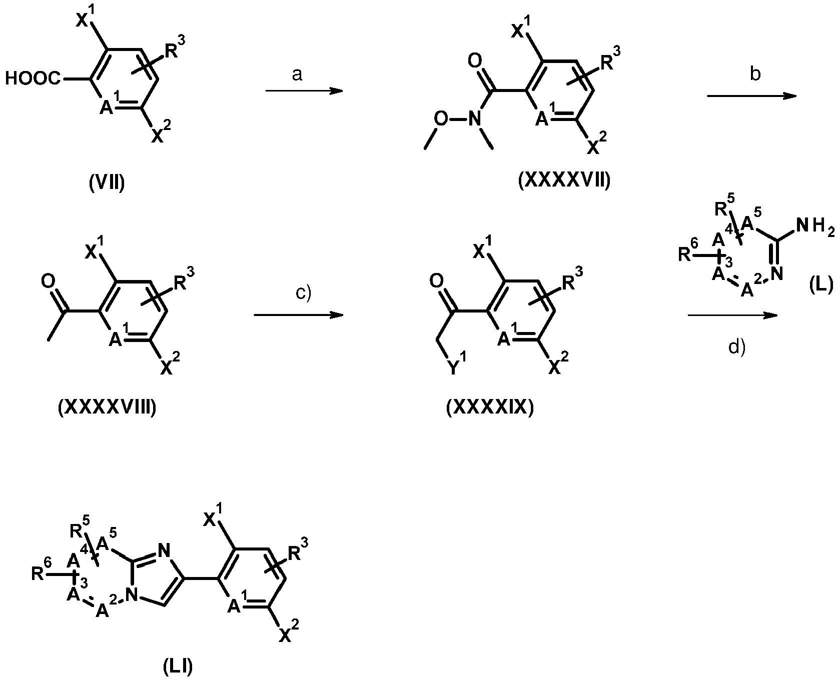

Die Reste A¹, R³, R⁵ und R⁶ haben die oben beschriebenen Bedeutungen. X¹, X² und Y¹ stehen für Halogen. A², A³, A⁴ und A⁵ stehen unabhängig voneinander für CH oder N (wobei A², A³, A⁴ und A⁵ nicht gleichzeitig für N stehen).

### Schritt a)

Carbonsäuren der Formel (VII) werden in Analogie zu dem in WO2011/75643 oder EP2671582 beschriebenen Verfahren in Gegenwart von O,N-Dimethyl-hydroxylamin Hydrochlorid in Weinreb-Amide der Formel (XXXXVII) überführt.

Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.

### Schritt b, c)

Verbindungen der Formel (XXXXVII) lassen sich anschließend nach bekannten Methoden, beispielsweise analog dem in WO2011/75643 beschriebenen Verfahren, mit einem Grignard Reagenz wie beispielsweise Methylmagnesiumbromid in Ketone der Formel (XXXXVIII) überführen. Durch anschließende Halogenierung analog der beispielsweise in US2012/302573 beschriebenen bekannten Methode sind Verbindungen der Formel (XXXXIX) zugänglich.

### Schritt d)

Die Verbindungen der Formel (LI) lassen sich herstellen durch Cyclisierung der Verbindungen der Formel (XXXIX) mit Aminen der Formel (L). Die Cyclisierung erfolgt beispielsweise in Ethanol, Acetonitril oder N,N-Dimethylformamid nach bekannten Methoden analog der beispielsweise in WO2005/66177, WO2012/88411, WO2013/3298, US2009/203705, US2012/258951, WO2012/168733, WO2014/187762 oder J. Med. Chem. 31 (1988) 1590-1595 beschriebenen Verfahren.

Die Verbindungen der Formel (L) sind kommerziell erhältlich.

Die weitere Umsetzung von Verbindungen der Formel (LI) zu Verbindungen der Formel (I) erfolgt analog zu Verfahren A bis M, Q, R, T und U.

### Verfahren O

Die Verbindungen der Formel (I), bei denen X für H17 steht, können nach bekannten Methoden hergestellt werden, beispielsweise analog der in WO2014/142292 beschriebenen Verfahren.

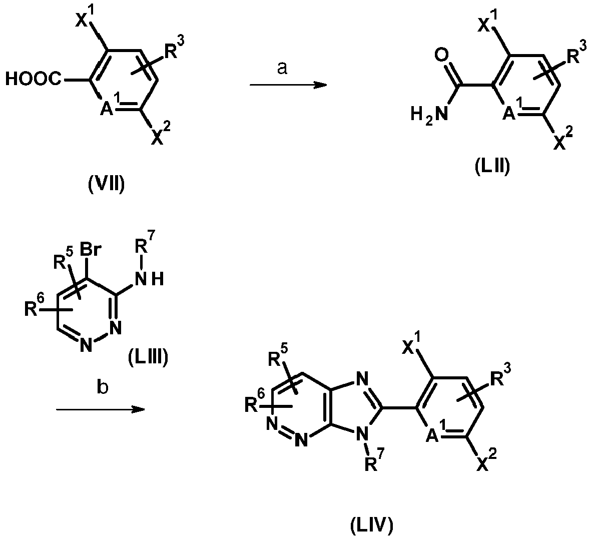

Die Reste R³, R⁵, R⁶ und R⁷ haben die oben beschriebenen Bedeutungen. X¹ und X² stehen für Halogen.

### Schritt a)

Die Verbindungen der Formel (LII) können in Analogie zu dem in US5374646 oder Bioorganic and Medicinal Chemistry Letters 2003, 13, 1093 - 1096 beschriebenen Verfahren durch die Umsetzung von Verbindungen der Formel (VII) mit einer Ammoniakquelle in Gegenwart eines Kondensationsmittels hergestellt werden. Die Herstellung von Verbindungen der Formel (LIII) mit R⁶ = H und R⁵ = Trifluormethyl ist in WO2016/039441 beschrieben.

Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.

Die Umsetzung der Verbindungen der Formel (VII) mit der Ammoniakquelle wird vorzugsweise in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Dioxan oder Tetrahydrofuran.

Ein geeignetes Kondensationsmittel ist beispielsweise Carbonyldiimidazol.

Die Reaktion kann im Vakuum, bei Normaldruck oder unter Überdruck durchgeführt werden. Vorzugsweise erfolgt die Reaktion bei Normaldruck und Temperaturen von 20 bis 70 °C.

### Schritt b)

Die Verbindungen der Formel (LIV) können in Analogie zu dem in WO2014/142292 beschriebenen Verfahren durch die Umsetzung von Verbindungen der Formel (LII) mit Verbindungen der Formel (LIII) in Gegenwart eines Palladiumkatalysators im Basischen hergestellt werden.

Verbindungen der Formel (LIII) können beispielsweise analog der in WO2014/142292 beschriebenen Verfahren hergestellt werden. Als Palladiumkatalysator kann beispielsweise [1,1'-Bis-(diphenylphosphino)ferrocen]dichlorpalladium(II) verwendet werden. Als Base finden häufig anorganische Basen wie Kaliumtertbutanolat Verwendung

Die Umsetzung erfolgt in einem Lösungsmittel. Häufig wird Toluol verwendet.

Die Reaktion kann im Vakuum, bei Normaldruck oder unter Überdruck durchgeführt werden. Vorzugsweise erfolgt die Reaktion bei Normaldruck und Temperaturen von 20 bis 110 °C.

Die weitere Umsetzung von Verbindungen der Formel (LIV) zu Verbindungen der Formel (I) erfolgt analog zu Verfahren A bis M, Q, R, T und U.

### Verfahren P

Die Verbindungen der Formel (I), bei denen X für H3, H12, H13 oder H18 steht, können nach bekannten Methoden hergestellt werden, beispielsweise analog der in WO2010/091310, WO 2012/66061 oder WO2013/099041 beschriebenen Verfahren.

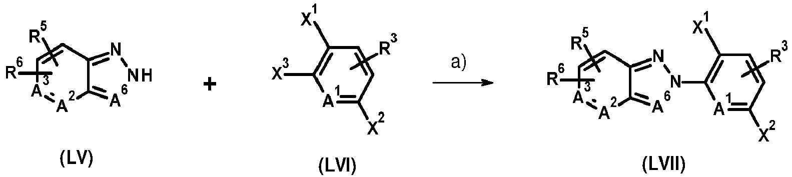

Die Reste A¹, R³, R⁵ und R⁶ haben die oben beschriebenen Bedeutungen. A², A³ und A⁶ stehen unabhängig voneinander für CH oder N (wobei A² und A³ nicht gleichzeitig für N stehen können). X¹, X² und X³ stehen für Halogen.

### Schritt a)

Die Verbindungen der Formel (LVII) lassen sich herstellen durch Umsetzung von Verbindungen der Formel (LV) mit Verbindungen der Formel (LVI) unter basischen Bedingungen, z.B. analog der in WO2010/091310, WO 2012/66061 oder WO2013/099041 beschriebenen Verfahren.

Verbindungen der Formel (LV) sind entweder kommerziell erhältlich oder können nach bekannten Methoden hergestellt werden, beispielsweise analog der in WO2005/100353, WO 2012/66061 oder in European Journal of Medicinal Chemistry 2010, 45, 2214 - 2222 beschriebenen Verfahren.

Verbindungen der Formel (LVI) sind entweder kommerziell erhältlich oder können nach bekannten Methoden hergestellt werden, beispielsweise analog der in WO2013/43518, EP2168965 oder in Journal of Medicinal Chemistry 2003, 46, 1449 - 1455 beschriebenen Verfahren.

Als Basen werden meist anorganische Basen wie Natriumhydrid, Kaliumcarbonat oder Cäsiumcarbonat verwendet.

Die Umsetzung zu Verbindungen der Formel (LVII) erfolgt meist in einem Lösungsmittel, vorzugsweise in einem Nitril, wie beispielsweise Acetonitril oder Propionitril oder in einem aprotischen, polaren Lösungsmittel wie beispielsweise N,N-Dimethylformamid oder N-Methylpyrrolidon.

Die Reaktion kann im Vakuum, bei Normaldruck oder unter Überdruck und bei Temperaturen von 0 °C bis 200 °C durchgeführt werden.

Alternativ kann die Umsetzung von Verbindungen der Formel (LV) mit Verbindungen der Formel (LVI) zu Verbindungen der Formel (LVII) auch durch Palladium-katalysierte *N*-Arylierung erfolgen, z.B. analog der in Angewandte Chemie Int. Ed. 2011, 50, 8944-8947 beschriebenen Verfahren.

Die weitere Umsetzung von Verbindungen der Formel (LVII) zu Verbindungen der Formel (I) erfolgt analog zu Verfahren A bis M, Q, R, T und U.

### Verfahren Q

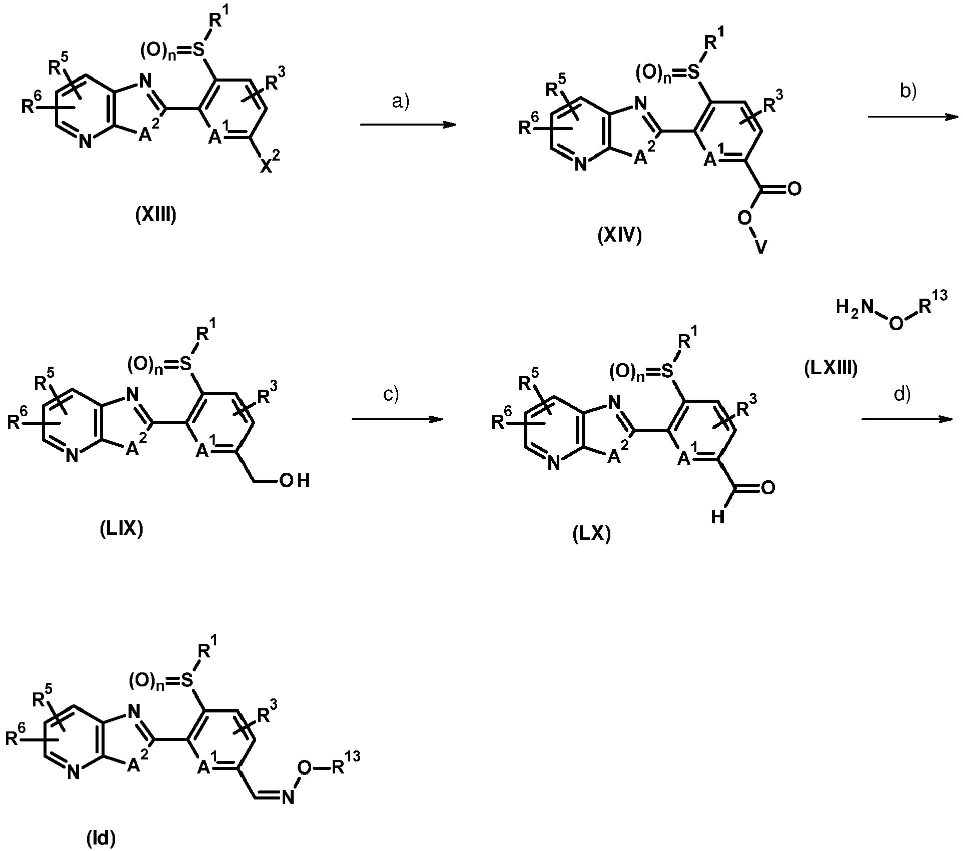

Die Reste R¹, R³, R⁵, R⁶, R¹³, A¹ und n haben die oben beschriebenen Bedeutungen, A² steht für -N-R⁷, O oder S, wobei R⁷ die oben beschriebene Bedeutung hat. X² steht für Halogen und V für (C₁-C₄)Alkyl.

### Schritt a)

Verbindungen der Formel (Id) (entspricht Formel I(G18)), können beispielsweise durch Carbonylierung der Verbindungen der Formel (XIII) analog S.A.Vinogradov, D.F.Wilson, Tetrahedron Letters 39 (1998), 8935-8938 hergestellt werden. Der Rest V ist bevorzugt Methyl, Ethyl, n-Propyl oder n-Butyl. Als Katalysator für die Umsetzung lassen sich Palladiumphosphankomplexe einsetzen, beispielsweise ein Katalysator aus Palladiumchlorid, Triphenylphosphan und DPPP (1,3-Bis(diphenylphosphino)propan) (1:1:1). Bevorzugte Basen sind beispielsweise Hünigs Base (Diisopropylethylamin) oder DBU (1,8-Diazabicyclo(5.4.0)undec-7-en).

### Schritt b, c, d)

Der Ester der Formel (XIV) kann unter Verwendung von Standardmethoden wie beispielsweise Natriumtetrahydroborat oder Lithiumaluminiumhydrid in Tetrahydrofuran oder Ethanol, vgl. WO2004/14361, WO2008/156757 oder US2006/235028, zunächst in den Alkohol der Formel (LIX) überführt werden

Anschließend wird der Alkohol der Formel (LIX) nach Standardverfahren zum Aldehyd der Formel (LX) oxidiert, beispielsweise mit Mangandioxid analog der in WO2006/81178 oder WO2009/128019 beschriebenen Verfahren.

Verbindungen der Formel (LXIII) sind entweder kommerziell erhältlich oder können nach bekannten Methoden hergestellt werden.

Die weitere Umsetzung nach Standardverfahren mit einem Hydroxylamin der Formel (LXIII) in einem Verdünnungsmittel wie beispielsweise Methanol oder Ethanol und gegebenenfalls in Gegenwart einer Säure wie beispielsweise Essigsäure analog WO2014/102244 führt zu erfindungsgemäßen Verbindungen der Formel (Id).

### Verfahren R

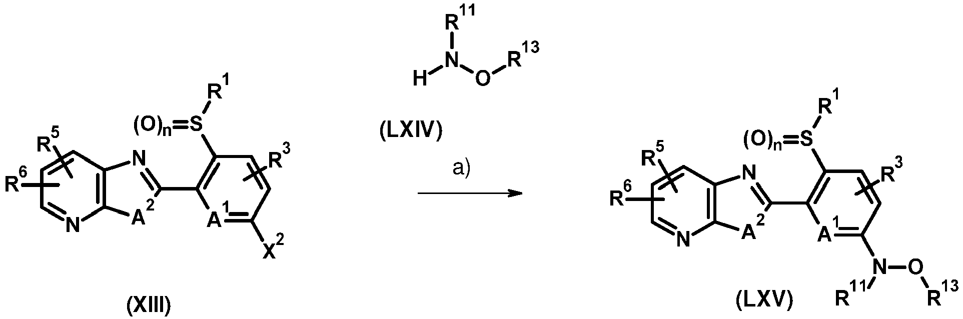

Die Reste R¹, R³, R⁵, R⁶, R¹¹, R¹³, A¹ und n haben die oben beschriebenen Bedeutungen, A² steht für -N-R⁷, O oder S, wobei R⁷ die oben beschriebene Bedeutung hat. X² steht für Halogen.

Die Verbindungen der Formel (LXV) (entspricht Formel I(G15)) lassen sich herstellen durch Umsetzung der Verbindungen der Formel (XIII) mit Verbindungen der Formel (LXIV).

Verbindungen der Formel (LXIV) sind entweder kommerziell erhältlich oder können nach bekannten Methoden hergestellt werden.

Die Umsetzung verläuft unter analogen Reaktionsbedingungen wie im Verfahren E beschrieben.

### Verfahren S

Alternativ zu Verfahren A lassen sich Verbindungen der Formel (I), bei denen X für H4, H5 oder H6 steht folgendermaßen herstellen:

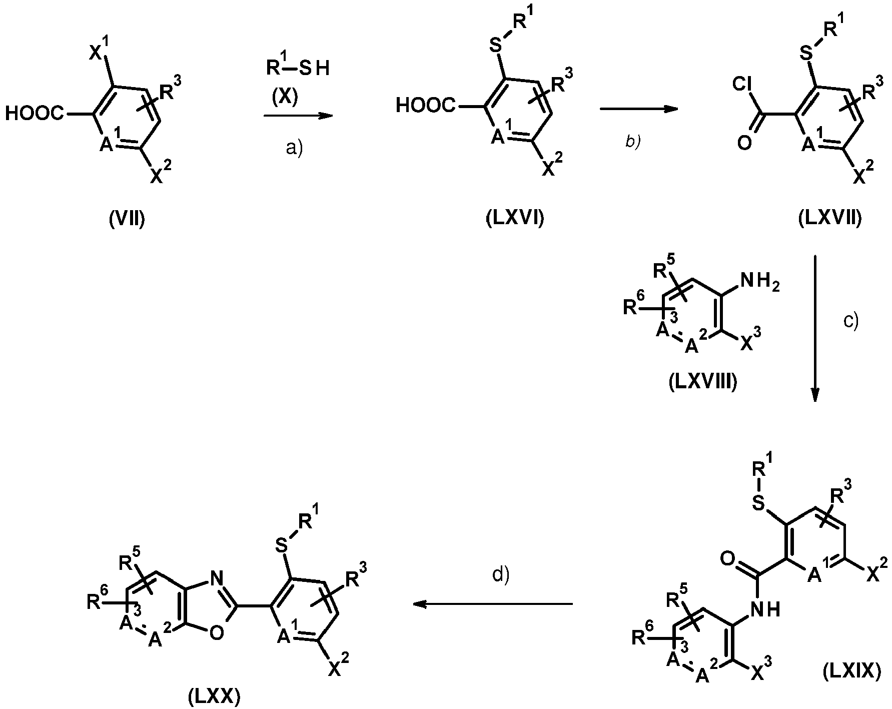

Die Reste R¹, R³, R⁵, R⁶, A¹ und n haben die oben beschriebenen Bedeutungen, A² und A³ stehen für CH oder N, X¹ steht für Halogen, X¹, X² und X³ stehen für Halogen und R⁷ steht für (C₁-C₄)Alkyl.

### Schritt a)

Die Verbindungen der Formel (LXVI) lassen sich herstellen durch Umsetzung der Verbindungen der Formel (VII) mit den Verbindungen der Formel (X) in Gegenwart einer Base.

Mercaptanderivate der Formel (X) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindungen der Formel (LXVI) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

### Schritt b)

Die Verbindungen der Formel (LXVII) können durch die Umsetzung von Verbindungen der Formel (LXVI) nach allgemein üblichen Methoden mit Chlorierungsmitteln wie z.B. Oxalylchlorid oder Thionylchlorid hergestellt werden.

### Schritt c)

Die Verbindungen der Formel (LXIX) können durch die Umsetzung von Verbindungen der Formel (LXVIII) mit Säurechloriden der Formel (LXVII) in Gegenwart einer Base hergestellt werden.

Die Verbindungen der Formel (LXVIII) sind entweder kommerziell erhältlich oder können nach bekannten Methoden hergestellt werden, beispielsweise analog der in US2003/069257, US2012/0319050, WO2011/107998 oder WO2010/91310 beschriebenen Verfahren.

Die Umsetzung der Verbindungen der Formel (LXVIII) mit Säurechloriden der Formel (LXVII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus organischen Aminbasen, basischen aromatischen Heterocyclen, sowie Acetaten, Phosphaten, Carbonaten und Hydrogencarbonaten von Alkali- oder Erdalkalimetallen. Besonders bevorzugt sind dabei Triethylamin, Diisopropylamin, Pyridin, Piperidin, Natriumacetat, Natriumphosphat, Kaliumphosphat, Caesiumcarbonat, Natriumcarbonat, Kaliumcarbonat, Natriumhydrogencarbonat, Kaliumhydrogencarbonat. Weitere geeignete Basen sind Alkalimetallhydride wie z.B. Natriumhydrid und Hydroxide wie NaOH, KOH und LiOH.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

### Schritt d)

Die Verbindungen der Formel (LXX) lassen sich durch Kondensation der Verbindungen der Formel (LXIX) in Gegenwart einer Base herstellen.

Die Umsetzung zu Verbindungen der Formel (LXX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 200 °C durchgeführt werden.

Die weitere Umsetzung von Verbindungen der Formel (LXX) zu Verbindungen der Formel (I) erfolgt analog zu Verfahren A bis M, Q, R, T und U.

### Verfahren T

Die Verbindungen der Formel (I), bei denen X für H1, H2, H4, H5, H6, H7, H8, H9, H10, H11, H14, H15, H16, H17, H19 oder H20 steht, können alternativ nach folgender Methode hergestellt werden. Die Methode wird nachfolgend anhand von Beispiel H10, H11, H15 bzw. H16 beschrieben.

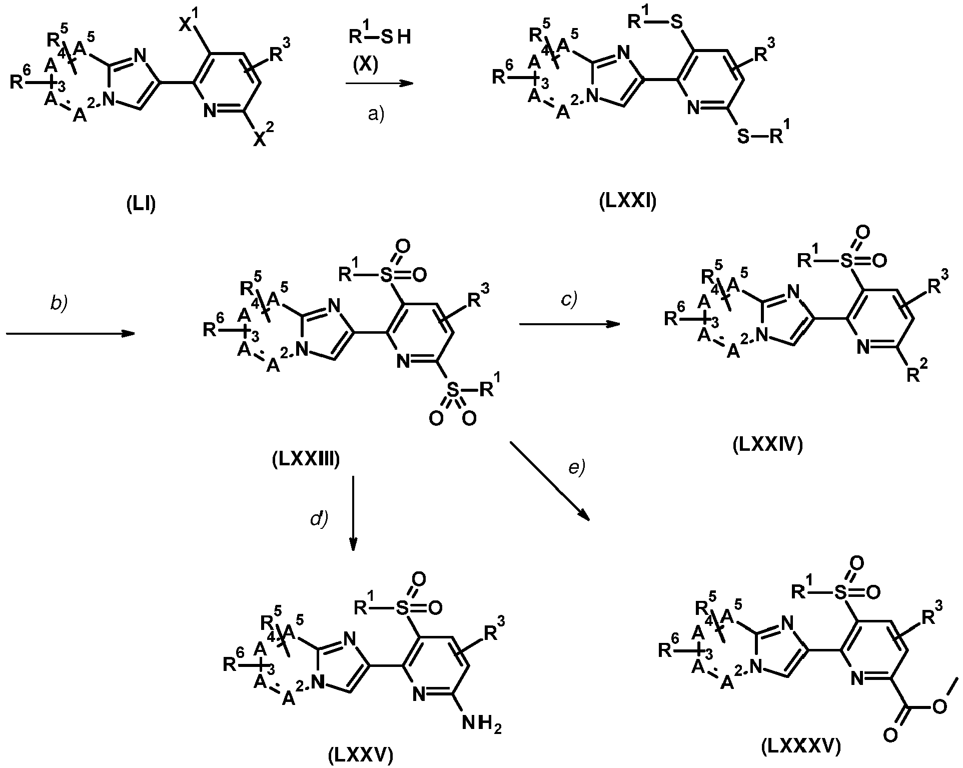

Die Reste R¹, R², R³, R⁵ und R⁶ haben die oben beschriebenen Bedeutungen, A² bis A⁵ stehen für CH oder N, X¹ und X² stehen für Halogen.

### Schritt a)

Analog Verfahren A Schritt c) kann statt eines Sulfids auch das Disulfid der Formel (LXXI) gebildet werden.

### Schritt b)

Das Disulfid kann analog Verfahren A Schritt f) in das Disulfon der Formel (LXXIII) überführt werden.

### Schritt c)

Das Disulfon eignet sich analog wie Verbindungen der Formel (XIII) als Ausgangsprodukt für die Herstellung von Verbindungen der Formel (LXXIV), in denen R² für einen N-verknüpften Ring steht. Siehe hierzu Verfahren A Schritt g).

### Schritt d)

Ebenso kann das Disulfon der Formel (LXXIII) dazu verwendet werden, ein Amin der Formel (LXXV) herzustellen, welches seinerseits als Ausgangsprodukt für weitere Derivatisierungen dienen kann. Die Umsetzung zum Amin der Formel (LXXV) erfolgt analog Verfahren D Schritt a).

### Schritt e)

Analog Verfahren Q Schritt a) kann das Disulfon der Formel (XXIII) auch in Gegenwart eines geeigneten Palladiumkatalysators carbonyliert, und durch Zusatz von Methanol in einen Methylster der Formel (LXXXV) umgesetzt werden.

Das Verfahren T kann auch zur Herstellung von Verbindungen der Formel (IX) angewendet werden.

### Verfahren U

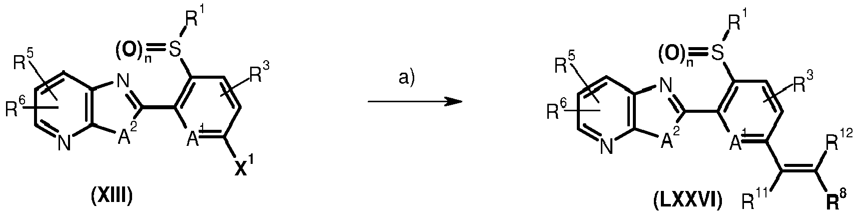

Die Reste R¹, R³, R⁵, R⁶, R⁸, R¹¹, R¹², A¹ und n haben die oben beschriebenen Bedeutungen, A² steht für -N-R⁷, O oder S, wobei R⁷ die oben beschriebene Bedeutung hat und X¹ steht für Halogen.

### Schritt a)

Verbindungen der Formel (LXXVI) (entspricht I(G19)) können beispielsweise aus Verbindungen der Formel (XIII), für die X¹ bevorzugt für Halogen aus der Reihe Chlor oder Brom steht, nach allgemein bekannten Methoden (vgl. Chem. Rev. 1995, 95, 2457-2483; Tetrahedron 2002, 58, 9633-9695; Metal-Catalyzed Cross-Coupling Reactions (Eds.: A. de Meijere, F. Diederich), 2nd ed., Wiley-VCH, Weinheim, 2004) hergestellt werden.

Beispielsweise können Verbindungen der Formel (XIII) mit geeigneten olefinischen Boronsäuren oder deren Estern nach bekannten Methoden (vgl. WO2006/21805) in Gegenwart geeigneter Katalysatoren aus der Reihe der Übergangsmetallsalze zu Verbindungen der Formel (LXXVI), umgesetzt werden. Als Kupplungskatalysatoren kommen beispielsweise bevorzugt Palladiumkatalysatoren wie [1,1'-Bis (diphenylphosphino)ferrocen]dichlorpalladium (II) oder Tetrakis(triphenylphosphin)palladium in Betracht. Als geeignete basische Reaktionshilfsmittel zur Durchführung der Verfahren finden bevorzugt Carbonate des Natriums oder Kaliums Verwendung.

Die benötigten olefinischen Boronsäuren oder olefinischen Boronsäureester sind teilweise bekannt und/oder kommerziell erhältlich bzw. können nach allgemein bekannten Methoden hergestellt werden (vgl. Boronic Acids (Eds.: D. G. Hall), 2nd ed., Wiley-VCH, Weinheim, 2011).

### Verfahren V

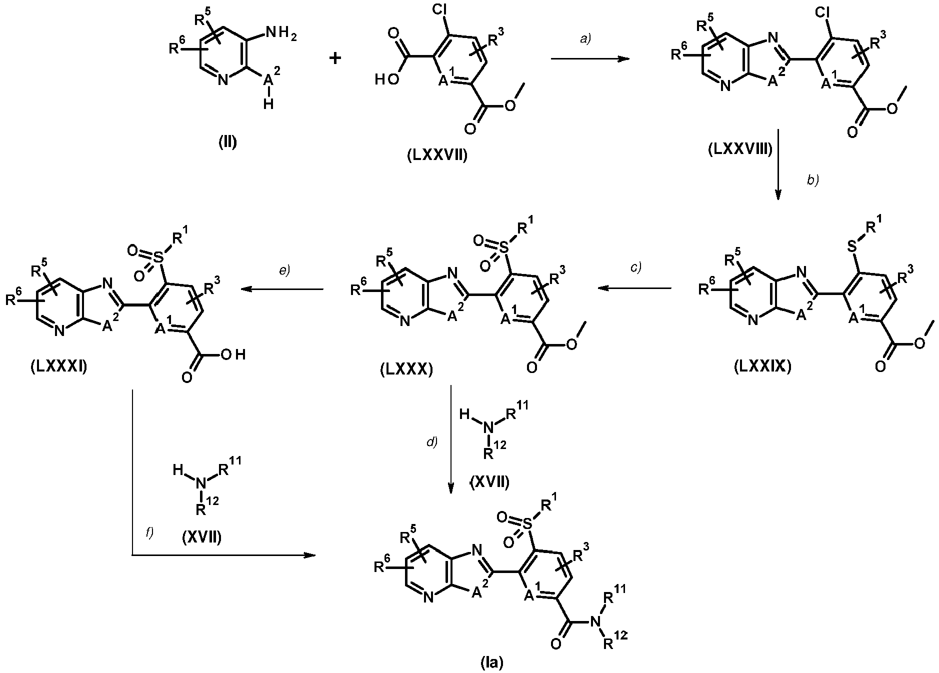

Die Reste R¹, R³, R⁵, R⁶, R¹¹, R¹², A¹ haben die oben beschriebenen Bedeutungen. A² steht für -N-R⁷, O oder S.

### Schritt a)

Analog Verfahren A Schritt a) und b) kann ausgehend von Verbindungen der Formel (LXXVII) statt der Formel (VII) Verbindungen der Formel (LXXVIII) gebildet werden.

Verbindungen der Formel (LXXVII) sind entweder kommerziell erhältlich oder können nach bekannten Methoden hergestellt werden, siehe zum Beispiel European Journal of Medicinal Chemistry, 2015, (90), 170.

### Schritt b)

Verbindungen der Formel (LXXVIII) können analog Verfahren A, Schritt c) in das Sulfid der Formel (LXXIX) umgesetzt werden.

### Schritt c)

Verbindungen der Formel (LXXIX) können analog Verfahren A Schritt f) zum Sulfon der Formel (LXXX) oxidiert werden.

### Schritt d)

Im Falle dass R¹¹ = H und R¹² = Alkyl (bevorzugt Methyl) steht, können Verbindungen der Formel (LXXX) direkt durch Umsetzung mit einem entsprechenden Amin der Formel (XVII) in einem geeigneten Lösemittel und optional in Gegenwart eines Katalysators wie zum Beispiel MgCl₂ zu Verbindungen der Formel (Ia) (entspricht I(G3) umgesetzt werden. Siehe hierzu zum Beispiel WO2004/37789, US2003/144278 oder US2013/35492.

### Schritt e)

Alternativ zu Schritt d) können Verbindungen der Formel (LXXX) nach allgemein bekannten Methoden zur Säure (Verbindungen der Formel (LXXXI)) verseift werden.

### Schritt f)

Für die Umsetzung zum Amid der Formel (Ia) (entspricht I(G3)) steht ausgehend von Verbindungen der Formel (LXXXI) eine Vielzahl von Methoden zur Verfügung. Es sei beispielsweise auf allgemein übliche Methoden zur Herstellung von Amiden aus Säuren in Gegenwart eines Kondensationsmittels wie beispielsweise eines Carbodiimids wie 1-(3-Dimethylaminopropyl)-3-ethylcarbodiimid hydrochlorid (EDCI) oder 1,3-Dicyclohexylcarbodiimid (CDI) und einer Base wie beispielsweise N,N-Dimethylpyridin-4-amin (DMAP) hingewiesen.

### Verfahren W

Alternativ zu Verfahren P lassen sich Verbindungn der Formel (I), bei denen X für H3, H18 oder H20 steht folgendermaßen herstellen:

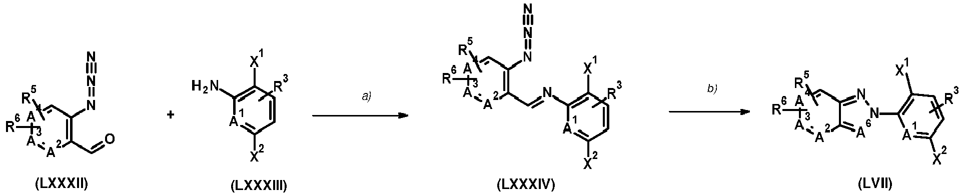

Die Reste R⁵, R⁶, A¹ haben die oben beschriebenen Bedeutungen, A² bis A⁴ stehen für CH oder N, A⁶ steht ausschließlich für CH, X¹ und X² stehen für Halogen.

### Schritt a)

Verbindungen der Formel (LXXXIV) können durch Kondensation von Verbindungen der Formel (LXXXII) mit Verbindungen der Formel (LXXXIII) hergestellt werden, wie beschrieben in Chemical Communications, 2011, vol. 47, p. 10133 - 10135, WO2012/66061 A1, 2012, Tetrahedron, 1992, vol. 48, 3091 - 3110.

Verbindungen der Formel (LXXXII) sind entweder kommerziell erhältlich oder können nach bekannten Methoden hergestellt werden, siehe zum Beispiel Methoden analog WO2012/66061 oder Australian Journal of Chemistry, 1980, 33, 91.

Verbindungen der Formel (LXXXIII) sind entweder kommerziell erhältlich oder können nach bekannten Methoden hergestellt werden, siehe zum Beispiel WO2007/87549.

Die Kondensation kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt sind Alkohole wie Ethanol oder Methanol oder Ether wie THF.

Die Reaktion wird durch Einsatz von Broenstedt- oder Lewissäuren katalysiert, besonders bevorzugt sind Essigsäure und Titansalze wie Titantetrachlorid oder Titantetraethanolat.

### Schritt b)

Analog zu WO2012/66061 A1, oder Organic Letters, 2011, vol. 13, p. 3542 - 3545 können Verbindungen der Formel (LXXXIV) zu Verbindungen der Formel (LVII) cyclisiert werden. Die Cyclisierung kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Besonders bevorzugt sind hochsiedende Lösemittel wie Toluol oder DMSO.

Die Reaktion kann im Vakuum, bei Normaldruck oder unter Überdruck und bei Temperaturen von 0 °C bis 200 °C durchgeführt werden. Bevorzugt ist eine Temperatur größer als 100°C.

Die Reaktion kann durch Zusatz eines Kupfer(I)-Salzes katalysiert werden.

Die weitere Umsetzung von Verbindungen der Formel (LVII) zu Verbindungen der Formel (I) erfolgt analog zu Verfahren A bis M, Q, R, T und U.

Die Verbindungen der Formeln (I) bis (LXXXIV) in den Schemata A bis W sind beispielhaft anhand spezieller X-Substituenten aus der Reihe H1 bis H20 beschrieben, gelten aber auch für die analogen X-Substituenten aus der Reihe H1 bis H20.

Gegenstand der Erfindung sind auch Verbindungen der Formel (IX) 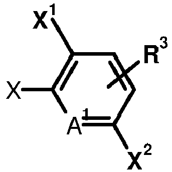 in welcher
X, A¹ und R³ die oben beschriebenen Bedeutungen haben, X¹ steht für Halogen und X² steht für Halogen.

Bevorzugt steht A¹ für N, R³ für Wasserstoff, X¹ für Brom, Fluor oder Chlor, X² für Fluor oder Chlor.

Besonders bevorzugt steht A¹ für N, R³ für Wasserstoff, X¹ für Fluor oder Chlor, X² für Fluor oder Chlor, X hat die oben genannte Bedeutung, wobei X nicht für H1, H2, H4, H5, H6, H7, H8, H9 oder H14 steht.

Gegenstand der Erfindung sind auch Verbindungen der Formel (XI) 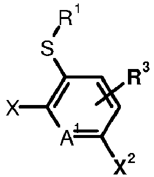 in welcher
X, A¹, R¹ und R³ die oben beschriebenen Bedeutungen haben und X² steht für Halogen.

Bevorzugt steht A¹ für N, R¹ für Ethyl, R³ für Wasserstoff, X² für Fluor oder Chlor.

Besonders bevorzugt steht A¹ für N, R¹ für Ethyl, R³ für Wasserstoff, X² für Fluor oder Chlor, X hat die oben genannte Bedeutung, wobei X nicht für H1, H2, H4, H5, H6, H7, H8, H9 oder H14 steht.

Gegenstand der Erfindung sind auch Verbindungen der Formel (XIII) 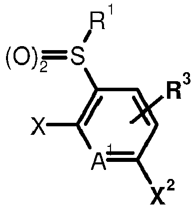 in welcher
X, A¹, R¹ und R³ die oben beschriebenen Bedeutungen haben, X² steht für Halogen.

Bevorzugt steht A¹ für N, R¹ für Ethyl, R³ für Wasserstoff, X² für Fluor oder Chlor.

Besonders bevorzugt steht A¹ für N, R¹ für Ethyl, R³ für Wasserstoff, X² für Fluor oder Chlor, X hat die oben genannte Bedeutung, wobei X nicht für H1, H2, H4, H5, H6, H7, H8, H9 oder H14 steht.

Gegenstand der Erfindung sind auch Verbindungen der Formel (LXXIII) 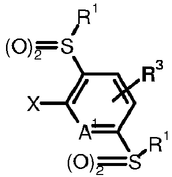 in welcher
X, A¹, R¹ und R³ die oben beschriebenen Bedeutungen haben.

Bevorzugt steht A¹ für N, R¹ für Ethyl, R³ für Wasserstoff.

Besonders bevorzugt steht A¹ für N, R¹ für Ethyl, R³ für Wasserstoff, X hat die oben genannte Bedeutung, wobei X nicht für H1, H2, H4, H5, H6, H7, H8, H9 oder H14 steht.

Gegenstand der Erfindung sind auch Verbindungen der Formel (LXXI) 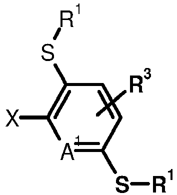 in welcher
X, A¹, R¹ und R³ die oben beschriebenen Bedeutungen haben.

Bevorzugt steht A¹ für N, R¹ für Ethyl, R³ für Wasserstoff.

Besonders bevorzugt steht A¹ für N, R¹ für Ethyl, R³ für Wasserstoff, X hat die oben genannte Bedeutung, wobei X nicht für H1, H2, H4, H5, H6, H7, H8, H9 oder H14 steht.

Gegenstand der Erfindung sind auch Verbindungen der Formel (LXXIX) 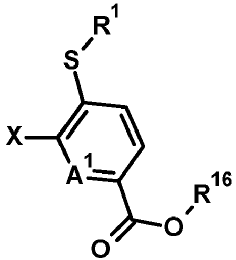 in welcher
X, A¹ und R¹ die oben beschriebenen Bedeutungen haben und R¹⁶ steht für Methyl, Ethyl oder Propyl.

Bevorzugt steht A¹ für N, R¹ für Ethyl, R¹⁶ für Methyl.

Gegenstand der Erfindung sind auch Verbindungen der Formel (LXXX) 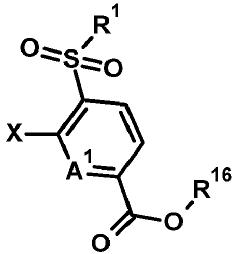 in welcher
X, A¹ und R¹ die oben beschriebenen Bedeutungen haben und R¹⁶ steht für Methyl, Ethyl oder Propyl.

Bevorzugt steht A¹ für N, R¹ für Ethyl, R¹⁶ für Methyl.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung wird unter dem Begriff "Hygiene" die Gesamtheit aller Maßnahmen, Verfahren und Verhaltensweisen verstanden, die das Ziel haben, Erkrankungen - insbesondere Infektionskrankheiten - zu vermeiden und der Gesunderhaltung des Menschen, Tieres und/oder der Umwelt zu dienen und/oder die Sauberkeit zu erhalten. Hierunter fallen erfindungsgemäß insbesondere Maßnahmen zur Reinigung, Desinfektion und Sterilisation beispielsweise von Textilien oder harten Oberflächen, vornehmlich aus Glas, Holz, Beton, Porzellan, Keramik, Kunststoff oder auch aus Metall(en), und deren Reinhaltung von Hygieneschädlingen bzw. deren Fäkalien. Erfindungsgemäß ausgeschlossen sind diesbezüglich wiederum Verfahren zur chirurgischen oder therapeutischen Behandlung des menschlichen oder tierischen Körpers und Diagnostizierverfahren, die am menschlichen oder tierischen Körper vorgenommen werden.

Der Begriff "Hygienesektor" umfasst demnach alle Bereiche, technischen Gebiete und gewerblichen Nutzungen, in denen derartige Maßnahmen, Verfahren und Verhaltensweisen zur Hygiene von Wichtigkeit sind, wie beispielsweise die Hygiene in Küchen, Bäckereien, Flughäfen, Bädern, Schwimmbädern, Einkaufshäusern, Hotels, Krankenhäusern, Stallungen, etc.

Unter dem Begriff "Hygieneschädling" werden folglich ein oder mehrere tierischer(e) Schädling(e) verstanden, deren Vorhandensein im Hygienesektor insbesondere aus gesundheitlichen Gründen problematisch ist. Es ist folglich das vornehmliche Ziel, Hygieneschädlinge bzw. den Kontakt mit ihnen im Hygienesektor zu vermeiden bzw. zu minimieren. Dies kann insbesondere durch die Anwendung eines Schädlingsbekämpfungsmittels erfolgen, wobei das Mittel sowohl prophylaktisch als auch erst bei Befall zur Bekämpfung des Schädlings eingesetzt werden kann. Es ist ebenfalls möglich, Mittel einzusetzen, die bewirken, dass ein Kontakt mit dem Schädling vermieden oder reduziert wird. Als Hygieneschädlinge kommen beispielsweise die nachstehend genannten Organismen in Betracht.

Der Begriff "Hygieneschutz" umfasst demnach alle Handlungen zur Aufrechterhaltung und/oder Verbesserung von derartigen Maßnahmen, Verfahren und Verhaltensweisen zur Hygiene.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z.B. Periplaneta americana, Periplaneta australasiae, Pycnoscelus surinamensis, Supella longipalpa;
aus der Ordnung der Coleoptera z.B. Acalymma vittatum, Acanthoscelides obtectus, Adoretus spp., Aethina tumida, Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Listronotus (= Hyperodes) spp., Lixus spp., Luperodes spp., Luperomorpha xanthodera, Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oulema spp., z.B. Oulema melanopus, Oulema oryzae, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Dermaptera z.B. Anisolabis maritime, Forficula auricularia, Labidura riparia;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya oder Pegomyia spp., z.B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex, Toxotrypana curvicauda;
aus der Ordnung der Hemiptera z.B. Acizzia acaciaebaileyanae, Acizzia dodonaeae, Acizzia uncatoides, Acrida turrita, Acyrthosiphon spp., z.B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lopholeucaspis japonica, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Neomaskellia spp., Nephotettix spp., z.B. Nephotettix cincticeps,, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Perkinsiella spp., Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pulvinaria spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Aelia spp., Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Megacopta cribraria, Miridae, Monalonion atratum, Nezara spp., z.B. Nezara viridula, Nysius spp., Oebalus spp., Pentomidae, Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Camponotus spp., Dolichovespula spp., Diprion spp., z.B. Diprion similis, Hoplocampa spp., z.B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z.B. Vespa crabro, Wasmannia auropunctata, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z.B. Ostrinia nubilalis, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (= Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Chaetanaphothrips leeuweni, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, z.B. Ancylostoma duodenale, Ancylostoma ceylanicum, Acylostoma braziliensis, Angiostrongylus spp., Anisakis spp., Anoplocephala spp., Ascaris spp., Ascaridia spp., Baylisascaris spp., Brugia spp., z. B. Brugia malayi, Brugia timori, Bunostomum spp., Capillaria spp., Chabertia spp., Clonorchis spp., Cooperia spp., Crenosoma spp., Cyathostoma spp., Dicrocoelium spp., Dictyocaulus spp., z. B. Dictyocaulus filaria, Diphyllobothrium spp., z. B. Diphyllobothrium latum, Dipylidium spp., Dirofilaria spp., Dracunculus spp., z. B. Dracunculus medinensis, Echinococcus spp., z.B. Echinococcus granulosus, Echinococcus multilocularis, Echinostoma spp., Enterobius spp., z.B. Enterobius vermicularis, Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., z.B. Hymenolepis nana, Hyostrongylus spp., Litomosoides spp., Loa spp., z.B. Loa Loa, Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, z.B. Onchocerca volvulus, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., z.B. Strongyloides fuelleborni, Strongyloides stercoralis, Strongylus spp., Syngamus spp., Taenia spp., z.B. Taenia saginata, Taenia solium, Teladorsagia spp., Thelazia spp., Toxascaris spp., Toxocara spp., Trichinella spp., z.B. Trichinella spiralis, Trichinella nativa, Trichinella britovi, Trichinella nelsoni, Trichinella pseudopsiralis, Trichobilharzia spp., Trichostrongylus spp., Trichuris spp., z.B. Trichuris trichuria, Uncinaria spp., Wuchereria spp., z.B. Wuchereria bancrofti;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hirschmaniella spp., Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z.B. Paratrichodorus minor, Paratylenchus spp., Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia, z.B. Eimeria spp., bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamineethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Momfluorothrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Broflanilide, Bromopropylate, Chinomethionat, Cryolite, Cyclaniliprole, Cycloxaprid, Cyhalodiamide Dicloromezotiaz, Dicofol, Diflovidazin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, Lotilaner, Meperfluthrin, Paichongding, Pyflubumide, Pyridalyl, Pyrifluquinazon, Pyriminostrobin, Sarolaner, Tetramethylfluthrin, Tetraniliprole, Tetrachlorantraniliprole, Tioxazafen, Thiofluoximate, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 4-[5-(3,5-Dichlorphenyl)-5-(trifluormethyl)-4,5-dihydro-1 ,2-oxazol-3-yl] -2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, Butyl-[2-(2,4-dichlorphenyl)-3-oxo-4-oxaspiro[4.5]dec-1-en-1-yl]-carbonat (bekannt aus CN 102060818), 3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926), 5-Brom-4-chlor-N-[4-chlor-2-methyl-6-(methylcarbamoyl)phenyl] -2-(3-chlor-2-pyridyl)pyrazole-3-carboxamid (bekannt aus CN103232431).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazol-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl] -2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2- [4-(4-Chlorphenoxy)-2-(trifluormethyl)phenyl]-1-(1H-1,2,4-triazol-1-yl)butan-2-ol, (1.86) 2- [4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,SS)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{ [4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid,(2.36)1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylacetamid, (3.24) (2E)-2-(Methoxyimino)-N-methyl-2-(2-{[({(1E)-1-[3-(trifluormethyl)phenyl]ethyliden}amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy]-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothal-isopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlornicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({ [(1-Methyl-1H-tetrazol-5-yl)(phenyl)methylen]amino}oxy)methyl]pyridin-2-yl}-carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]amino}-oxy)methyl]pyridin-2-yl}carbamidsäure-tert.-butylester, (15.092) (5-Brom-2-methoxy-4-methylpyridin-3-yl)(2,3,4-trimethoxy-6-methylphenyl)methanon, (15.093) N-[2-(4-{[3-(4-Chlorphenyl)prop-2-in-1-yl]oxy)-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]-amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dibydro-1H-inden-2-yloxy)-2-methylpyridin-3-yl] -N-ethyl-N-methylimidoformamid, (15.107) N'- {5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-l-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimetbyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimetbyl-1H-pyrazol-5-amin, (15.144) 4-(2-Brom-4-fluorphenyl)-N-(2-brom-6-fluorphenyl)-1,3-dimetbyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{ [3-(2,2,3,3-tetrafluorpropoxy)phenyl] sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]acetyl}piperidin-4-yl)-1 ,3-thiazol-4-yl]-4,5-dihydro-1 ,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1- [4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(SR)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(SR)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika und Chili, Gurken, Melonen, Karotten, Wassermelonen, Zwiebel, Salat, Spinat, Lauch, Bohnen, *Brassica oleracea* (z.B. Kohl) und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien der Pflanzen verstanden werden, beispielsweise Samen, Stecklinge sowie junge (unreife) Pflanzen bis hin zu reifen Pflanzen. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut (geerntete Pflanzen oder Pflanzenteile) sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Be-handlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formu-lierungen enthalten sein können, kommen alle für derartige Zwecke in agrochemischen Mitteln ein-setzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Anthelminthische Mischungspartner

Beispielhaft seien folgende anthelmintische Mischungspartner genannt:
Anthelminthische Wirkstoffe, einschließlich trematicide und cestocide Wirkstoffe:
aus der Klasse der **macrocyclischen Lactone,** z. B.: Abamectin, Doramectin, Emamectin, Eprinomectin, Ivermectin, Milbemycin, Moxidectin, Nemadectin, Selamectin;
aus der Klasse der **Benzimidazole** und **Probenzimidazole,** z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;
aus der Klasse der **Cyclooctadepsipeptide,** z. B.: Emodepsid, PF1022;
aus der Klasse der **Aminoacetonitril-Derivate,** z. B.: Monepantel;
aus der Klasse der **Tetrahydropyrimidine,** z. B.: Morantel, Pyrantel, Oxantel;
aus der Klasse der **Imidazothiazole,** z. B.: Butamisol, Levamisol, Tetramisol;
aus der Klasse der **Salicylanilide,** z. B.: Bromoxanid, Brotianid, Clioxanid, Closantel, Niclosamid, Oxyclozanid, Rafoxanid, Tribromsalan;
aus der Klasse der **Paraherquamide,** z. B.: Derquantel, Paraherquamid;
aus der Klasse der **Aminophenylamidine,** z. B.: Amidantel, deacyliertes Amidantel (dAMD), Tribendimidin;
aus der Klasse der **Organophosphate,** z. B.: Coumaphos, Crufomat, Dichlorvos, Haloxon, Naphthalofos, Trichlorfon;
aus der Klasse der **substituierten Phenole,** z. B.: Bithionol, Disophenol, Hexachlorophen, Niclofolan, Meniclopholan, Nitroxynil;
aus der Klasse der **Piperazinone,** z. B.: Praziquantel, Epsiprantel;
aus anderen **diversen Klassen,** z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel I-1

### 2-{3-(Ethylsulfonyl)-6-[4-(trifluormethyl)-1H-pyrazol-1-yl]pyridin-2-yl}-1-methyl-5-(trifluormethyl)-1H-benzimidazol

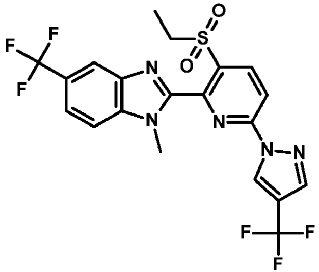

55 mg 2-[3-(Ethylsulfonyl)-6-fluorpyridin-2-yl]-1-methyl-5-(trifluormethyl)-1H-benzimidazol (0.14 mmol) wurden in 2 ml Acetonitril gelöst. Es wurden 29 mg (0.21 mmol) 4-(Trifluoromethyl)-1H-pyrazol und 59 mg (0,42 mmol) Kaliumcarbonat zugegeben und für 3 h bei Raumtemperatur gerührt. Das Gemisch wurde filtriert, eingeengt und säulenchromatographisch mit einem Cyclohexan / Ethylacetat Gradienten als Laufmittel gereinigt.
logP (neutral): 4.16; MH⁺: 504; ¹H-NMR (400MHz, D6-DMSO) δ ppm: 9.38 (s, 1H), 8.712 (d, 1H), 8.46 (s, 1H), 8.42 (d, 1H), 8.16 (s, 1H), 7.98 (d, 1H), 7.70 (dd, 1H), 3.906 (q, 2H), 3.90 (s, 3H), 1.24 (t, 3H).

### Beispiel I-2

### N-{5-(Ethylsulfonyl)-6-[1-methyl-5-(trifluormethyl)-1H-benzimidazol-2-yl]pyridin-2-yl}acetamid

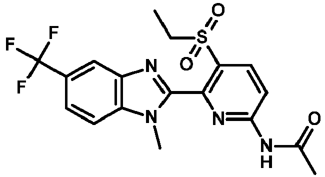

50 mg (0.13 mmol, 1 eq.) 5-(Ethylsulfonyl)-6-[1-methyl-5-(trifluormethyl)-1H-benzimidazol-2-yl]pyridin-2-amin wurden in einem Gemisch aus 1 ml Toluol und 1 ml Pyridin gelöst. Es wurden 16 mg (0.13 mmol, 1 eq.) 4-Dimethylaminopyridin und 30 mg (0.39 mmol, 3 eq.) Acetylchlorid zugegeben und 1 h auf 100°C erhitzt. Nach Abkühlen auf Raumtemperatur wurde die Reaktionsmischung eingeengt und säulenchromatographisch mit einem Cyclohexan / Ethylacetat Gradienten als Laufmittel aufgereinigt.
(logP (neutral): 2.5; MH⁺: 427; ¹H-NMR(400 MHz, D₆-DMSO) δ ppm: 11.26 (s, 1H), 8.48 (m, 2H), 8.12 (s, 1H), 7.93 (d, 1H), 7.72 (d, 1H), 3.76 (s, 3H), 3.69 (q, 2H), 2.17 (s, 3H), 1.17 (t, 3H)).

### Beispiel I-3

### 5-(Ethylsulfonyl)-6-[1-methyl-5-(trifluormethyl)-1H-benzimidazol-2-yl]pyridin-2-amin

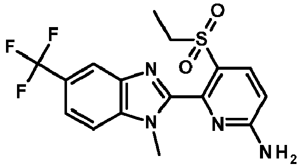

400 mg 2-[3-(Ethylsulfonyl)-6-fluorpyridin-2-yl]-1-methyl-5-(trifluormethyl)-1H-benzimidazol (1.03 mmol, 1 eq.) wurden in 2 ml Ethanol gelöst und es wurden 1.33 g Ammoniaklösung (wässrig, 33%, 25.8 mmol, 25 eq.) zugegeben. Unter Rühren wurde in der Mikrowelle für 1 h auf 100°C erhitzt. Nach Abkühlen auf Raumtemperatur wurde die Lösung eingeengt und Säulenchromatographisch gereinigt (Laufmittel: Gradient Cyclohexan/Ethylacetat).
(logP (neutral): 2.18; MH⁺: 385; ¹H-NMR(400 MHz, D₆-DMSO) δ ppm: 8.07 (s, 1H), 7.91 (m, 2H), 7.68 (d, 1H), 7.36 (s, 1H), 6.74 (d, 2H), 3.71 (s, 3H), 3.51 (q, 2H), 1.13 (t, 3H)).

### 2-[3-(Ethylsulfonyl)-6-fluorpyridin-2-yl]-1-methyl-5-(trifluormethyl)-1H-benzimidazol (Bsp. XIII-1)

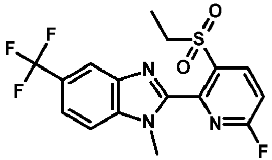

80 mg A (0.21 mmol) 2-[3-(Ethylsulfanyl)-6-fluorpyridin-2-yl]-1-methyl-5-(trifluormethyl)-1H-benzimidazol wurden bei Raumtemperatur in 10 ml Dichlormethan gelöst. Es wurden 49 mg (1,06 mmol) Ameisensäure und 0,13 ml 35% wässrige Wasserstoffperoxid-Lösung (1,49 mmol) zugegeben. Nach Rühren für 4 h bei Raumtemperatur (DC-Kontrolle: Umsetzung vollständig) wurden 1 ml Wasser und dann langsam 2 ml 40% Natriumbisulfitlösung zugegeben um die Reaktion zu beenden. Das Gemisch wurde für 1 h gerührt, dann wurden die Phasen getrennt. Die wässrige Phase wurde 3 Mal mit Dichlormethan extrahiert, anschließend wurden die vereinigten organischen Extrakte mit gesättigter NaHCO₃-Lösung gewaschen. Die organische Phase wurde über MgSO₄ getrocknet, filtriert und im Vakuum vom Lösemittel befreit.
logP (neutral): 2.96; MH⁺: 388; ¹H-NMR(400 MHz, D₆-DMSO) δ ppm: 8.69 (m, 1H), 8.14 (s, 1H), 7.94 (d, 1H), 7.75 (m, 2H), 3.83 (q, 2H), 3.81 (s, 3H), 1.21 (t, 3H).

### 2-[3-(Ethylsulfanyl)-6-fluorpyridin-2-yl]-1-methyl-5-(trifluormethyl)-1H-benzimidazol (Bsp. XI-1)

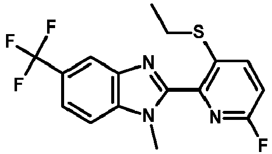

500 mg (1.34 mmol) 2-(3,6-Difluorpyridin-2-yl)-1-methyl-5-(trifluormethyl)-1H-benzimidazol wurden in 5ml trockenem THF gelöst und auf -10°C gekühlt. 56 mg (60%, 1,39 mmol) NaH wurden zugegeben und das Gemisch unter Rühren auf Raumtemperatur erwärmt. Sehr langsam wurden 91 mg (1.47 mmol) Ethylmercaptan, gelöst in 3 ml trockenem THF, zugetropft und es wurde für 3 h bei Raumtemperatur gerührt. Das Reaktionsgemisch wurde mit Ethylacetat verdünnt und zwei mal mit gesättigter NaCl-Lösung gewaschen. Nach Trocknen der organischen Phase wurde diese im Vakuum aufkonzentriert und der Rückstand säulenchromatographisch aufgereinigt (Laufmittel: Gradient Cyclohexan/Ethylacetat).
(logP (neutral): 3.44; MH⁺: 356; ¹H-NMR(400 MHz, D₆-DMSO) δ ppm: 7.66 (dd, 1H), 7.61 (dt, 1H), 7.56 (dd, 1H), 7.52 (dd, 1H), 4.26 (q, 2H), 3.44 (s, 3H), 1.29 (t, 3H).

### 2-(3,6-Difluorpyridin-2-yl)-1-methyl-5-(trifluormethyl)-1H-benzimidazol (Bsp. IX-1)

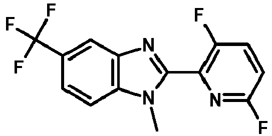

Unter Argon wurden 5.00 g (26.2 mmol) N1-Methyl-4-(trifluoromethyl)benzol-1,2-diamin in 107 ml trockenem Pyridin gelöst und es wurden 4.18 g (26,2 mmol) 3,6-Difluoropyridin-2-carbonsäure sowie 5.04 g (26.2 mmol) N-(3-Dimethylaminopropyl)-N'-ethylcarbodiimid hydrochlorid zugegeben. Das Gemisch wurde für 9 h bei 120°C gerührt. Nach Abkühlen auf Raumtemperatur wurde der Ansatz unter Vakuum aufkonzentriert und anschließend mit Ethylacetat verdünnt. Die organische Phase wurde mit Wasser gewaschen, über MgSO₄ getrocknet und im Vakuum eingeengt. Das Produkt kann durch Umkristallisation aus Methylterbutylether teilweise aufgereinigt werden.
(logP (neutral): 2.83; MH⁺: 314; ¹H-NMR(400 MHz, D₆-DMSO) δ ppm:8.26 (m, 1H), 8.16 (s, 1H), 7.93 (d, 1H), 7.72 (d, 1H), 7.57 (m, 1H), 4.02 (s, 3H).

### Beispiel I-4

### 2-{3-(Ethylsulfonyl)-6-[4-(trifluormethyl)-1H-pyrazol-1-yl]pyridin-2-yl}-3-methyl-6-(trifluormethyl)-3H-imidazo[4,5-b]pyridin

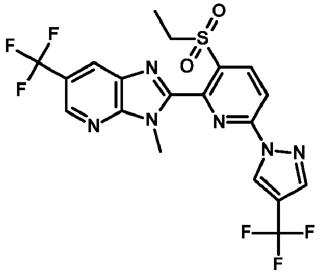

Gemäß der obigen Vorschrift zur Herstellung von 2-{3-(Ethylsulfonyl)-6-[4-(trifluormethyl)-1H-pyrazol-1-yl]pyridin-2-yl)-1-methyl-5-(trifluormethyl)-1H-benzimidazol lässt sich ebenfalls die Verbindung 2-{3-(Ethylsulfonyl)-6-[4-(trifluormethyl)-1H-pyrazol-1-yl]pyridin-2-yl}-3-methyl-6-(trifluormethyl)-3H-imidazo[4,5-b]pyridin herstellen.
(logP (neutral): ; 4.04; MH⁺: 505; ¹H-NMR(400 MHz, D₆-DMSO) δ ppm: 9.42 (s, 1H), 8.94 (s, 1H), 8.73 (m, 2H), 8.45 (m, 2H), 3.91 (m, 5H), 1.25 (t, 3H)

### Beispiel 1-5

### N-{5-(Ethylsulfonyl)-6-[3-methyl-6-(trifluormethyl)-3H-imidazo[4,5-b]pyridin-2-yl]pyridin-2-yl}acetamid

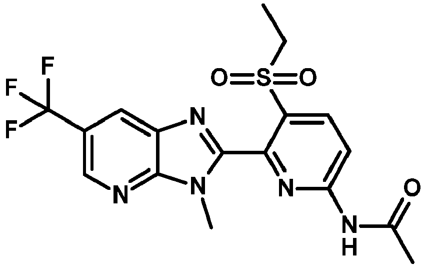

Gemäß der obigen Vorschrift zur Herstellung von N-{5-(Ethylsulfonyl)-6-[1-methyl-5-(trifluormethyl)-1H-benzimidazol-2-yl]pyridin-2-yl}acetamid lässt sich ebenfalls die Verbindung N-{5-(Ethylsulfonyl)-6-[3-methyl-6-(trifluormethyl)-3H-imidazo[4,5-b]pyridin-2-yl]pyridin-2-yl}acetamid herstellen.
(logP (neutral): 2.3; MH⁺: 428; ¹H-NMR(400 MHz, D₆-DMSO) δ ppm: 11.29 (s, 1H), 8.89 (s, 1H), 8.68 (s, 1H), 8.51 (q, 2H), 3.78 (s, 3H), 3.66 (q, 2H), 2.17 (s, 3H), 1.18 (t, 3H).

### Beispiel I-6

### 5-(Ethylsulfonyl)-6-[3-methyl-6-(trifluormethyl)-3H-imidazo[4,5-b]pyridin-2-yl]pyridin-2-amin

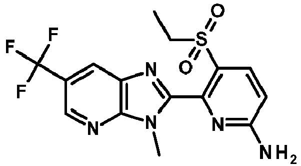

Gemäß der obigen Vorschrift zur Herstellung von 5-(Ethylsulfonyl)-6-[1-methyl-5-(trifluormethyl)-1H-benzimidazol-2-yl]pyridin-2-amin lässt sich ebenfalls 5-(Ethylsulfonyl)-6-[3-methyl-6-(trifluormethyl)-3H-imidazo[4,5-b]pyridin-2-yl]pyridin-2-amin herstellen.
(logP (neutral): 2.02; MH⁺: 386; ¹H-NMR(400 MHz, D₆-DMSO) δ ppm: 8.84 (s, 1H), 8.61 (s, 1H), 7.93 (d, 1H), 7.42 (b-s, 1H), 6.76 (d, 1H), 3.73 (s,3H), 3.49 (q, 2H); 1.14 (t, 3H).

### 2-[3-(Ethylsulfonyl)-6-fluorpyridin-2-yl]-3-methyl-6-(trifluormethyl)-3H-imidazo[4,5-b]pyridin (Bsp. XIII-2)

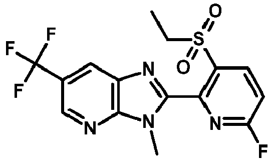

Gemäß der obigen Vorschrift zur Herstellung von 2-[3-(Ethylsulfonyl)-6-fluorpyridin-2-yl]-1-methyl-5-(trifluormethyl)-1H-benzimidazol lässt sich ebenfalls 2-[3-(Ethylsulfonyl)-6-fluorpyridin-2-yl]-3-methyl-6-(trifluormethyl)-3H-imidazo[4,5-b]pyridin herstellen.
(logP (neutral): 2.80; MH⁺: 389; ¹H-NMR(400 MHz, D₆-DMSO) δ ppm: 8.91 (m, 1H), 8.70 (m, 2H), 7.81 (dd, 1H), 3.82 (m, 5H), 1.22 (t, 3H)

### 2-[3-(Ethylsulfanyl)-6-fluorpyridin-2-yl]-3-methyl-6-(trifluormethyl)-3H-imidazo[4,5-b]pyridin (Bsp. XI-2)

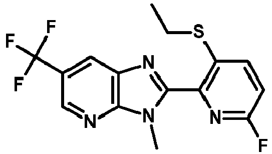

Gemäß der obigen Vorschrift zur Herstellung von 2-[3-(Ethylsulfanyl)-6-fluorpyridin-2-yl]-1-methyl-5-(trifluormethyl)-1H-benzimidazol lässt sich ebenfalls die Verbindung 2-[3-(Ethylsulfanyl)-6-fluorpyridin-2-yl]-3-methyl-6-(trifluormethyl)-3H-imidazo[4,5-b]pyridin herstellen.
(logP (neutral): 3.45; MH⁺: 357; ¹H-NMR(400 MHz, D₆-DMSO) δ ppm: 8.87 (s, 1H), 8.68 (m, 1H), 8.29 (m, 1H), 7.49 (dd, 1H), 3.94 (s, 3H), 3.01 (q, 2H), 1.19 (t, 3H).

### 2-(3,6-Difluorpyridin-2-yl)-3-methyl-6-(trifluormethyl)-3H-imidazo[4,5-b]pyridin (Bsp. IX-2)

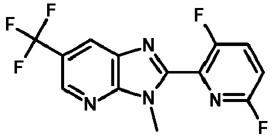

Gemäß der obigen Vorschrift zur Herstellung von 2-(3,6-Difluorpyridin-2-yl)-1-methyl-5-(trifluormethyl)-1H-benzimidazol lässt sich ebenfalls 2-(3,6-Difluorpyridin-2-yl)-3-methyl-6-(trifluormethyl)-3H-imidazo[4,5-b]pyridin herstellen.
(logP (neutral): 2.72; MH⁺: 315; ¹H-NMR(400 MHz, D₆-DMSO) δ ppm:8.88 (m, 1H), 8.70 (m, 1H), 8.29 (m, 1H), 7.60 (m, 1H), 4.07 (s, 3H).

### Beispiel I-7

### N-[6-(2,2-Difluor-7-methyl-[1,3]dioxolo[4,5-f]benzimidazol-6-yl)-5-ethylsulfonyl-2-pyridyl]-acetamid

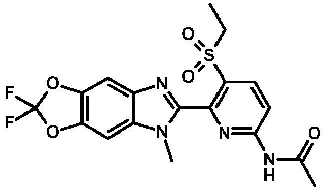

35 mg (0,08 mmol) 6-(2,2-Difluor-7-methyl-[1,3]dioxolo[4,5-f]benzimidazol-6-yl)-5-ethylsulfonyl-pyridin-2-amin wurden in einer Mischung aus 2 ml Toluol und 2 ml Pyridin gelöst, 10,8 mg 4-Dimethylaminopyridin (0,08 mmol) zugegeben und anschließend mit 21,0 mg (0,26 mmol) Acetylchlorid versetzt. Die Mischung wurde 45 min bei 100°C gerührt und anschließend das Lösungsmittel im Vakuum abdestilliert. Der Rückstand wurde durch säulenchromatographische Aufreinigung mit einem Dichlormethan / Essigester Gradienten (100:0; 50:50; 0:100) als Laufmittel gereinigt.
(logP (neutral): 2,42; MH⁺: 439; ¹H-NMR(400 MHz, D₆-DMSO) δ ppm: 1,16 (t, 3H), 2,16 (s, 1H), 3,69 (q, 2H), 3,70 (s, 3H), 7,77 (s, 1H), 7,87 (s, 1H), 8,44 (m, 2H), 11,23 (s, 1H).

### Beispiel I-8

### 6-(2,2-Difluor-7-methyl-[1,3]dioxolo[4,5-f]benzimidazol-6-yl)-5-ethylsulfonyl-pyridin-2-amin

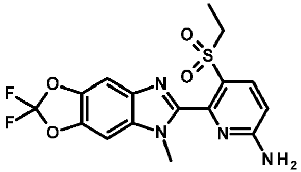

105 mg (0,25 mmol) 6-(6-Chlor-3-ethylsulfonyl-2-pyridyl)-2,2-difluor-7-methyl-[1,3]dioxolo[4,5-f]benzimidazol wurden mit 3 ml Ethanol und 3 ml 26%iger wäßriger Ammoniaklösung in der Mikrowelle für 2 h bei 120°C und 12 bar gerührt. Der Ansatz wurde unter Vakuum vom Lösungsmittel befreit und der Rückstand durch säulenchromatographische Aufreinigung über präparative HPLC mit einem Wasser / Acetonitril Gradienten als Laufmittel gereinigt.
(logP (neutral): 2,10; MH⁺: 397; ¹H-NMR(400 MHz, D₆-DMSO) δ ppm: 1,13 (t, 3H), 3,53 (q, 2H), 3,65 (s, 3H), 6,70 (d, 1H), 7,30 (br. s., 2H), 7,72, (s, 1H), 7,81 (s, 1H), 7,90 (d, 1H).

### 6-(6-Chlor-3-ethylsulfonyl-2-pyridyl)-2,2-difluor-7-methyl-[1,3]dioxolo[4,5-f]benzimidazol (Bsp. XIII-3)

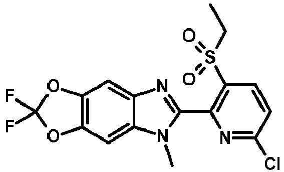

334 mg (0,84 mmol) 6-(6-Chlor-3-ethylsulfanyl-2-pyridyl)-2,2-difluor-7-methyl-[1,3]dioxolo[4,5-f]benzimidazol wurden in 20 ml Dichlormethan gelöst, bei Raumtemperatur 195,3 mg (4,24 mmol) Ameisensäure und 577,2 mg (5,93 mmol) 35%iges Wasserstoffperoxid zugegeben und anschließend 6 h bei Raumtemperatur gerührt. Der Ansatz wurde erneut mit 117,2 mg (2,54 mmol) Ameisensäure versetzt und 1h bei Raumtemperatur gerührt. Das Reaktionsgemisch wurde mit Wasser verdünnt und mit Natriumbisulfit-Lösung versetzt, 1 h gerührt, und anschließend mit gesättigter Natriumhydrogencarbonat Lösung versetzt. Die organische Phase wurde abgetrennt, die wässrige Phase zweimal mit Dichlormethan extrahiert und die vereinigten organischen Phasen anschließend unter Vakuum vom Lösungsmittel befreit.
(logP (neutral): 3,20; MH⁺: 416; ¹H-NMR(400 MHz, D₆-DMSO) δ ppm: 1,20 (t, 3H), 3,75 (s, 3H), 3,84 (q, 2H), 7,79 (s, 1H), 7,87 (s, 1H), 8,04 (d, 1H), 8,51 (d, 1H).

### 6-(6-Chlor-3-ethylsulfanyl-2-pyridyl)-2,2-difluor-7-methyl-[1,3]dioxolo[4,5-f]benzimidazol (Bsp. XI-3)

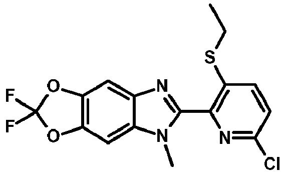

2,37 g (6,62 mmol) 6-(3,6-Dichlor-2-pyridyl)-2,2-difluor-7-methyl-[1,3]dioxolo[4,5-f]benzimidazol wurden in 35 ml Tetrahydrofuran gelöst, mit 276 mg (6,89 mmol) Natriumhydrid bei -5°C versetzt und 30 Minuten bei 0°C gerührt. Anschließend wurden 453 mg (7,28 mmol) Ethanthiol über 30 Minuten bei -5°C zugetropft, das Kältebad entfernt und 2 h bei Raumtemperatur nachgerührt. Das Reaktionsgemisch wurde unter Argon mit 20 ml Wasser hydrolisiert, die organische Phase wurde abgetrennt und die wässrige Phase zweimal mit Essigester extrahiert. Die organischen Phasen wurden vereinigt, mit Natriumchlorid Lösung gewaschen, über Natriumsulfat getrocknet und anschließend das Lösungsmittel im Vakuum abdestilliert. Der Rückstand wurde durch säulenchromatographische Aufreinigung mit einem Cyclohexan / Essigester Gradienten (20:80 bis 60:40) als Laufmittel gereinigt.
(logP (neutral): 3,80; MH⁺: 384; ¹H-NMR(400 MHz, D₆-DMSO) δ ppm: 1,20 (t, 3H), 3,00 (q, 2H), 3,86 (s, 3H), 7,66 (d, 1H), 7,78 (s, 1H), 7,84 (s, 1H), 8,05 (d, 1H).

### 6-(3,6-Dichlor-2-pyridyl)-2,2-difluor-7-methyl-[1,3]dioxolo[4,5-f]benzimidazol (Bsp. IX-3)

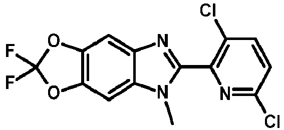

2,7 g (7,84 mmol) 6-(3,6-Dichlor-2-pyridyl)-2,2-difluor-5H-[1,3]dioxolo[4,5-f]benzimidazol, 1,24 g (8,63 mmol) Methyliodid und 2,17 g (15,6 mmol) Kaliumcarbonat wurden in Aceton gelöst und 6 h unter Rückfluß gerührt. Das Reaktionsgemisch wurde abfiltriert, die Mutterlauge vom Lösungsmittel befreit, der Rückstand in Dichlormethan aufgenommen, mit Wasser gewaschen, über Natriumsulfat getrocknet und anschließend das Lösungsmittel im Vakuum abdestilliert. Der Rückstand wurde durch säulenchromatographische Aufreinigung mit einem Cyclohexan / Essigester Gradienten (20:80 bis 60:40) als Laufmittel gereinigt.
(logP (neutral): 3,22; MH⁺: 358; ¹H-NMR(400 MHz, D₆-DMSO) δ ppm: 3,82 (s, 3H), 7,79-7,81 (m, 2H), 7,86 (s, 1H), 8,28 (d, 1H).

### 6-(3,6-Dichlor-2-pyridyl)-2,2-difluor-5H-[1,3]dioxolo[4,5-f]benzimidazol (Bsp. IX-4)

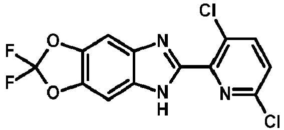

10,02 g (50,6 mmol) 2,2-Difluor-1,3-benzodioxol-5,6-diamin, 12,27 g (63,2 mmol) 3,6-Dichlorpyridin-2-carbonsäure und 9,90 g (50,6 mmol) 1-(3-Dimethylaminopropyl)-3-ethylcarbodiimid hydrochlorid (EDCI) wurden in 100 ml Pyridin unter Argon 8 h bei 120 °C gerührt. Das Reaktionsgemisch wurde unter Vakuum vom Lösungsmittel befreit, der Rückstand in Essigester aufgenommen und mit Wasser gewaschen. Die wäßrige Phase wurde zweimal mit Essigester extrahiert, die organischen Phasen vereinigt, über Natriumsulfat getrocknet und anschließend das Lösungsmittel im Vakuum abdestilliert. Der Rückstand wurde durch säulenchromatographische Aufreinigung mit einem Cyclohexan / Essigester Gradienten (20:80 bis 0:100) als Laufmittel gereinigt.
(logP (neutral): 3,20; MH⁺: 344; ¹H-NMR(400 MHz, D₆-DMSO) δ ppm: 7,54 (s, 1H), 7,67 (d, 1H), 7,82 (s, 1H), 8,20 (d, 1H), 13,29 (s, 1H).

### Beispiel I-17

### 2-[6-(4-Chlor-1H-pyrazol-1-yl)-3-(ethylsulfonyl)pyridin-2-yl]-7-(trifluormethyl)imidazo[1,2-c]pyrimidin

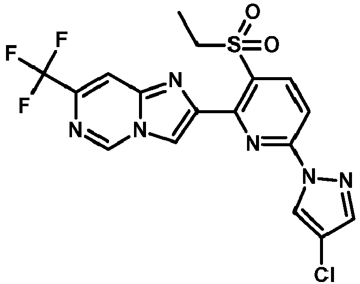

2-[3-(Ethylsulfonyl)-6-fluorpyridin-2-yl]-7-(trifluormethyl)imidazo[1,2-c]pyrimidin (49.0 mg, 0.13 mmol, 1 eq.) wurde in 6 ml Acetonitril gelöst. Es wurden Cäsiumcarbonat (51 mg, 0.15 mmol, 1.2 eq.) Kaliumiodid (10.9 mg, 0.06 mmol, 0.5 eq.) und 4-Chlor-1H-pyrazol (40.2 mg, 0.39 mmol, 3 eq.) zugegeben und für 3 h auf 75°C erhitzt. Nach Abkühlen auf Raumtemperatur wurde die Reaktionsmischung eingeengt, der Rückstand chromatographisch aufgereinigt und das gewünschte Isomer isoliert (Laufmittel: Cyclohexan, Ethylacetat).
logP (neutral): 3.46; MH⁺: 457; ¹H-NMR(400 MHz, D₆-DMSO) δ ppm: 9.63 (s, 1H), 9.06 (d, 2H), 8.63 (s, 1H), 8.15-8.13 (m, 2H), 4.21 - 4.16 (q, 2H); 1.28 (t, 3H).

### 2-[3-(ethylsulfonyl)-6-fluoropyridin-2-yl]-7-(trifluoromethyl)imidazo[1,2-c]pyrimidine

### (Bsp. XIII-4)

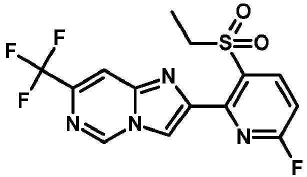

2-[3-(Ethylsulfanyl)-6-fluorpyridin-2-yl]-7-(trifluormethyl)imidazo[1,2-c]pyrimidin (65.0 mg, 0.19 mmol, 1 eq. Isomerengemisch) wurde in 3 ml Dichlormethan gelöst, dann wurden Ameisensäure (5eq., 0.94 mmol) und Wasserstoffperoxidlösung (7 eq., 1.32 mmol) zugegeben. Die Reaktionsmischung wurde für 12 h bei Raumtemperatur gerührt. Überschüssiges Oxidationsmittel wurde durch Rühren mit 40% Natriumbisulfitlösung (1 h) reduziert. Die Phasen wurden getrennt und die organische Phase wurde mit gesättigter Natriumhydrogencarbonat-Lösung gewaschen, über Natriumsulfat getrocknet und eingeengt. Der Rückstand wurde chromatographisch aufgereinigt und das gewünschte Produkt isoliert (Laufmittel: Ethylacetat/Cyclohexan).
MH⁺: 375; ¹H-NMR (400 MHz, D₆-DMSO) δ ppm: 9.66 (s, 1H); 8.71 (s, 1H), 8.42 - 8.34 (m, 2H); 7.56-7.53 (m, 1H), 4.13 - 4.07 (m, 2H), 1.27 - 1.20 (m, 3H).

### 2-[3-(ethylsulfanyl)-6-fluoropyridin-2-yl]-7-(trifluoromethyl)imidazo[1,2-c]pyrimidine (Bsp. XI-4)

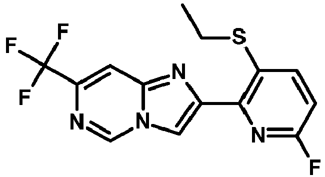

Unter einer Argonatmosphäre wurde 2-(3,6-Difluorpyridin-2-yl)-7-(trifluormethyl)imidazo[1,2-c]pyrimidin (329 mg, 1.09 mmol, 1 eq.) in 5 ml trockenem Dimethylformamid und 10 ml trockenem Tetrahydrofuran gelöst. Die Mischung wurde auf -10°C gekühlt, dann wurde Natriumhydrid (45 mg, 1.14 mmol, 1.04 eq.) zugegeben. Über einen Zeitraum von 1 h wurde das Gemisch auf Raumtemperatur erwärmt, dann wurde langsam Ethylmercaptan (75 mg, 1.2 mmol, 1.1 eq.) gelöst in 5 ml Tetrahydrofuran zugetropft. Nach einer Reaktionszeit von 2 h wurden gesättigte Natriumchlorid-Lösung und Ethylacetat zugegeben. Die Phasen wurden getrennt und die organische Phase wurde mit gesättigter Natriumchloridlösung gewaschen, über Natriumsulfat getrocknet und am Rotationsverdampfer eingeengt. Der Rückstand wurde chromatographisch aufgereinigt (Kieselgel, Laufmittel: Cyclohexan, Ethylacetat).
logP (neutral): 2.78 ; MH⁺: 343; ¹H-NMR(400 MHz, D₆-DMSO) δ ppm: 9.64 (s, 1H), 8.72 (s, 1H), 8.33 (s, 1H), 7.26-7.23 (m, 1H), 7.20-7.15 (m, 1H).

### 2-(3,6-Difluorpyridin-2-yl)-7-(trifluormethyl)imidazo[1,2-c]pyrimidin (Bsp. IX-5)

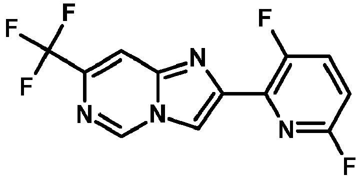

1-(3,6-Difluorpyridin-2-yl)ethanon (2.0 g, 12.7 mmol, 1 eq.) und Kupfer(II)bromid (4.2 g, 19.0 mmol 1.5 eq.) wurden in 30 ml Ethanol für 4 h auf 60°C erhitzt. Das Reaktionsgemisch wurde aufkonzentriert und der Rückstand in Ethylacetat aufgenommen. Die organische Phase wurde zweimal mit gesättigter Ammoniumchloridlösung und einmal mit gesättigter Natriumchlorid-Lösung gewaschen, über Natriumsulfat getrocknet und eingeengt. Das Rohprodukt **2**-Brom-1-(3,6-difluorpyridin-2-yl)ethanon (logP (neutral): 1.93; MH⁺: 236) wurde ohne weitere Aufreinigung weiter umgesetzt.

In einem zweiten Reaktionsgefäß wurden 6-(Trifluormethyl)pyrimidin-4-amin (201 mg, 1.23 mmol, 1 eq.) und Natriumhydrogencarbonat (155 mg, 1.85 mmol, 1.5 eq.) in 3 ml Chloroform gelöst bzw. suspendiert. 2-Brom-1-(3,6-difluorpyridin-2-yl)ethanon (502 mg, 1.23 mmol, 1 eq.) wurde gelöst in 1 ml Chloroform zugetropft, dann wurde das Reaktionsgemisch für 4 h auf 60°C erhitzt. Natriumhydrogencarbonat wurde abfiltriert und die organische Phase aufkonzentriert. Der Rückstand wurde chromatographisch aufgereinigt (Kieselgel, Laufmittel Cyclohexan/Ethylacetat).
logP (neutral): 2.02; ; MH⁺: 301; ¹H-NMR(400 MHz, D₆-DMSO) δ ppm: 9.64 (s, 1H), 8.77 (d, 1H), 8.35 (s, 1H), 8.17 - 8.10 (m, 1H), 7.36 - 7.32 (m, 1H).

### 2-Brom-1-(3,6-difluorpyridin-2-yl)ethanon

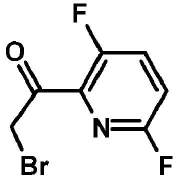

1-(3,6-Difluorpyridin-2-yl)ethanon (6.4 g, 40.7 mmol) wurde in 96 ml Ethanol gelöst und es wurde Kupfer(II)bromid (13.7 g, 61.1 mmol) zugegeben. Das Reaktionsgemisch wurde für 4 h auf 60 °C erhitzt. Nach abkühlen auf Raumtemperatur wurde wurde das Lösemittel am Rotationsverdampfer entfernt und der Rückstand wurde mit gesättigter Ammoniumchloridlösung und mit Ethylacetat aufgenommen. Die wässrige Phase wurde zwei Mal mit Ethylacetat extrahiert. Die vereinigten orgabnischen Extrakte wurden mit gesättigter Natriumchloridlösung gewaschen, über Natriumsulfat getrocknet und eingeengt. Das Produkt wurde als Gemisch isoliert und ohne weitere Aufreinigung in der Folgereaktion eingesetzt.
logP (neutral): 1.95; MH⁺:2.38; ¹H-NMR(400 MHz,CDCl3) δ ppm: 8.17 - 8.10 (m, 1H), 7.62 - 7.57 (m, 1H), 2.56 (s, 2H)

### 1-(3,6-Difluorpyridin-2-yl)ethanon

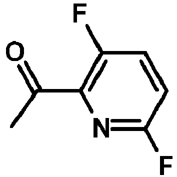

Unter einer Argonatmosphäre wurde 3,6-Difluor-N-methoxy-N-methylpyridin-2-carboxamid (10.1 g, 50.2 mmol) in 270 ml trockenem THF gelöst und auf 0°C gekühlt. Langsam wurde Methylmagnesiumbromid (3M in Diethylether, 50 ml, 150 mmol) zugetropft, dann wurde für 2 h bei 0°C gerührt. Um die Reaktion zu beenden wurde unter Kühlung langsam 50 ml Ammoinumchloridlösung zugegegeben. Die organische Phase wurde abgetrennt und mit gesättigter Natriumchloridlösung gewaschen. Die organische Phase wurde abgetrennt und mit gesättigter Natriumchloridlösung gewaschen. Das Lösemittel wurde am Rotationsverdampfer entfernt und das Rohprodukt ohne weitere Aufreinigung in der Folgereaktion eingesetzt.
logP (neutral): 1.24; MH⁺: 158; ¹H-NMR(400 MHz,CDCl3) δ ppm: 8.16 - 8.10 (m, 1H), 7.61 - 7.57 (m, 1H), 2.56 - 2.55 (m, 3H).

### 3,6-Difluor-N-methoxy-N-methylpyridin-2-carboxamid

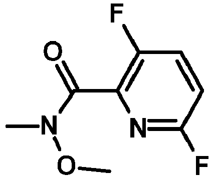

3,6-Difluorpyridin-2-carbonsäure (15 g, 94 mmol) und N,O-Dimethylhydroxylamin Hydrochlorid (9.1 g, 94.2 mmol) wurden in 600 ml Dichlormethan gelöst und auf 0°C gekühlt. Es wurden 4-Dimethylaminopyridin (DMAP, 13.8 g, 113 mmol) und N(-Dimethylaminopropyl)-N'-ethylcarbodiimid Hydrochlorid (EDCI, 19.9 g, 103 mmol) zugegeben und die Reaktionsmischung wurde für 2 h bei 0°C, anschließend 12 h bei Raumtemperatur gerührt. Die Lösung wurde einmal mit gesättigter Natriumhydrogencarbonatlösung und einmal mit Natriumchloridlösung gewaschen, über Natriumsulfat getrocknet und eingeengt. Das Rohprodukt wurde ohne weitere Aufreinigung in der Folgereaktion eingesetzt.
logP (neutral): 1.05; MH⁺: 203; ¹H-NMR(400 MHz,CDCl3) δ ppm: 8.15 - 8.09 (m, 1H), 7.44 - 7.42 (m, 1H), 3.57 (s, 3H), 3.31 (s, 3H).

### Beispiel I-38

### 5-(Ethylsulfonyl)-N-methyl-6-[7-(trifluormethyl)imidazo[1,2-c]pyrimidin-2-yl]pyridin-2-carboxamid

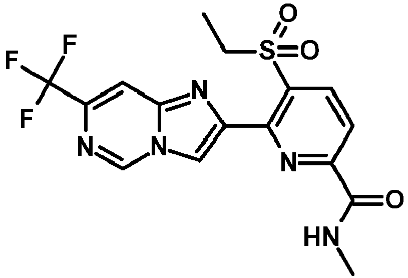

2-[3,6-Bis(ethylsulfonyl)pyridin-2-yl]-7-(trifluormethyl)imidazo[1,2-c]pyrimidin (135 mg, 0.12 mmol, Reinheit ca. 40%), Natriumacetat (18 mg, 0.22 mmol) und [1,1'-Bis(diphenylphosphino)ferrocene]dichloropalladium(II)-Aceton-Komplex (PD-107; 10 mg, 0.01 mmol) wurden in 10 ml Methanol gelöst. Die Reaktionslösung wurde bei 10 bar mit Kohlenmonoxid gespült, dann 36 h bei 10 bar Kohlenmonoxidatmosphäre auf 80°C erhitzt. Es wurden weiteres Natriumacetat (18 mg, 0.22 mmol) und [1,1'-Bis(diphenylphosphino)ferrocene]dichloropalladium(II)-Aceton-Komplex (PD-107; 10 mg, 0.01 mmol) zugegeben und weitere 21 h unter gleichen Bedingungen erhitzt.

Die Reaktionslösung wurde eingeengt und chromatographisch aufgereinigt (Laufmittel: Cyclohexan, Essigester).

Methyl-5-(ethylsulfonyl)-6-[7-(trifluormethyl)imidazo[1,2-c]pyrimidin-2-yl]pyridin-2-carboxylat (125 mg, 0.12 mmol, Reinheit ca. 40%) wurde bei Raumtemperatur in Methanol gelöst und es wurden Methylamin (33% in Ethanol, 113 mg, 1.2 mmol) und 0.1 ml Wasser zugegeben. Die Reaktionslösung wurde 20 min bei Raumtemperatur gerührt und dann eingeengt. Die Aufreinigung erfolgte per HPLC (Acetonitril, Wasser, Ameisensäure).
logP (sauer): 1.95; MH⁺: 414; ¹H-NMR(400 MHz, D₆-DMSO) δ ppm: 9.68 (s, 1H), 9.04 (s, 1H), 8.98 - 8.95 (m, 1H), 8.65-8.63 (d, 1H), 8.40 (s, 1H), 8.27 - 8.26 (d, 1H), 4.21 - 4.17 (q, 2H), 2.91 - 2.90 (d, 3H), 1.28 - 1.26 (t, 3H).

### 2-[3,6-Bis(ethylsulfonyl)pyridin-2-yl]-7-(trifluormethyl)imidazo[1,2-c]pyrimidin (LXXIII-1)

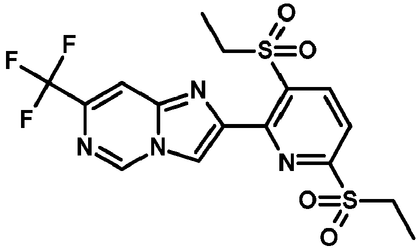

2-(3,6-Difluorpyridin-2-yl)-7-(trifluormethyl)imidazo[1,2-c]pyrimidin (100 mg, 0.33 mmol, 1 eq.) wurde unter Argonatmosphäre in 2 ml trockenem DMF gelöst. Es wurde Natriumethanthiolat (168 mg, 1.99 mmol, 6 eq.) zugegeben und für 12 h bei Raumtemperartur gerührt. Das Gemisch wurde mit Essigester verdünnt und mit gesättigter Natriumchloridlösung gewaschen. Die organische Phase wurde über Natriumsulfat getrocknet und das Lösemittel wurde am Rotationsverdampfer entfernt. Das Rohprodukt wurde ohne weitere Aufreinigung in der Folgereaktion eingesetzt.

2-[3,6-Bis(ethylsulfanyl)pyridin-2-yl]-7-(trifluormethyl)imidazo[1,2-c]pyrimidin (75 mg, 0.19 mmol, 1 eq.) wurde in 9 ml Dichlormethan gelöst. Bei Raumtemperatur wurden Ameisensäure (90 mg, 1.95 mmol, 10 eq.) und Wasserstoffperoxidlösung (35%, 265 mg, 12.7 mmol, 14 eq.) zugegeben und das Gemisch wurde für 12 h gerührt. Nach Zugabe von 1 ml Wasser wurden vorsichtig 3 ml 40% Natriumbisulfitlösung zugetropft und 1 h gerührt. Anschließend wurde die organische Phase abgetrennt, mit gesättigter Natriumhydrogencarbonatlösung gewaschen, über Natriumsulfat getrocknet und eingeengt. Der Rückstand wurde chromatographisch aufgereinigt (Laufmittel: Cyclohexan, Essigester).
logP (neutral): 2.28; MH⁺:449; ¹H-NMR (400 MHz, D₆-DMSO) δ ppm: 9.67 (s, 1H), 8.86 (s, 1H), 8.82 - 8.80 (m, 1H), 8.41 (s, 1H), 8.36 - 8.34 (m, 1H), 4.23 - 4.18 (m, 2H), 3.64 - 3.59 (m, 2H), 1.30 - 1.23 (m, 6H).

### Beispiel I-18

### 2-[6-(4-Chlor-1H-pyrazol-1-yl)-3-(ethylsulfonyl)pyridin-2-yl]-6-(trifluormethyl)imidazo[1,2-a]pyrazin

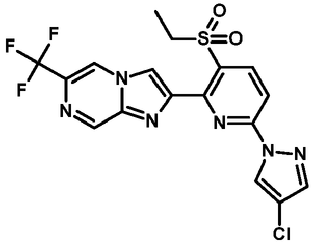

2-[3,6-Bis(ethylsulfonyl)pyridin-2-yl]-6-(trifluormethyl)imidazo[1,2-a]pyrazin (88 mg, 0.19 mmol, 1 eq.) und 4-Chlor-1H-pyrazol (20 mg, 0.19 mmol, 1eq.) wurden in 2 ml Acetonitril gelöst. Es wurden Cäsiumcarbonat (77 mg, 0.23 mmol, 1.2 eq) und Kaliumiodid (16 mg, 0.09 mmol, 0.5 eq.) zugegeben und für 12 h bei Raumtemperatur gerührt. Nach Zugabe von etwas Kieselgel wurde das Reaktionsgemisch zur Trockne eingeengt und anschließend säulenchromatographisch aufgereinigt (Laufmittel: Cyclohexan, Ethylacetat).
logP (neutral): 3.44; MH⁺: 457; ¹H-NMR(400 MHz, D₆-DMSO) δ ppm: 9.34 (ψ-s, 2H), 9.00 (s, 2H), 8.66 - 8,64 (m, 1H), 8.17 - 8.14 (m, 2H), 4.18 - 4.13 (q, 2H), 1.30 - 1.28 (t, 3H).

### 2-[3,6-Bis(ethylsulfonyl)pyridin-2-yl]-6-(trifluormethyl)imidazo[1,2-a]pyrazin (Bsp. LXXIII-2)

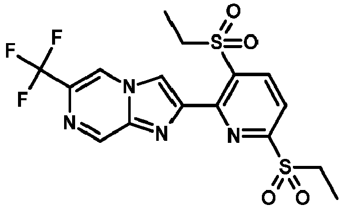

2-[3,6-Bis(ethylsulfanyl)pyridin-2-yl]-6-(trifluormethyl)imidazo[1,2-a]pyrazin (101 mg, 0.26 mmol, 1 eq.) wurde in 10 ml Dichlormethan gelöst. Bei Raumtemperatur wurden Ameisensäure (120 mg, 2.62 mmol, 10 eq.) und Wasserstoffperoxidlösung (35%, 3.67 mmol, 14 eq.) zugegeben. Das Reaktionsgemisch wurde für 12 h bei Raumtemperatur gerührt. Dann wurden 1 ml Wasser und sehr langsam 40% Natriumbisulfitlösung zugesetzt. Das Gemisch wurde 1 h bei Raumtemperatur gerührt, dann wurden die Phasen getrennt. Die organische Phase wurde mit gesättigter Natriumhydrogencarbonat-Lösung gewaschen, über Natriumsulfat getrocknet und eingeengt. Der Rückstand wurde säulenchromatographisch gereinigt (Kieselgel, Laufmittel Cyclohexan/Ethylacetat).
logP (neutral): 2.23; MH⁺: 449; ¹H-NMR(400 MHz, D₆-DMSO) δ ppm: 9.40-9.36 (d, 2H), 8.85 - 8.82 (m, 2H), 8.37 - 8.35 (d, 2H), 4.21-4.16 (m, 2H), 3.63 - 3.58 (m, 2H), 1.31 - 1.23 (m, 6H).

### 2-[3,6-Bis(ethylsulfanyl)pyridin-2-yl]-6-(trifluormethyl)imidazo[1,2-a]pyrazin (Bsp. LXXI-1)

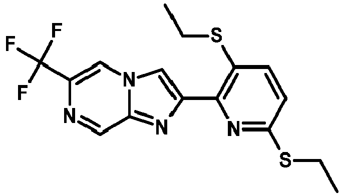

2-(3,6-Difluorpyridin-2-yl)-6-(trifluormethyl)imidazo[1,2-a]pyrazin (290 mg, 0.96 mmol, 1 eq.) wurde unter einer Argonatmosphäre in 10 ml trockenem Tetrahydrofuran und 10 ml trockenem Dimethylformamid suspendiert. Die Suspension wurde auf - 10°C gekühlt und es wurde Natriumhydrid (60%, 95 mg, 2.41 mmol, 2.5 eq.) zugegeben. Bei Raumtemperatur wurde Ethylmercaptan (150 mg, 2.41 mmol, 2.5 eq. gelöst in 2 ml Dimethylformamid) langsam zugetropft. Das Reaktionsgemisch wurde für 24 h bei Raumtemperatur gerührt, dann wurde gesättigte Natriumchlorid-Läsung zugegeben. Die Mischung wurde mit Ethylacetat verdünnt, dann wurden die Phasen getrennt. Die organische Phase wurde mit gesättigter Natriumchlorid-Lösung gewaschen, über Natriumsulfat getrocknet und eingeengt. Der Rückstand wurde säulenchromatographisch aufgereinigt (Laufmittel: Cyclohexan, Ethylacetat).
logP (neutral): 4.00; MH⁺: 385; ¹H-NMR(400 MHz, D₆-DMSO) δ ppm: 9.40 (s, 1H), 9.28 (s, 1H), 8.76 (s, 1H), 7.77-7.75 (d, 1H), 7.31-7.29 (d, 1H), 3.20 - 3.18 (q, 2H), 3.02 - 2.97 (q, 2H), 1.40 - 1.34 (t, 3H), 1.27 - 1.23 (t, 3H).

### 2-(3,6-Difluorpyridin-2-yl)-6-(trifluormethyl)imidazo[1,2-a]pyrazin (Bsp. IX-6)

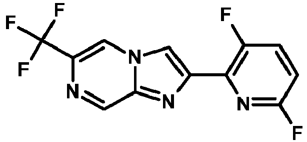

5-(Trifluormethyl)pyrazin-2-amin (500 mg, 3.06 mmol, 1 eq.) und 2-Brom-1-(3,6-difluorpyridin-2-yl)ethanon (1.02 g, 3.67 mmol, 2 eq.) wurden in 15 ml Ethanol gelöst und für 5 d auf 60 °C erhitzt. Das Reaktionsgemisch wurde eingeengt und aus Dichlormethan /Methyltertbutylether umkristallisiert.
logP (sauer): 2.05; MH⁺: 301; ¹H-NMR(400 MHz, D₆-DMSO) δ ppm:9.39 (s, 1H), 9.31 (s, 1H), 8.80-8.79 (m, 1H), 8.18-8.11 (m, 1H), 7.37 - 7.33 (m, 1H).

### Beispiel I-19

### N-{5-(Ethylsulfonyl)-6-[6-(trifluormethyl)[1,3]oxazolo[5,4-b]pyridin-2-yl]pyridin-2-yl}acetamid

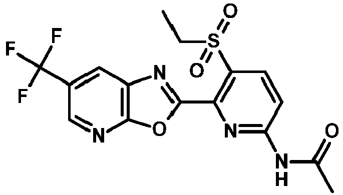

Gemäß der obigen Vorschrift zur Herstellung von N-{5-(Ethylsulfonyl)-6-[3-methyl-6-(trifluormethyl)-3H-imidazo[4,5-b]pyridin-2-yl]pyridin-2-yl}acetamid lässt sich über 5-(Ethylsulfonyl)-6-[6-(trifluormethyl)[1,3]oxazolo[5,4-b]pyridin-2-yl]pyridin-2-amin als Zwischenstufe (roh eingesetzt) ebenfalls N-{5-(Ethylsulfonyl)-6-[6-(trifluormethyl)[1,3]oxazolo[5,4-b]pyridin-2-yl]pyridin-2-yl}acetamid herstellen.
logP (neutral): 2.44; ; MH⁺: 415; ¹H-NMR(400 MHz, D₆-DMSO) δ ppm: 11.45 (s, 1H), 9.03-9.01 (m, 2H), 8.56 - 8.52 (m, 2H), 3.79 - 3.75 (m, 2H), 2.19 (s, 3H), 1.27 - 0.91 (m, 3H).

### 2-[6-Brom-3-(ethylsulfanyl)pyridin-2-yl]-6-(trifluormethyl)[1,3]oxazolo[5,4-b]pyridine (Bsp. XI-5)

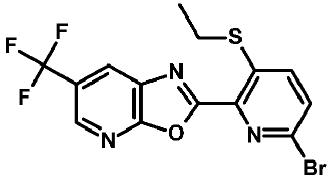

6-Brom-N-[2-chlor-5-(trifluormethyl)pyridin-3-yl]-3-(ethylsulfanyl)pyridin-2-carboxamid (1.2 g, 2.72 mmol) und Natriumcarbonat (288 mg, 2.72 mmol) wurden in 20 ml Dimethylformamid für 12 h bei 145°C gerührt. Durch Zugabe von 40 ml Eiswasser wurde die Reaktion beendet, das Produkt ausgefällt und durch Filtration als grauer Feststoff isoliert.
logP (neutral):4.16; ; MH⁺: 404; ¹H-NMR(400 MHz, D₆-DMSO) δ ppm: 8.97-8.95 (m, 2H), 8.00 - 7.86 (m, 2H), 3.17-3.11 (m, 2H), 1.35 -1.31 (m, 3H).

### 6-Brom-N-[2-chlor-5-(trifluormethyl)pyridin-3-yl]-3-(ethylsulfanyl)pyridin-2-carboxamid (Bsp. LXIX-1)

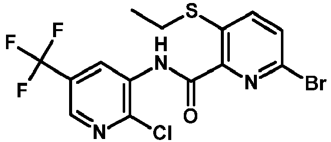

6-Brom-3-(ethylsulfanyl)pyridin-2-carbonsäure (3 g, 11.5 mmol) wurde in 60 ml Dichlormethan gelöst und auf 0°C gekühlt. Unter Rühren wurden Oxalylchlorid (14.5 g, 115 mmol) und ein Tropfen Dimethylformamid zugegeben, dann wurde bei Raumtemperatur für 30 min gerührt. Nach Entfernen des Lösemittels wurde das Rohprodukt 6-Brom-3-(ethylsulfanyl)pyridin-2-carbonylchlorid direkt weiter umgesetzt.

2-Chlor-5-(trifluoromethyl)pyridin-3-amin (1.05 g, 5.38 mmol) wurde bei 0 °C zu einer Suspension von Natriumhydrid (60% in Mineralöl, 258 mg, 6.46 mmol) in Tetrahydrofuran addiert. Das Gemisch wurde für 30 min gerührt, dann wurde portionsweise 6-Brom-3-(ethylsulfanyl)pyridin-2-carbonylchlorid (3 g, 10.75 mmol) zugegeben. Nach Rühren für 12 h wurde die Reaktion durch Zugabe von 10 ml Eiswasser beendet. Das Produkt wurde in Form eines Niederschlages durch Filtration isoliert.
logP (neutral): 5.27; MH⁺: 442; ¹H-NMR(400 MHz, D₆-DMSO) δ ppm: 10.57 (s, 1H); 8.91 (s, 1H), 8.70 (s, 1H), 7.95 - 7.87 (m, 2H), 3.05 - 3.00 (m, 2H), 1.30 - 1.27 (m, 3H).

### 6-Brom-3-(ethylsulfanyl)pyridin-2-carbonsäure

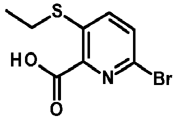

6-Brom-3-fluor-2-methylpyridine (5 g, 26.3 mmol) wurde in 100 ml Wasser gelöst und es wurde Kaliumpermanganat (12.5 g, 78.9 mmol) zugegeben. Das Reaktionsgemisch wurde für 12 h zum Rückfluß erhitzt. Feste Bestandteile wurden durch Filtration entfernt. Der Filterkuchen wurde dreimal mit 100 ml Wasser gewaschen. Die gesammelten Waschlösungen wurden aufkonzentriert und das Rohprodukt wurde als weisser Feststoff erhalten, der direkt weiter umgesetzt wurde.

Das Rohprodukt (6 g) wurde in 20 ml Dimethylformamid gelöst und nach Zugabe von Natriumethanthiolat (6.6 g, 78.9 mmol) wurde für 12 h bei Raumtemperatur gerührt. Das Reaktionsgemisch wurde filtriert und die organische Phase wurde eingeengt. Der Rückstand wurde chromatographisch aufgereinigt (Laufmittel: Wasser/Acetonitril) und das Produkt wurde in Form eines gelben Feststoffes erhalten.
logP (neutral): 1.79; MH⁺: 262 ; ¹H-NMR(400 MHz, D₆-DMSO) δ ppm: 7.85 - 7.39 (m, 2H), 3.34 - 2.92 (m, 2H), 1.39 -1.15 (m, 3H).

### Beispiel I-34

### 5-(Ethylsulfonyl)-N-methyl-6-[3-methyl-6-(trifluormethyl)-3H-imidazo[4,5-b]pyridin-2-yl]pyridin-2-carboxamid

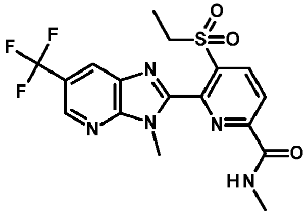

Methyl-5-(ethylsulfonyl)-6-[3-methyl-6-(trifluormethyl)-3H-imidazo[4,5-b]pyridin-2-yl]pyridin-2-carboxylat (50 mg, 0.11 mmol, 1 eq.) wurde in Methanol bei Raumtemperatur gelöst. Methylamin (33% in Ethanol, 109 mg, 1.16 mmol, 10 eq.) und 0.1 ml Wasser wurden zugegeben und das Gemisch wurde für 20 min bei Raumtemperatur gerührt. Die Reaktionslösung wurde eingeengt und säulenchromaographisch aufgereinigt (Laufmittel: Cyclohexan, Ethylacetat).
logP (neutral): 2.36; MH⁺: 428; ¹H-NMR(400 MHz, D₆-DMSO) δ ppm: 9.01 - 8.98 (m, 1H), 8.93 - 8.92 (m, 1H), 8.73 - 8.71 (m, 2H), 8.50 - 8.48 (m, 1H), 3.89 - 3.84 (m, 5H), 2.84 - 2.83 (d, 3H), 1.24 - 1.20 (t, 3H).

### Methyl-5-(ethylsulfonyl)-6-[3-methyl-6-(trifluormethyl)-3H-imidazo[4,5-b]pyridin-2-yl]pyridin-2-carboxylat (Beispiel LXXX-1)

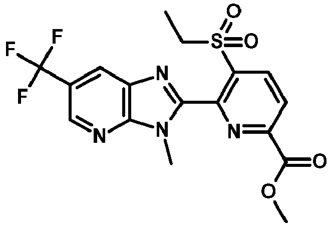

Methyl-5-(ethylsulfanyl)-6-[3-methyl-6-(trifluormethyl)-3H-imidazo[4,5-b]pyridin-2-yl]pyridin-2-carboxylat (211 mg, 0.53 mmol, 1 eq.) wurde in 145 ml Dichlormethan gelöst. Bei Raumtemperatur wurden Ameisensäure (245 mg, 5.32 mmol, 10 eq.) und Wasserstoffperoxid (35% in Wasser, 724 mg, 7.45 mmol, 14 eq.) zugegeben und das Reaktionsgemisch wurde für 12 h gerührt. Unter Eiskühlung wurden langsam 2 ml Wasser und 2 ml 40% Natriumbisulfit-Lösung zugegeben und für 1 h gerührt. Die organische Phase wurde abgetrennt, über Natriumsulfat getrocknet und eingeengt. Das Rohprodukt wurde säulenchromaographisch aufgereinigt (Laufmittel: Cyclohexan, Ethylacetat).
logP (neutral): 2.61; MH⁺: 429; ¹H-NMR(400 MHz, D₆-DMSO) δ ppm: 8.93 - 8.92 (m, 1H), 8.76 - 8.74 (m, 1H), 8.72 - 8,71 (m, 1H), 8.54 - 8.52 (m, 1H), 3.96 (s, 3H), 3.89 - 3.82 (q, 2H), 3.78 (s, 3H), 1.23 - 1.20 (t, 3H).

### Methyl-5-(ethylsulfanyl)-6-[3-methyl-6-(trifluormethyl)-3H-imidazo[4,5-b]pyridin-2-yl]pyridin-2-carboxylat (Beispiel LXXIX-1)

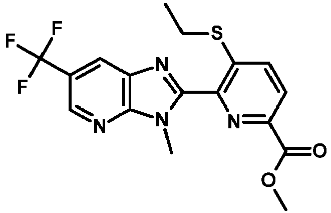

Methyl-5-chlor-6-[3-methyl-6-(trifluormethyl)-3H-imidazo[4,5-b]pyridin-2-yl]pyridin-2-carboxylat (200 mg, 0.53 mmol, 1 eq.) wurde in 5 ml Tetrahydrofuran gelöst und es wurden Natriumhydrid (16 mg, 0.40 mmol, 0.75 eq.) sowie Natriumthioethylat (47 mg, 0.56 mmol, 1.05 eq) zugegeben. Die Reaktionsmischung wurde für 1 h bei Raumtemperatur gerührt, dann wurde das Gemisch in 1N Salzsäurelösung gegossen. Nach Zugabe von Ethylacetat wurden die Phasen getrennt. Die wässrige Phase wurde mit Ethylacetat extrahiert. Die vereinigen organischen Phasen wurden mit gesättigter Natriumchloridlösung gewaschen, über Natriumsulfat getrocknet und eingeengt. Das Rohprodukt wurde ohne weitere Aufreinigung weiter umgesetzt.
logP (neutral): 3.25; MH⁺: 397; ¹H-NMR(400 MHz, D₆-DMSO) δ ppm: 8.89 - 8.88 (m, 1H), 8.70 - 8.67 (m, 1H), 8.20 - 8.15 (m, 2H), 4.03 (s, 3H), 3.92 (s, 3H), 3.13 - 3.08 (m, 2H), 1.29 - 1.26 (t, 3H).

### Methyl-5-chlor-6-[3-methyl-6-(trifluormethyl)-3H-imidazo[4,5-b]pyridin-2-yl]pyridin-2-carboxylat (Beispiel LXXVIII-1)

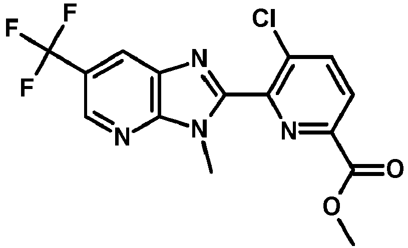

N2-Methyl-5-(trifluormethyl)pyridin-2,3-diamin (1.5 g, 7.84 mmol, 1 eq.), 3-Chlor-6-(methoxycarbonyl)pyridin-2-carbonsäure (2.2 g, 10.2 mmol, 1.3 eq.) und 1-Ethyl-3-(3-dimethylaminopropyl)carbodiimid (1.5 g, 7.84 mmol, 1 eq.) wurden in 25 ml Pyridin gelöst und bei Raumtemperatur für 12 h gerührt. Die Reaktionsmischung wurde eingeengt und in 25 ml Toluol aufgenommen. Nach Zugabe von 4-Toluolsulfonsäure (2.0 g, 11.7 mmol, 1.5 eq.) wurde für 8 h zum Rückfluss erhitzt. Die Reaktionsmischung wurde auf Raumtemperatur gekühlt, mit Ethylacetat verdünnt und nacheinander mit Ammoniumchlorid-Lösung und Natriumchlorid-Lösung gewaschen. Das Rohprodukt wurde säulenchromaographisch aufgereinigt (Laufmittel: Cyclohexan, Ethylacetat).
logP (neutral): 2.7; MH⁺: 371; ¹H-NMR(400 MHz, D₆-DMSO) δ ppm: 8.90 (s, 1H), 8.71 (s, 1H), 8.47 - 8.45 (m, 1H), 8.29 - 8.27 (m, 1H), 3.93 (m, 6H).

### Beispiel I-35

### 6-[6-(4-Chlorpyrazol-1-yl)-3-ethylsulfonyl-2-pyridyl]-7-methyl-3-(trifluormethyl)imidazo[4,5-c]pyridazin

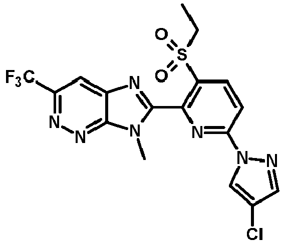

47 mg (0,11 mmol) 6-(6-Chlor-3-ethylsulfonyl-2-pyridyl)-7-methyl-3-(trifluormethyl)imidazo[4,5-c]pyridazin werden in 5 ml Acetonitril gelöst, 56,6 mg Cäsiumcarbonat (0,17 mmol), 10 mg (0,05 mmol) Kaliumiodid und 13,1 mg (0,12 mmol) 4-Chlor-1H-pyrazol zugegeben und anschließend 24 h bei Raumtemperatur gerührt. Anschließend wird das Reaktionsgemisch über eine Kieselgelkartusche mit Essigester filtriert. Das Lösungsmittel wird im Vakuum abdestilliert und der Rückstand durch säulenchromatographische Aufreinigung mit einem Wasser / Acetonitril Gradienten als Laufmittel gereinigt.
(logP (neutral): 3,38; MH⁺: 472; ¹H-NMR(400 MHz, D₆-DMSO) δ ppm: 1,25 (t, 3H), 3,81 (q, 2H), 4,09 (s, 3H), 8,18 (s, 1H), 8,39 (d, 1H), 8,72 (d, 1H), 8,83 (s, 1H), 9,01 (s, 1H).

### 6-(6-Chlor-3-ethylsulfonyl-2-pyridyl)-7-methyl-3-(trifluormethyl)imidazo[4,5-c]pyridazin (Beispiel XIII-5)

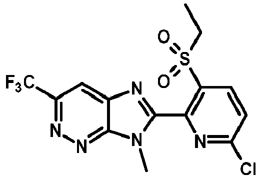

54 mg (0,14 mmol) 6-(6-Chlor-3-ethylsulfanyl-2-pyridyl)-7-methyl-3-(trifluormethyl)imidazo[4,5-c]pyridazin werden in 5 ml Dichlormethan gelöst, bei Raumtemperatur 80 mg (1,73 mmol) Ameisensäure und 140 mg (1,44 mmol) 35%iges Wasserstoffperoxid zugegeben und anschließend 6 h bei Raumtemperatur gerührt. Das Reaktionsgemisch wird mit Wasser verdünnt und mit Natriumbisulfit-Lösung versetzt, 1 h gerührt, und anschließend mit gesättigter Natriumhydrogencarbonat Lösung versetzt. Die organische Phase wird abgetrennt, die wässrige Phase zweimal mit Dichlormethan extrahiert und die vereinigten organischen Phasen anschließend unter Vakuum vom Lösungsmittel befreit.
(logP (neutral): 2,60; MH⁺: 406; ¹H-NMR(400 MHz, D₆-DMSO) δ ppm: 1,21 (t, 3H), 3,73 (q, 2H), 4,00 (s, 3H), 8,18 (d, 1H), 8,60 (d, 1H), 8,80 (s, 1H).

### 6-(6-Chlor-3-ethylsulfanyl-2-pyridyl)-7-methyl-3-(trifluormethyl)imidazo[4,5-c]pyridazin (Beispiel XI-6)

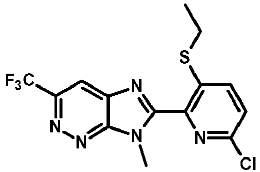

88 mg (0,19 mmol) 6-(3,6-Dichlor-2-pyridyl)-7-methyl-3-(trifluormethyl)imidazo[4,5-c]pyridazin werden in 3 ml Tetrahydrofuran gelöst, mit 8 mg (0,19 mmol) Natriumhydrid bei -20°C versetzt und 15 Minuten bei -20°C gerührt. Anschließend werden 13 mg (0,20 mmol) Ethanthiol bei -20°C zugetropft, das Kältebad entfernt und 1 h bei 0°C und 1 h bei Raumtemperatur nachgerührt. Das Reaktionsgemisch wird auf Eiswasser gegeben und über Nacht nachgerührt. Die wässrige Phase wird dreimal mit Essigester extrahiert. Die organischen Phasen werden vereinigt, über Natriumsulfat getrocknet und anschließend das Lösungsmittel im Vakuum abdestilliert.
(logP (neutral): 3,28; MH⁺: 374; ¹H-NMR(400 MHz, D₆-DMSO) δ ppm: 1,23 (t, 3H), 3,07 (q, 2H), 4,15 (s, 3H), 7,79 (d, 1H), 8,17 (d, 1H), 8,76 (s, 1H).

### 6-(3,6-Dichlor-2-pyridyl)-7-methyl-3-(trifluormethyl)imidazo[4,5-c]pyridazin (Beispiel IX-7)

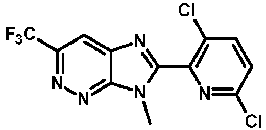

255 mg (1,33 mmol) 3,6-Dichlorpyridin-2-carboxamid, 341,8 mg (1,33 mmol) 4-Brom-N-methyl-6-(trifluormethyl)pyridazin-3-amin, 175 mg Kaliumtertiärbutylat und 87 mg [1,1'-Bis(diphenylphosphino)-ferrocen]palladium(ll)chlorid werden im 10 ml Toluol vorgelegt und 11 h unter Rückfluß gerührt. Anschließend wird das Reaktionsgemisch mit Wasser verdünnt und zweimal mit Essigester extrahiert. Die vereinigten organischen Phasen werden über Natriumsulfat getrocknet und anschließend das Lösungsmittel im Vakuum abdestilliert. Der Rückstand wird durch säulenchromatographische Aufreinigung mit einem Wasser / Acetonitril Gradienten als Laufmittel gereinigt.
(logP (neutral): 2,65; MH⁺: 348; ¹H-NMR(400 MHz, D₆-DMSO) δ ppm: 4,10 (s, 3H), 7,93 (d, 1H), 8,39 (d, 1H), 8,79 (s, 1H).

### Beispiel I-39

### 5-(Ethylsulfonyl)-N-methyl-6-[7-methyl-3-(trifluormethyl)-7H-imidazo[4,5-c]pyridazin-6-yl]pyridin-2-carboxamid

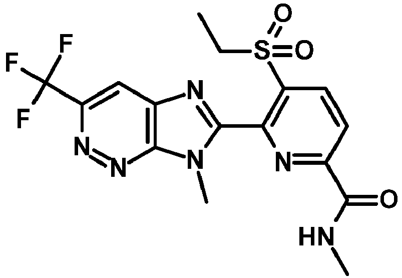

Methyl-5-(ethylsulfonyl)-6-[7-methyl-3-(trifluormethyl)-7H-imidazo[4,5-c]pyridazin-6-yl]pyridin-2-carboxylat (55 mg, 0.12 mmol) wurde in Methanol gelöst und es wurden Methylamin (33% in Ethanol, 120 mg, 1,28 mmol) sowie 0.1 ml Wasser zugegeben. Nach Rühren für 20 min bei Raumtemperatur wurde die Reaktionslösung eingeengt und via HPLC aufgereinigt (Acetonitril, Wasser, Ameisensäure).
logP (neutral): 1.96; MH⁺: 429; ¹H-NMR (400 MHz, D₆-DMSO) δ ppm: 9.02 - 8.99 (m, 1H), 8.83 (s, 1H), 8.77 - 8.75 (m, 1H), 8.54 - 8.52 (m, 1H), 4.02 (s, 1H), 3.82 - 3.77 (m, 2H), 2.85 - 2.84 (m, 3H), 1.24 - 1.20 (m, 3H).

### Methyl-5-(ethylsulfonyl)-6-[7-methyl-3-(trifluormethyl)-7H-imidazo[4,5-c]pyridazin-6-yl]pyridin-2-carboxylat (LXXX-2)

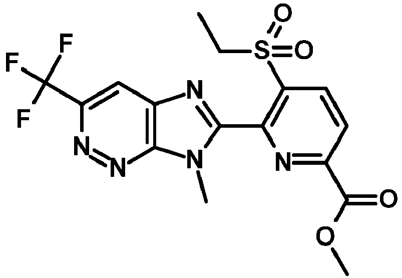

Methyl-5-chlor-6-[7-methyl-3-(trifluormethyl)-7H-imidazo[4,5-c]pyridazin-6-yl]pyridin-2-carboxylat (120 mg, 0.27 mmol, 1 eq.) wurde unter Argonatmosphäre in 3 ml trockenem DMF gelöst. Es wurde Natriumethanthiolat (70 mg, 0.83 mmol, 3 eq.) zugegeben und für 2 h bei Raumtemperartur gerührt. Das Gemisch wurde in 1N HCl gegossen und mit Essigester verdünnt. Anschließend wurde mit gesättigter Natriumchloridlösung gewaschen. Die organische Phase wurde über Natriumsulfat getrocknet und das Lösemittel wurde am Rotationsverdampfer entfernt. Das Rohprodukt wurde ohne weitere Aufreinigung in der Folgereaktion eingesetzt.

Methyl-5-(ethylsulfanyl)-6-[7-methyl-3-(trifluormethyl)-7H-imidazo[4,5-c]pyridazin-6-yl]pyridin-2-carboxylat (105 mg, 0.26 mmol, 1 eq.) wurde in 8 ml Dichlormethan gelöst. Bei Raumtemperatur wurden Ameisensäure (121 mg, 2.64 mmol, 10 eq.) und Wasserstoffperoxidlösung (35%, 359 mg, 3.69 mmol, 14 eq.) zugegeben und das Gemisch wurde für 12 h gerührt. Nach Zugabe von 2 ml Wasser wurden vorsichtig 2 ml 40% Natriumbisulfitlösung zugetropft und 1 h gerührt. Anschließend wurde die organische Phase abgetrennt, mit gesättigter Natriumhydrogencarbonatlösung gewaschen, über Natriumsulfat getrocknet und eingeengt. Der Rückstand wurde chromatographisch aufgereinigt (Laufmittel: Cyclohexan, Essigester).
logP (neutral): 2.19; MH⁺: 430; ¹H-NMR (400 MHz, D₆-DMSO) δ ppm: 8.80 - 8.78 (m, 2H), 8.59 - 8.57 (m, 1H), 3.97 - 3.96 (m, 6H), 3.81 - 3.75 (m, 2H), 1.23 - 1.19 (m, 3H).

### Methyl-5-chlor-6-[7-methyl-3-(trifluormethyl)-7H-imidazo[4,5-c]pyridazin-6-yl]pyridin-2-carboxylat (LXXVIII-2)

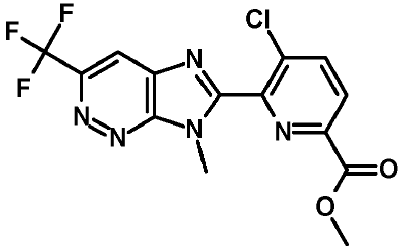

3-Chlor-6-(methoxycarbonyl)pyridin-2-carbonsäure (168 mg, 0.78 mmol) wurde in 4 ml DCM und 0.001 ml DMF gelöst. Bei Raumtemperatur wurde Oxalylchlorid (0.168 ml, 1.9 mmol) zugetropft und das Reaktionsgemisch wurde für 1 h gerührt. Alle flüchtigen Bestandteile wurden am Rotationsverdampfer entfernt und das Rohprodukt wurde in 10 ml 1,4-Dioxan gelöst. N3-Methyl-6-(trifluormethyl)pyridazin-3,4-diamin (150 mg, 0.78 mmol) wurde zugegeben und die Lösung wurde unter Rühren für 12 h auf 90°C erhitzt. Nach Entfernen des Dioxans am Rotationsverdampfer wurde der Rückstand chromatographisch aufgereinigt (Laufmittel: Cyclohexan, Essigester).

In Analogie zu den Beispielen und gemäß den oben beschriebenen Herstellverfahren lassen sich folgende Verbindungen der Formel (I) erhalten: 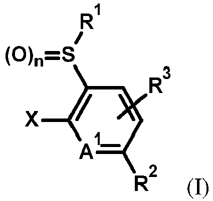 wobei die Substituenten R¹, R², R³, A¹, X und n die in der folgenden Tabelle angegebenen Bedeutungen haben und wobei die Bindung von R² sowie von X zum Restmolekül mit einer Wellenline gekennzeichnet ist:

| **Bsp.** | **R¹** | **n** | **A¹** | **R³** | **X** | **R²** |
|---|---|---|---|---|---|---|
| Bsp. I-9 | -CH₂CH₃ | 2 | N | H | 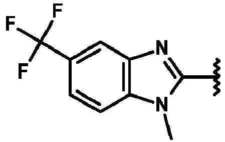 | 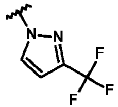 |
| Bsp. I-10 | -CH₂CH₃ | 2 | N | H | 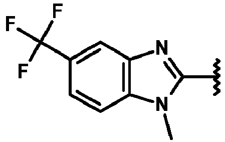 | 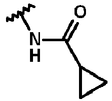 |
| Bsp. I-11 | -CH₂CH₃ | 2 | N | H | 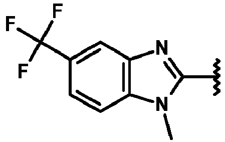 | 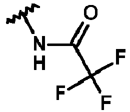 |
| Bsp. I-12 | -CH₂CH₃ | 1 | N | H | 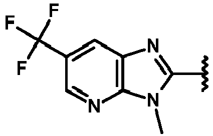 | 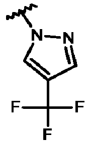 |
| Bsp. I-13 | -CH₂CH₃ | 2 | N | H | 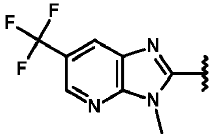 | 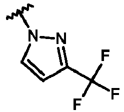 |
| Bsp. I-14 | -CH₂CH₃ | 2 | N | H | 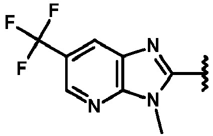 | 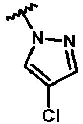 |
| Bsp. I-15 | -CH₂CH₃ | 2 | N | H | 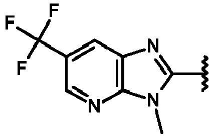 | 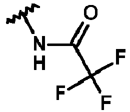 |
| Bsp. I-16 | -CH₂CH₃ | 2 | N | H | 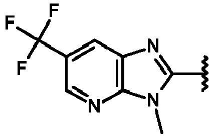 | 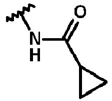 |
| Bsp. I-20 | -CH₂CH₃ | 2 | N | H | 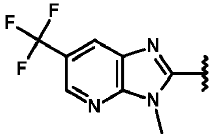 | 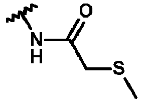 |
| Bsp. I-21 | -CH₂CH₃ | 2 | N | H | 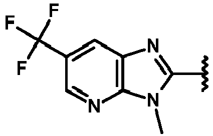 | 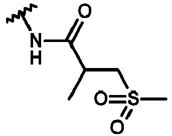 |
| Bsp. I-22 | -CH₂CH₃ | 2 | N | H | 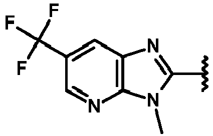 | 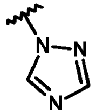 |
| Bsp. I-23 | -CH₂CH₃ | 2 | N | H | 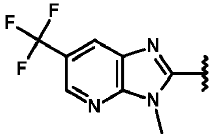 | 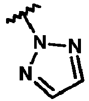 |
| Bsp. I-24 | -CH₂CH₃ | 2 | N | H | 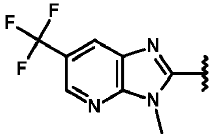 | 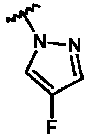 |
| Bsp. I-25 | -CH₂CH₃ | 2 | N | H | 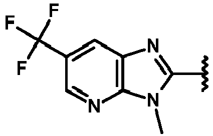 | 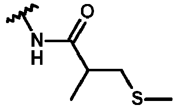 |
| Bsp. I-26 | -CH₂CH₃ | 2 | N | H | 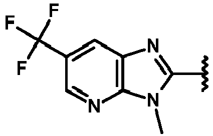 | 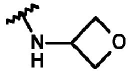 |
| Bsp. I-27 | -CH₂CH₃ | 2 | N | H | 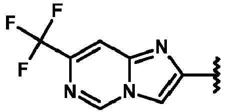 | 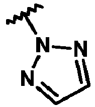 |
| Bsp. I-28 | -CH₂CH₃ | 2 | N | H | 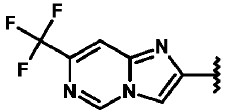 | 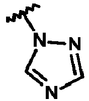 |
| Bsp. I-29 | -CH₂CH₃ | 2 | N | H | 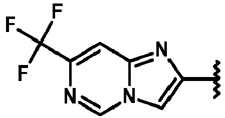 | 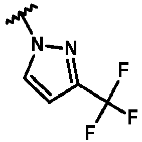 |
| Bsp. I-30 | -CH₂CH₃ | 2 | N | H | 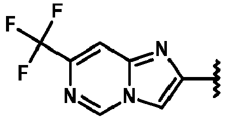 | 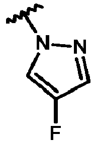 |
| Bsp. I-31 | -CH₂CH₃ | 2 | N | H | 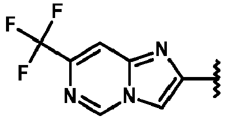 | 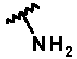 |
| Bsp. I-32 | -CH₂CH₃ | 2 | N | H | 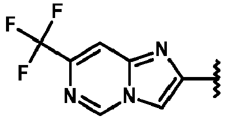 | 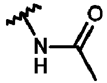 |
| Bsp. I-33 | -CH₂CH₃ | 2 | N | H | 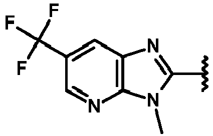 | 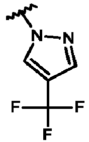 |
| Bsp. I-40 | -CH₂CH₃ | 2 | N | H | 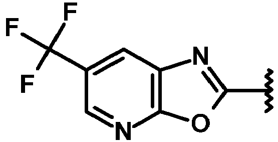 | 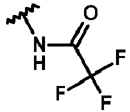 |
| Bsp. I-41 | -CH₂CH₃ | 2 | N | H | 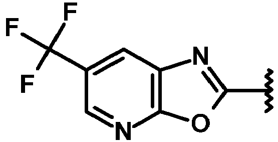 | 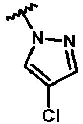 |
| Bsp. I-42 | -CH₂CH₃ | 2 | N | H | 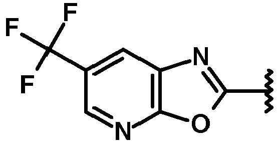 | 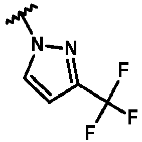 |
| Bsp I-43 | -CH₂CH₃ | 2 | N | H | 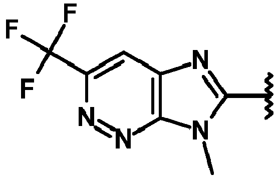 | 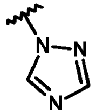 |
| Bsp I-44 | -CH₂CH₃ | 2 | N | H | 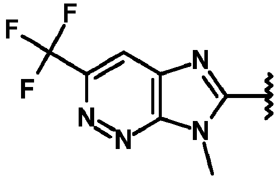 | 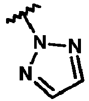 |
| Bsp. I-45 | -CH₂CH₃ | 2 | N | H | 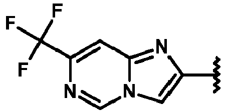 | 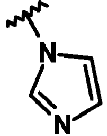 |
| Bsp. I-46 | -CH₂CH₃ | 2 | N | H | 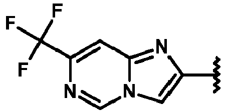 | 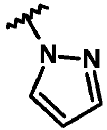 |

| Bsp. | Log P (neutral) | ¹H NMR |
|---|---|---|
| I-9 | 4.21 | 1H-NMR(400,0 MHz, DMSO): δ= 8,977(1,6); 8,975(1,8); 8,970(1,9); 8,968(1,7); 8,729(3,0); 8,707(3,5); 8,419(3,5); 8,397(3,2); 8,315(0,5); 8,161(2,6); 8,000(1,7); 7,979(2,5); 7,970(1,7); 7,766(1,4); 7,763(1,4); 7,745(1,2); 7,741(1,2); 7,176(2,6); 7,169(2,6); 6,709(1,9); 6,703(1,9); 5,755(1,6); 3,951(0,9); 3,933(3,2); 3,914(3,4); 3,895(16,0); 3,327(23,5); 2,526(0,5); 2,513(11,7); 2,508(24,8); 2,504(33,6); 2,499(24,2); 2,495(11,5); 1,990(0,8); 1,398(1,0); 1,263(3,3); 1,245(7,4); 1,226(3,2); 1,176(0,5) |
| I-10 | 2.92 | 1H-NMR(400,0 MHz, DMSO): δ= 11,564(2,7); 11,314(0,3); 8,706(0,4); 8,679(0,5); 8,658(0,5); 8,497(2,0); 8,486(0,7); 8,474(5,1); 8,468(0,7); 8,446(4,4); 8,424(1,8); 8,119(3,1); 8,101(0,4); 8,070(0,6); 8,049(0,6); 7,952(1,9); 7,942(0,5); 7,931(2,3); 7,920(0,6); 7,741(0,4); 7,728(1,7); 7,725(1,9); 7,707(1,4); 7,703(1,4); 5,753(5,9); 4,278(0,9); 4,260(1,7); 4,243(0,9); 3,908(0,4); 3,902(0,6); 3,889(0,4); 3,884(0,6); 3,782(3,8); 3,774(16,0); 3,711(1,1); 3,699(1,5); 3,693(3,4); 3,682(1,4); 3,675(3,5); 3,653(1,4); 3,636(0,5); 3,322(32,3); 2,920(0,4); 2,633(0,5); 2,564(0,5); 2,524(0,6); 2,511(16,7); 2,506(34,9); 2,502(47,0); 2,497(33,9); 2,493(16,3); 2,444(0,7); 2,424(1,4); 2,403(0,9); 2,382(0,4); 2,364(0,7); 2,346(0,4); 2,162(0,6); 2,142(0,7); 2,123(0,5); 2,087(0,4); 2,074(0,6); 2,056(1,2); 2,048(0,7); 2,041(1,3); 2,026(1,0); 2,011(0,6); 1,951(0,4); 1,934(0,5); 1,916(0,4); 1,628(0,4); 1,256(0,7); 1,243(0,9); 1,237(1,5); 1,224(0,5); 1,219(0,6); 1,189(3,6); 1,171(8,1); 1,152(3,5); 1,050(0,5); 1,042(0,8); 1,031(0,8); 0,997(0,4); 0,990(0,7); 0,982(0,4); 0,978(0,5); 0,971(0,8); 0,963(0,4); 0,928(0,3); 0,909(7,7); 0,896(3,8); 0,892(3,6); 0,862(0,4); 0,845(0,4); 0,826(0,4); 0,818(0,4); 0,811(0,5); 0,806(0,5); 0,146(0,4); 0,008(2,7); 0,000(76,8); -0,009(2,8); -0,150(0,4) |
| I-11 | 3.27 | 1H-NMR(400,0 MHz, DMSO): δ= 12,721(1,4); 8,623(2,8); 8,601(3,5); 8,432(3,5); 8,410(2,9); 8,134(2,3); 7,965(1,5); 7,944(1,9); 7,742(1,3); 7,738(1,3); 7,720(1,1); 7,716(1,1); 3,804(15,0); 3,779(3,0); 3,761(3,1); 3,742(0,9); 3,320(28,3); 2,670(0,3); 2,524(0,7); 2,519(1,1); 2,510(20,2); 2,506(43,3); 2,501(58,5); 2,497(41,8); 2,492(19,7); 2,328(0,3); 1,988(0,9); 1,398(16,0); 1,213(3,2); 1,194(7,3); 1,176(3,4); 0,146(0,3); 0,008(2,6); 0,000(82,6); -0,009(2,7); -0,150(0,4) |
| I-12 | 4.23 | 1H-NMR(400,0 MHz, DMSO): δ= 20.013, 9.359, 8.940, 8.936, 8.758, 8.754, 8.745, 8.724, 8.445, 8.439, 8.417, 5.754, 4.341, 4.227, 3.592, 3.574, 3.560, 3.541, 3.318, 3.119, 3.100, 3.086, 3.067, 2.525, 2.520, 2.511, 2.507, 2.502, 2.498, 2.493, 1.344, 1.325, 1.307, 0.000 |
| I-13 | 4.09 | 1H-NMR(400,0 MHz, DMSO): δ= 9,006(1,4); 9,004(1,5); 8,999(1,6); 8,997(1,4); 8,938(1,9); 8,934(2,0); 8,752(2,6); 8,730(3,8); 8,723(2,0); 8,448(3,1); 8,426(2,7); 7,971(0,7); 7,184(2,2); 7,177(2,2); 6,708(0,8); 6,703(0,7); 5,755(1,4); 3,930(0,8); 3,911(16,0); 3,893(2,8); 3,875(0,8); 3,322(18,0); 2,526(0,4); 2,513(9,9); 2,508(20,9); 2,504(28,1); 2,499(20,1); 2,495(9,4); 1,273(2,9); 1,255(6,5); 1,236(2,9) |
| I-14 | 3.9 | 1H-NMR(400,0 MHz, DMSO): δ= 9,009(4,8); 8,934(2,1); 8,930(2,1); 8,717(2,2); 8,713(2,2); 8,702(3,2); 8,680(3,7); 8,365(3,7); 8,343(3,4); 8,156(4,8); 7,959(0,5); 7,548(0,4); 5,754(3,7); 3,908(16,0); 3,889(3,1); 3,870(3,0); 3,852(0,9); 3,325(48,7); 2,526(0,4); 2,513(11,6); 2,508(24,7); 2,504(33,3); 2,499(23,7); 2,495(11,1); 1,268(3,3); 1,250(7,5); 1,231(3,3); 0,000(0,3) |
| I-15 | 2.00 | 1H-NMR(400,0 MHz, DMSO): δ= 12,747(1,4); 8,908(2,2); 8,905(2,2); 8,702(2,3); 8,698(2,3); 8,641(1,4); 8,618(1,7); 8,453(2,2); 8,431(1,8); 8,313(0,6); 3,821(16,0); 3,812(0,9); 3,775(0,9); 3,757(3,1); 3,738(3,2); 3,720(1,0); 3,317(166,8); 2,679(0,6); 2,675(1,3); 2,670(1,9); 2,666(1,4); 2,661(0,6); 2,524(3,9); 2,519(5,8); 2,510(107,8); 2,506(234,7); 2,501(320,4); 2,497(228,0); 2,492(106,0); 2,337(0,6); 2,333(1,3); 2,328(1,9); 2,323(1,3); 2,319(0,6); 1,398(11,3); 1,353(0,4); 1,221(3,5); 1,203(8,0); 1,184(3,4); 0,146(1,9); 0,008(13,8); 0,000(464,6); -0,009(14,8); -0,150(1,9) |
| I-16 | 2.76 | 1H-NMR(400,0 MHz, DMSO): δ= 11,596(2,6); 8,896(2,3); 8,893(2,4); 8,685(2,4); 8,680(2,3); 8,525(2,0); 8,503(5,1); 8,474(4,3); 8,452(1,8); 5,754(3,0); 3,790(16,0); 3,690(0,9); 3,671(3,2); 3,653(3,3); 3,634(1,0); 3,353(9,4); 3,343(16,3); 3,338(18,2); 3,334(18,0); 3,323(16,5); 2,525(0,6); 2,512(15,5); 2,507(32,9); 2,503(44,6); 2,498(31,9); 2,494(15,1); 2,060(0,8); 2,044(1,0); 2,029(0,9); 1,198(3,4); 1,180(7,8); 1,161(3,3); 0,914(7,3); 0,899(4,8); 0,008(2,0); 0,000(64,8); -0,009(2,0) |
| I-20 | 2.79 | 1H-NMR(400,0 MHz, d6-DMSO): δ= 12,491(0,4);11,394(2,6);8,896(2,3);8,893(2, 3);8,686(2,3);8,683(2,3);8,540(0,3);8,517(6,7);8,516(6,3);8,493(0,4);5,754(1,5);3,7 88(13,0);3,702(0,8);3,683(2,8);3,665(2,8);3,646(0,9);3,401(7,5);3,323(3,9);3,199(3 ,5);2,508(18,2);2,503(24,4);2,499(18,6);2,179(16,0);2,118(5,4);1,205(2,9);1,187(6, 4);1,168(2,9);0,000(1,7) |
| I-21 | 2.31 | 1H-NMR(400,0 MHz, d6-DMSO): δ= 11,471(2,9);8,897(2,5);8,894(2,5);8,691(2,6 );8,687(2,5);8,543(0,6);8,521(6,6);8,516(6,1);8,494(0,6);5,753(2,3);4,038(0,8);4,02 0(0,8);3,792(16,0);3,693(0,8);3,675(2,7);3,656(2,8);3,638(1,1);3,609(0,9);3,595(0, 9);3,572(0,6);3,317(45,5);3,302(2,7);3,288(0,8);3,272(1,3);3,262(0,8);3,009(14,6); 2,675(0,4);2,670(0,5);2,666(0,4);2,524(1,4);2,510(30,2);2,506(62,1);2,501(85,8);2, 497(64,9);2,493(32,1);2,333(0,4);2,328(0,5);2,324(0,4);2,115(0,7);1,988(3,3);1,28 7(5,0);1,270(5,0);1,235(2,9);1,201(3,5);1,193(1,4);1,183(7,9);1,175(2,4);1,164(3,4) ;1,158(1,2);0,881(0,4);0,854(0,3);0,008(2,8);0,000(72,9);-0,008(3,0) |
| I-22 | 2.49 | 1H-NMR(400,0 MHz, d6-DMSO): δ= 9,588(6,3);8,938(2,3);8,935(2,4);8,779(3,6); 8,757(4,1);8,727(2,4);8,723(2,3);8,460(7,3);8,352(4,0);8,330(3,7);5,753(0,3);3,934 (1,1);3,923(16,0);3,916(3,7);3,897(3,0);3,879(0,9);3,353(23,6);3,340(16,9);3,335(1 8,2);3,332(16,8);2,527(0,4);2,514(9,4);2,510(20,0);2,505(27,3);2,500(19,7);2,496( 9,4);1,277(3,6);1,259(8,2);1,240(3,5);0,000(8,4) |
| I-23 | 2.66 | 1H-NMR(400,0 MHz, d6-DMSO): δ= 9,064(3,9);9,061(3,8);8,940(2,5);8,935(3,5); 8,929(2,3);8,820(3,0);8,798(3,9);8,770(2,7);8,748(3,3);8,734(2,7);8,730(4,2);8,646 (3,9);8,625(3,2);8,534(3,3);8,512(2,8);8,360(11,8);8,091(4,0);8,087(4,0);3,942(1,0) ;3,924(3,5);3,912(16,0);3,906(4,3) ;3,887 (1,7);3,878(14,3);3,855(2,9);3,836(0,9);3, 321(55,0);2,677(0,4);2,672(0,5);2,668(0,4);2,525(1,2);2,512(28,8);2,507(58,5);2,5 03(79,8);2,498(60,0);2,494(29,6);2,334(0,3);2,330(0,5);2,325(0,4);2,075(6,8);1,28 5(3,4);1,266(9,4);1,249(8,6);1,231(2,9);0,000(1,1) |
| I-24 | 3.55 | 1H-NMR(400,0 MHz, d6-DMSO): δ= 8,931(2,4);8,927(2,6);8,897(2,2);8,885(2,3); 8,715(2,5);8,712(2,5);8,682(3,2);8,660(3,7);8,368(3,6);8,346(3,3);8,161(2,2);8,150 (2,3);5,754(1,6);3,899(16,0);3,878(3,3);3,859(3,2);3,841(1,0);3,318(27,4);2,525(0, 4);2,512(11,4);2,508(24,9);2,503(35,7);2,499(27,2);1,264(3,5);1,246(7,7);1,227(3, 4) |
| I-25 | 3,26 | 1H-NMR(400,0 MHz, d6-DMSO): δ= 12,256(1,4);11,381(1,4);8,896(1,3);8,892(1, 3);8,688(1,3);8,684(1,3);8,582(1,2);8,559(2,3);8,508(2,0);8,486(1,1);5,754(4,5);3,7 86(8,1);3,681(0,4);3,662(1,4);3,643(1,4);3,625(0,5);3,321(1,3);3,007(0,4);2,986(0, 4);2,814(0,6);2,793(0,5);2,781(0,7);2,760(0,6);2,731(0,8);2,714(1,1);2,700(1,1);2,6 83(1,5);2,603(0,6);2,586(1,2);2,569(1,3);2,559(0,7);2,552(0,9);2,544(0,7);2,536(0, 3);2,526(2,5);2,508(10,6);2,503(14,0);2,499(10,6);2,494(6,4);2,478(0,9);2,074(10, 3);2,052(16,0);1,199(2,2);1,194(3,6);1,181(4,8);1,177(4,0);1,163(1,8);1,138(6,6);1, 121(6,6);0,000(2,0) |
| I-26 | 2,27 | 1H-NMR(400,0 MHz, d6-DMSO): δ= 8,861(2,5);8,857(2,5);8,702(1,4);8,688(1,4); 8,613(2,5);8,609(2,5);8,003(1,9);7,981(2,0);6,874(0,6);6,852(0,6);4,950(0,3);4,934 (0,7);4,918(0,8);4,902(0,5);4,805(1,6);4,788(2,7);4,771(1,3);4,507(1,6);4,492(2,9); 4,477(1,4);4,038(0,9);4,020(0,9);3,740(16,0);3,626(0,7);3,607(2,1);3,589(2,2);3,57 0(0,7);3,318(26,9);2,675(0,6);2,671(0,8);2,666(0,6);2,524(1,9);2,511(46,0);2,506(9 7,7);2,502(137,2);2,497(103,1);2,493(50,3);2,333(0,6);2,329(0,8);2,324(0,6);1,989 (4,0);1,193(1,1);1,182(3,3);1,175(2,6);1,163(7,4);1,158(2,0);1,145(3,2);0,008(0,9); 0,000(27,8);-0,008(1,1) |
| I-27 | 2,28 | 1H-NMR(400,0 MHz, d6-DMSO): δ= 9,708(3,4);9,670(2,2);9,072(2,7);9,069(2,9); 8,968(3,3);8,774(5,3);8,759(2,3);8,737(2,6);8,708(3,6);8,686(4,1);8,428(2,7);8,413 (2,3);8,407(2,9);8,396(3,4);8,336(4,6);8,331(16,0);8,314(4,4);8,117(2,8);8,114(3,0) ;4,206(0,7);4,188(2,3);4,169(2,4);4,158(1,2);4,151(0,9);4,139(3,6);4,121(3,6);4,10 2(1,1);4,038(0,6);4,020(0,6);3,721(0,4);3,317(119,0);2,675(1,5);2,671(2,1);2,666(1 ,6);2,524(6,6);2,510(122,7);2,506(251,4);2,502(347,0);2,497(264,1);2,493(132,1);2 ,333(1,5);2,328(2,0);2,324(1,5);1,989(2,6);1,309(2,5);1,295(4,4);1,290(6,3);1,276( 8,9);1,258(3,8);1,193(0,7);1,175(1,3);1,157(0,6);0,146(0,4);0,008(2,9);0,000(77,5); -0,008(2,9) ;-0, 149(0,3) |
| I-28 | 2,19 | 1H-NMR(400,0 MHz, d6-DMSO): δ= 14,012(0,4);9,664(6,2);9,617(12,5);9,002(9, 5);8,719(6,8);8,697(7,5);8,560(0,6);8,557(0,6);8,442(13,1);8,408(6,1);8,314(1,8);8, 145(7,4);8,124(6,9);7,978(0,6);4,208(2,0);4,189(6,5);4,171(6,5);4,152(1,9);4,038(0 ,9);4,021(0,8);4,002(0,4);3,425(0,3);3,318(584,0);2,675(3,6);2,670(4,9);2,666(3,6); 2,631(0,5);2,524(15,2);2,510(290,5);2,506(594,5);2,501(820,4);2,497(623,2);2,492 (310,6);2,333(3,3);2,328(4,7);2,324(3,5);1,988(3,7);1,300(7,1);1,282(16,0);1,263(7 ,1);1,245(0,4);1,234(0,4);1,193(1,0);1,175(2,0);1,157(1,0);0,146(0,8);0,008(6,7);0, 000(190,3);-0,008(7,3);-0,150(0,9) |
| I-29 | 3,74 | 1H-NMR(400,0 MHz, d6-DMSO): δ= 9,660(6,3);9,048(3,8);9,043(3,9);8,994(9,6); 8,699(6,7);8,677(7,6);8,406(6,2);8,241(7,3);8,219(6,8);7,221(5,2);7,214(5,1);4,200 (1,9);4,181(6,4);4,163(6,5);4,144(1,9);4,039(0,5);4,021(0,5);3,321(57,0);2,677(0,5) ;2,672(0,7);2,668(0,5);2,525(2,0);2,512(39,9);2,508(81,4);2,503(111,7);2,498(84,4 );2,494(42,0);2,334(0,5);2,330(0,7);2,325(0,5);1,989(2,0);1,398(1,2);1,299(7,1);1,2 80(16,0);1,262(7,0);1,194(0,5);1,176(1,0);1,158(0,5);0,008(0,9);0,000(25,6);-0,008(1,0) |
| I-30 | Sauer: 3.13 | 1H-NMR(400,0 MHz, d6-DMSO): δ= 9,645(7,3);8,987(10,2);8,934(4,9);8,923(5,2 );8,630(6,1);8,608(7,0);8,394(7,1);8,313(0,8);8,163(6,8);8,141(6,6);8,129(5,2);8,11 9(5,4);5,753(0,6);4,179(2,1);4,161(6,9);4,142(7,3);4,124(2,5);3,320(305,1);3,301(1 8,1);2,671(2,2);2,541(2,6);2,506(258,0);2,502(360,8);2,498(301,0);2,329(2,1);1,98 9(0,8);1,398(0,4);1,290(7,2);1,272(16,0);1,253(7,8);1,176(0,5);0,000(18,3);-0,019(0,7) |
| I-31 | 1,59 | 1H-NMR(400,0 MHz, d6-DMSO): δ= 9,726(1,1);9,655(7,7);8,428(10,8);8,354(1,2 );8,340(1,9);8,293(7,5);8,024(0,9);8,002(1,0);7,931(6,0);7,909(6,3);7,280(1,2);7,10 9(7,0);6,667(1,0);6,644(1,0);6,617(6,4);6,595(6,3);4,094(0,5);4,081(0,5);3,809(2,1) ;3,791(7,1);3,772(7,2);3,754(2,2);3,319(23,6);3,176(2,1);3,163(2,0);3,030(0,3);3,0 12(1,1);2,994(1,1);2,975(0,4);2,671(0,6);2,506(74,4);2,502(99,7);2,498(77,7);2,32 9(0,6);1,195(7,4);1,177(16,0);1,158(7,3);1,017(1,2);0,999(2,5);0,981(1,1);0,007(2, 4);0,000(49,9);-0,008(2,4) |
| I-32 | 1,99 | 1H-NMR(400,0 MHz, d6-DMSO): δ= 11,154(2,4);9,723(3,2);8,544(4,9);8,448(0,4 );8,424(2,2);8,402(3,8);8,351(3,4);8,341(3,3);8,319(2,0);4,038(0,5);4,021(0,5);3,90 3(0,9);3,884(3,2);3,866(3,2);3,847(1,0);3,320(62,4);2,676(0,4);2,671(0,5);2,667(0, 4);2,524(1,5);2,511(28,9);2,507(58,7);2,502(80,5);2,498(60,4);2,493(29,6);2,333(0 ,4);2,329(0,5);2,324(0,4);2,173(16,0);1,989(2,0);1,214(3,6);1,195(8,1);1,177(3,9);1,158(0,5);1,018(0,4);0,008(0,8);0,000(20,3);-0,008(0,7) |
| I-33 | 4,04 | 1H-NMR(400,0 MHz, d6-DMSO): δ= 9,421(2,9);8,940(2,2);8,936(2,3);8,756(3,0); 8,734(3,6);8,725(2,4);8,721(2,3);8,473(4,1);8,451(3,5);8,429(3,1);3,913(16,0);3,89 5(3,2);3,876(3,2);3,858(0,9);3,320(76,4);2,676(0,3);2,671(0,5);2,667(0,3);2,525(1, 3);2,511(28,6);2,507(59,0);2,502(78,7);2,498(56,9);2,493(27,6);2,334(0,4);2,329(0 ,5);2,325(0,3);1,398(3,5);1,271(3,2);1,252(7,4);1,234(3,3);0,008(0,4);0,000(11,5);-0,008(0,5) |
| I-40 | 3.09 | 1H-NMR(400,2 MHz, d6-DMSO): d= 12,997(1,2);11,045(0,5);10,220(0,7);9,060(5 ,3);9,031(5,1);8,964(0,7);8,757(0,3);8,703(4,3);8,681(5,7);8,650(0,4);8,534(5,8);8, 512(4,7);8,371(0,5);8,349(0,3);7,958(0,4);7,714(0,4);7,682(0,4);7,558(0,4);4,239(0 ,5);4,222(1,0);4,206(0,5);3,845(2,1);3,827(6,5);3,808(6,5);3,790(2,2);3,708(0,4);3, 632(0,6);3,608(0,7);3,600(0,8);3,582(1,0);3,564(1,2);3,521(1,9);3,502(2,3);3,384(4 7,5);3,346(54,0);3,170(0,6);3,123(0,5);3,085(0,4);2,677(1,9);2,673(2,4);2,508(255, 9);2,504(327,2);2,500(240,9);2,331(2,1);2,327(1,7);2,088(0,6);1,911(6,5);1,660(0, 4);1,644(0,5);1,623(0,4);1,474(0,4);1,430(0,3);1,398(0,7);1,384(0,7);1,364(0,7);1,3 37(1,2);1,290(7,7);1,272(16,0);1,253(8,0);1,235(4,1);1,212(1,7);1,205(1,3);1,187(1 ,8);1,168(1,1);1,148(2,8);1,107(0,6);1,089(0,5);1,066(0,5);1,005(0,5);0,986(0,8);0, 968(0,5);0,931(1,1);0,913(1,8);0,894(1,0);0,855(1,4);0,834(1,4);0,813(1,1);0,746(0 ,4);0,723(0,4);0,147(0,5);0,001(95,8);-0,149(0,4) |
| I-41 | 4.01 | 1H-NMR(400,0 MHz, d6-DMSO): δ= 9.07 - 9.05 (m, 2H), 8.93 (s, 1H), 8.75 - 8.72 (m, 1H), 8.43 - 8.41 (m, 1H), 8.19 (s, 1H), 3.84 - 3.83 (m, 2H), 1.30 - 1.27 (m, 3H) |
| I-42 | 4.17 | 1H-NMR(400,0 MHz, d6-DMSO): 9.07 - 9.05 (m, 2H), 8.94 - 8.93 (m, 1H), 8.80 - 8.77 (m, 1H), 8.51 - 8.49 (m, 1H), 7.20-7-19 (m, 1H), 3.88 - 3.86 (m, 2H), 1.32 - 1.17 (m, 3H) |
| I-43 | 2.08 | 1H-NMR(400,0 MHz, d6-DMSO): d= 9,582(6,4);8,832(6,1);8,807(3,2);8,786(3,6); 8,476(6,4);8,399(3,6);8,377(3,3);4,100(16,0);3,863(1,0);3,845(3,4);3,826(3,4);3,80 8(1,1);3,322(94,8);2,675(0,6);2,671(0,8);2,506(103,3);2,502(134,9);2,498(98,2);2, 328(0,7);2,324(0,6);1,273(3,7);1,255(8,2);1,236(4,3);0,146(0,6);0,008(5,5);0,000(1 23,0);-0,150(0,6) |
| I-44 | 2.21 | 1H-NMR(400,0 MHz, d6-DMSO): d= 8,836(0,4);8,823(5,9);8,801(3,0);8,779(3,7); 8,585(3,6);8,563(3,1);8,372(13,0);4,088(0,7);4,047(16,0);3,812(1,0);3,794(3,3);3,7 75(3,4);3,757(1,0);3,322(45,9);2,675(0,3);2,671(0,4);2,667(0,3);2,506(58,1);2,502( 76,0);2,498(55,7);2,329(0,4);1,262(4,0);1,243(8,5);1,225(3,8);0,007(2, 8);-0,001(59,3);-0,009(2,9) |
| I-45 | 2.07 | 1H-NMR(400,0 MHz, d6-DMSO): d= 20,010(0,6);9,645(7,8);8,943(10,5);8,766(7, 9);8,611(5,8);8,589(6,5);8,395(7,8);8,316(0,8);8,189(0,6);8,140(8,2);8,137(8,5);8,1 13(5,6);7,220(7,5);4,635(0,6);4,180(2,3);4,162(7,0);4,143(7,1);4,126(2,3);3,321(39 4,3);2,906(0,6);2,670(4,6);2,501(814,9);2,328(4,6);1,296(7,5);1,277(16,0);1,259(7, 4);0,146(1,9);0,041(0,6);0,038(0,7);-0,001(436,0);-0,149(2,2);-3,611(0,6) |
| I-46 | 2.58 | 1H-NMR(400,0 MHz, d6-DMSO): d= 16,349(0,3);12,568(0,4);9,656(8,1);9,203(1, 0);8,957(0,4);8,931(10,4);8,805(6,3);8,798(6,3);8,715(1,4);8,626(5,8);8,605(6,4);8, 552(1,4);8,530(1,6);8,493(0,7);8,395(8,0);8,358(0,3);8,313(0,5);8,268(1,4);8,248(1 ,3);8,181(6,6);8,160(6,0);7,985(8,3);6,711(6,3);6,684(1,5);5,507(0,3);4,153(2,4);4, 135(7,3);4,116(7,3);4,098(2,6);3,719(0,4);3,643(0,4);3,600(0,5);3,589(0,8);3,571(1 ,9);3,553(2,0);3,533(0,9);3,488(0,9);3,330(497,4);3,213(0,8);3,174(0,5);3,105(0,3); 2,990(0,3);2,672(2,8);2,502(491,2);2,429(0,7);2,404(0,5);2,380(0,4);2,328(2,7);2,3 00(0,4);2,074(2,3);1,288(7,6);1,270(16,0);1,251(7,8);1,234(2,4);1,216(4,0);1,198(1 ,9);1,164(0,5);1,148(0,4);0,145(1,1);0,000(225,6);-0,001(218,4);-0,071(0,5);-0,151(1,4) |

Die Messung der logP Werte erfolgt gemäß EEC Directive 79/831 Annex V.A8 durch HPLC (High Performance Liquid Chromatography) an einer Phasenumkehrsäule (C 18). Temperatur: 55°C.

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. In der Tabelle logP (HCOOH) genannt.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. In der Tabelle logP (neutral) genannt.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Daten ausgewählter Beispiele werden entweder in klassischer Form (δ-Werte, Multiplettaufspaltung, Anzahl der H-Atome) oder als NMR-Peak-Listen aufgeführt.

Das Lösungsmittel, in welchem das NMR-Spektrum aufgenommen wurde ist jeweils angegeben.

### NMR-Peak-Listenverfahren

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ ₁ (Intensität₁); δ₂ (Intensität₂);........; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

### Anwendungsbeispiele

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-1, I-2, I-3, I-5, I-6, I-7, I-8, I-10, I-11, I-13, I-14, I-15, I-16, I-17, I-20, I-21, I-22, I-23, I-24, I-25, I-29, I-31, I-32

Bei diesem Test zeigen z. B. die folgenden Verbindungen der Herstellungsbeispiele Wirkung von 83% bei einer Aufwandmenge von 100g/ha: I-4

### Spodoptera frugiperda - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben *(Zea mays)* werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: I-1, I-2, I-4, I-8, I-9, I-10, I-12, I-13, I-14, I-16, I-17, I-22, I-23, I-24, I-29, I-31

Bei diesem Test zeigen z. B. die folgenden Verbindungen der Herstellungsbeispiele Wirkung von 83% bei einer Aufwandmenge von 500g/ha: I-7, I-15

Bei diesem Test zeigen z. B. die folgenden Verbindungen der Herstellungsbeispiele Wirkung von 80% bei einer Aufwandmenge von 500g/ha: I-32

Bei diesem Test zeigen z. B. die folgenden Verbindungen der Herstellungsbeispiele Wirkung von 100% bei einer Aufwandmenge von 100g/ha: I-5

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator : | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: 1-8

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 5-6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: I-2, I-3, I-15, I-16, I-22

Bei diesem Test zeigen z. B. die folgenden Verbindungen der Herstellungsbeispiele Wirkung von 90% bei einer Aufwandmenge von 500g/ha: I-5, I-6, I-8, I-11, I-20, I-21, I-23, I-24, I-25, I-31, I-32

Bei diesem Test zeigen z. B. die folgenden Verbindungen der Herstellungsbeispiele Wirkung von 90% bei einer Aufwandmenge von 100g/ha: I-9

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 (µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 (µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 (µg/cm² (= 500g/ha): I-2, I-10, I-11, I-22

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 (µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 (µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 80% bei einer Aufwandmenge von 5 (µg/cm² (= 500g/ha): I-11

### Boophilus microplus -Iniektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 80% bei einer Aufwandmenge von 20µg/Tier: I-22

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: I-10, I-11, I-22

Bei diesem Test zeigen z.B. die folgenden Verbindungen der Herstellungsbeispiele eine Wirkung von 95% bei einer Aufwandmenge von 100ppm: I-2

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-2, I-4, I-9, I-11, I-13, I-14, I-22

Bei diesem Test zeigen z.B. die folgenden Verbindungen der Herstellungsbeispiele eine Wirkung von 95% bei einer Aufwandmenge von 100ppm: I-10

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 90% bei einer Aufwandmenge von 100ppm: I-2

Bei diesem Test zeigen z.B. die folgenden Verbindungen der Herstellungsbeispiele eine Wirkung von 80% bei einer Aufwandmenge von 100ppm: I-4, I-13

### Meloidogyne incognita- Test

| | | |
|---|---|---|
| Lösungsmittel: | 125,0 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20ppm: I-6, I-15, I-21

## Patentansprüche

1. Verbindungen der Formel (I) 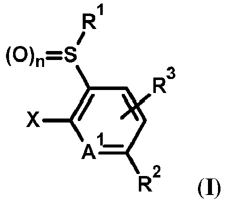 in welcher
A¹ für Stickstoff, =N⁺-O⁻ oder =C-R⁴ steht,
R¹ für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Amino, (C₁-C₆)Alkylamino, Di-(C₁-C₆)alkyl-amino, (C₃-C₈)Cycloalkylamino, (C₁-C₆)Alkylcarbonyl-amino, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₁-C₆)Alkylsulfonylamino, Aminosulfonyl-(C₁-C₆)alkyl, (C₁-C₆)Alkylaminosulfonyl-(C₁-C₆)alkyl, Di-(C₁-C₆)alkyl-aminosulfonyl-(C₁-C₆)alkyl,
oder für jeweils gegebenenfalls einfach oder mehrfach, gleich oder verschieden durch Aryl, Hetaryl oder Heterocyclyl substituiertes (C₁-C₆)Alkyl, (C₁-C₆)Alkoxy, (C₂-C₆)Alkenyl, (C₂-C₆)Alkinyl, (C₃-C₈)Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl oder Benzyl substituiert sein können, oder
R¹ für jeweils gegebenenfalls einfach oder mehrfach, gleich oder verschieden durch Halogen, Cyano, Nitro, Hydroxy, Amino, Carboxy, Carbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl, (=O) (nur im Fall von Heterocyclyl) oder (=O)₂ (nur im Fall von Heterocyclyl) substituiertes Aryl, Hetaryl oder Heterocyclyl steht,
R² weiterhin für eine Gruppe steht, ausgewählt aus -C(=O)-R⁸ (G1), -C(=S)-R⁸ (G2), -C(=O)-NR¹¹R¹² (G3), -C(=S)-NR¹¹R¹² (G4), -CR¹¹=N-OR¹³ (G18) oder -CR¹¹=CR⁸R¹²G(19),
R³ für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino), für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
R⁴ für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, -NHCO-(C₁-C₆)alkyl ((C₁-C₆)Alkylcarbonylamino),
X für ein teilweise gesättigtes oder gesättigtes heterozyklisches oder heteroaromatisches 8-, 9-, 10-, 11- oder 12-gliedriges annelliertes bicyclisches oder tricyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert ist, und wobei die Substituenten unabhängig voneinander ausgewählt sein können aus Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Alkinyloxy-(C₁-C₄)alkyl, (C₂-C₆)Halogenalkinyl, (C₂-C₆)Cyanoalkinyl, (C₁-C₆)Alkoxy, (C₁-C₆)Halogenalkoxy, (C₁-C₆)Halogenalkoxy-(C₁-C₆)alkyl, (C₂-C₆)Alkenyloxy-(C₁-C₆)alkyl, (C₂-C₆)Halogenalkenyloxy-(C₁-C₆)alkyl, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylcarbonyl-(C₁-C₆)alkyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino), oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
wobei X nicht für 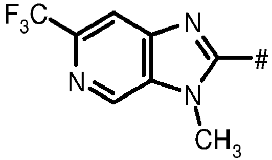 steht,
R⁸ für Wasserstoff, oder für jeweils gegebenenfalls einfach oder mehrfach, gleich oder verschieden substituiertes Amino, C₁-C₆-Alkyl, C₂-C₆-Alkenyl, C₂-C₆-Alkinyl, C₁-C₄-Alkoxy, C₃-C₁₂-Cycloalkyl, C₃-C₁₂-Cycloalkyl-C₁-C₆-Alkyl oder C₄-C₁₂-Bicycloalkyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-Alkyl, C₁-C₄-Alkylsulfimino-C₂-C₅-Alkylcarbonyl, C₁-C₄-Alkylsulfoximino, C₁-C₄-Alkylsulfoximino-C₁-C₄-alkyl, C₁-C₄-Alkylsulfoximino-C₂-C₅-Alkylcarbonyl, C₂-C₆-Alkoxycarbonyl, C₂-C₆-Alkylcarbonyl, C₃-C₆-Trialkylsilyl, Amino, C₁-C₄-Alkylamino, Di-(C₁-C₄-alkyl)amino, C₃-C₆-Cycloalkylamino, einem Phenylring oder einem 3- bis 6-gliedrigenaromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino, oder
R⁸ für einen Phenylring oder einen 3- bis 6-gliedrigen aromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, CN, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
R¹³ für jeweils gegebenenfalls einfach oder mehrfach, gleich oder verschieden substituiertes C₁-C₆-Alkyl, C₂-C₆-Alkenyl, C₂-C₆-Alkinyl, C₃-C₁₂-Cycloalkyl, C₃-C₁₂-Cycloalkyl-C₁-C₆-Alkyl oder C₄-C₁₂-Bicycloalkyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino- C₁-C₄-alkyl, C₁-C₄-Alkylsulfimino-C₂-C₅-alkylcarbonyl, C₁-C₄-Alkylsulfoximino, C₁-C₄-Alkylsulfoximino- C₁-C₄-alkyl, C₁-C₄-Alkylsulfoximino-C₂-C₅-alkylcarbonyl, C₂-C₆-Alkoxycarbonyl, C₂-C₆-Alkylcarbonyl, C₃-C₆-Trialkylsilyl, Amino, C₁-C₄-Alkylamino, Di-(C₁-C₄-Alkyl)amino, C₃-C₆-Cycloalkylamino, einem Phenylring oder einem 3- oder 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, Halogen, Cyano, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
R¹³ für einen Phenylring oder einen 3- bis 6-gliedrigen aromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Halogen-alkyl, C₂-C₆-Halogenalkenyl, C₂-C₆-Halogenalkinyl, C₃-C₆-Halogencycloalkyl, Halogen, Cyano, (C=O)OH, CONH₂, NO₂, OH, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)( C₁-C₄-Alkoxy)imino,
R¹¹, R¹² unabhängig voneinander für Wasserstoff oder für R¹³ stehen,
n für 0, 1 oder 2 steht, ausgenommen folgende Verbindung:

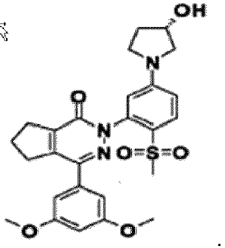

2. Verbindungen der Formel (I) gemäß Anspruch 1, in welcher
A¹ für Stickstoff, =N⁺-O⁻ oder =C-R⁴ steht,
R¹ für (C₁-C₄)Alkyl, (C₁-C₄)Hydroxyalkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyloxy-(C₁-C₄)alkyl, (C₂-C₄)Halogenalkinyl, (C₂-C₄)Cyanoalkinyl, (C₃-C₆)Cycloalkyl, (C₃-C₆)Cycloalkyl(C₃-C₆)cycloalkyl, (C₁-C₄)Alkyl-(C₃-C₆)cycloalkyl, Halogen(C₃-C₆)cycloalkyl, (C₁-C₄)Alkylamino, Di-(C₁-C₄)alkyl-amino, (C₃-C₆)Cycloalkylamino, (C₁-C₄)Alkylcarbonyl-amino, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Alkylcarbonyl-(C₁-C₄)alkyl, (C₁-C₄)Halogenalkylcarbonyl-(C₁-C₄)alkyl, (C₁-C₄)Alkylsulfonylamino,
oder für jeweils gegebenenfalls einfach oder zweifach, gleich oder verschieden durch Aryl, Hetaryl oder Heterocyclyl substituiertes (C₁-C₄)Alkyl, (C₁-C₄)Alkoxy, (C₂-C₄)Alkenyl, (C₂-C₄)Alkinyl, (C₃-C₆)Cycloalkyl steht, wobei Aryl, Hetaryl oder Heterocyclyl jeweils gegebenenfalls einfach oder zweifach gleich oder verschieden durch Halogen, Cyano, Carbamoyl, Aminosulfonyl, (C₁-C₄)Alkyl, (C₃-C₄)Cycloalkyl, (C₁-C₄)Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Alkylsulfimino substituiert sein können, oder
R¹ für jeweils gegebenenfalls einfach oder zweifach, gleich oder verschieden durch Halogen, Cyano, Carbamoyl, (C₁-C₄)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Alkylsulfimino, (C₁-C₄)Alkylsulfoximino, (C₁-C₄)Alkylcarbonyl, (C₃-C₄)Trialkylsilyl, (=O) (nur im Fall von Heterocyclyl) oder (=O)₂ (nur im Fall von Heterocyclyl) substituiertes Aryl, Hetaryl oder Heterocyclyl steht,
R² weiterhin für eine Gruppe steht, ausgewählt aus -C(=O)-R⁸ (G1), -C(=S)-R⁸ (G2), -C(=O)-NR¹¹R¹² (G3), -C(=S)-NR¹¹R¹² (G4), -CR¹¹=N-OR¹³ (G18) oder-CR¹¹=CR⁸R¹²G(19),
R³ für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino),
weiterhin für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Halogen, Nitro, Acetyl, Amino, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl((C₁-C₄)Alkylcarbonylamino),
R⁴ für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C1-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino) steht,
X für ein heteroaromatisches 8-, 9-, 10-, 11- oder 12-gliedriges annelliertes bicyclisches oder tricyclisches Ringsystem steht, wobei das Ringsystem gegebenenfalls einfach oder mehrfach, gleich oder verschieden substituiert ist, und wobei die Substituenten unabhängig voneinander ausgewählt sein können aus Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Alkinyloxy-(C₁-C₄)alkyl, (C₂-C₆)Halogenalkinyl, (C₁-C₆)Alkoxy, (C₁-C₆)Halogenalkoxy, (C₁-C₆)Halogenalkoxy-(C₁-C₆)alkyl, (C₂-C₆)Alkenyloxy-(C₁-C₆)alkyl, (C₂-C₆)Halogenalkenyloxy-(C₁-C₆)alkyl, (C₁-C₆)Cyanoalkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylcarbonyl-(C₁-C₆)alkyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino),
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, C₁-C₄-Alkoxy, C₁-C₄-Haloalkoxy substituiert sein können,
wobei X nicht für 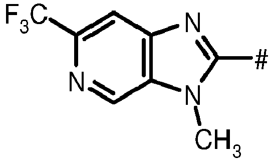 steht,
R⁸ für Wasserstoff, Amino, für jeweils gegebenenfalls einfach oder mehrfach, gleich oder verschieden substituiertes C₁-C₄-Alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, C₁-C₄-Alkylamino, C₃-C₆-Cycloalkyl, C₃-C₆-Cycloalkyl-C₁-C₄-alkyl, C₁-C₄-Alkoxy oder C₁-C₄-Alkoxy-C₁-C₄-alkyl, wobei die Substituenten unabhängig voneinander ausgewählt sein können aus Halogen, Cyano, einem Phenylring oder einem 3- bis 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen, Cyano, NO₂, C₁-C₄-Alkoxy oder C₁-C₄-Halogenalkoxy, oder
R⁸ für einen Phenylring oder einen 3- bis 6-gliedrigenaromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
R¹³ für jeweils gegebenenfalls einfach oder mehrfach, gleich oder verschieden substituiertes C₁-C₄-Alkyl oder C₃-C₆-Cycloalkyl steht, wobei die Substituenten unabhängig voneinander ausgewählt sein können aus Halogen, Cyano, einem Phenylring oder einem 3- bis 6-gliedrigen aromatischen, teilgesättigten oder gesättigten Heterocyclus, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen, Cyano, NO₂, C₁-C₄-Alkoxy oder C₁-C₄-Halogenalkoxy, oder
R¹³ für einen Phenylring oder einen 3- bis 6-gliedrigen aromatischen, teilgesättigten oder gesättigten Heterocyclus steht, wobei die Heteroatome aus der Reihe N,S,O ausgewählt sind, wobei der Phenylring bzw. Heterocyclus jeweils gegebenenfalls einfach oder mehrfach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
R¹¹, R¹² unabhängig voneinander für Wasserstoff oder für R¹³stehen,
n für 0, 1 oder 2 steht,
ausgenommen folgende Verbindung:

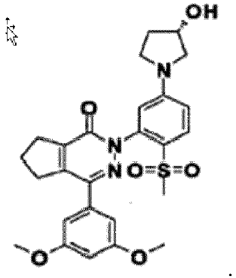

3. Verbindungen der Formel (I) gemäß Anspruch 1, in welcher
A¹ für Stickstoff oder =C-R⁴ steht,
R¹ für (C₁-C₄)Alkyl, (C₁-C₄)Hydroxyalkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl steht,
R² weiterhin für eine Gruppe ausgewählt aus G1, G2, G3, G4, G18 oder G19 steht,
R³ für Wasserstoff, Cyano, Halogen, Nitro, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Alkoxy-(Ci-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, NHCO-(Ci-C₄)alkyl ((C₁-C₄)Alkylcarbonylamino),
weiterhin für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Halogen, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
R⁴ für Wasserstoff, Halogen, Cyano oder (C₁-C₄)Alkyl steht,
X für ein heteroaromatisches 9-gliedriges oder 12-gliedriges annelliertes bicyclisches oder tricyclisches Ringsystem aus der Reihe H1 bis H20 steht, 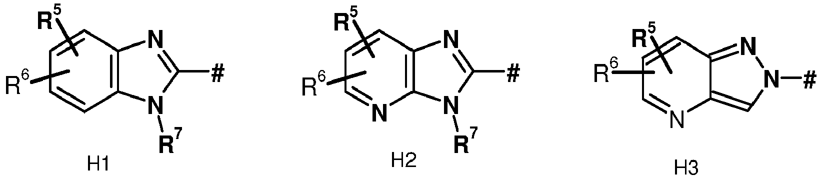 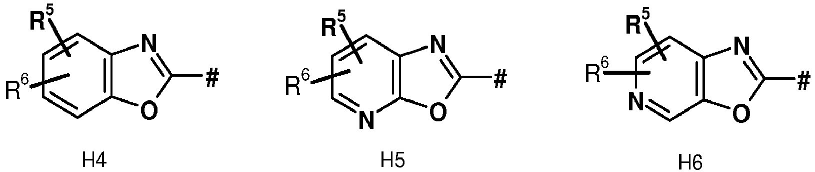 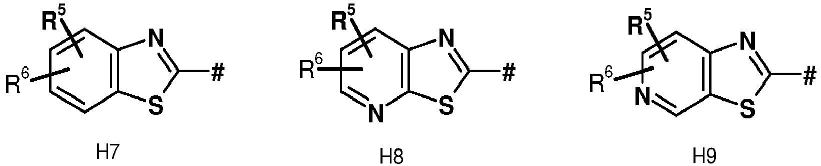 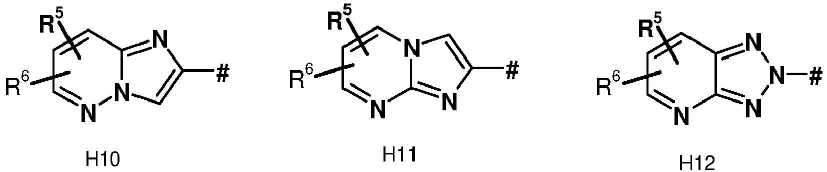 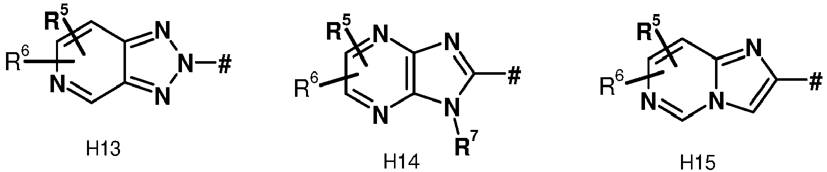 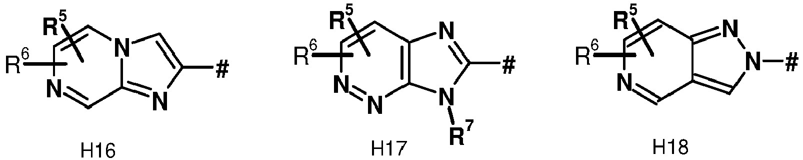 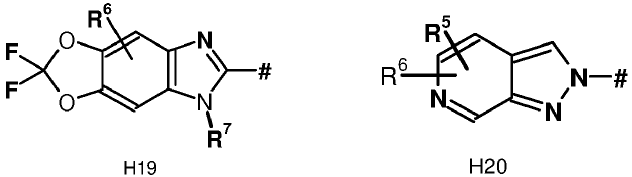 wobei die Bindung zum Restmolekül mit einer Raute ^{#}gekennzeichnet ist,
R⁵, R⁶ unabhängig voneinander für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl stehen,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁸ für Wasserstoff, Amino, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, (C₁-C₄)Alkylamino, (C₁-C₄)Alkoxy-(C₁-C₄)alkyl, (C₃-C₆)Cycloalkyl oder (C₃-C₆)Cycloalkyl-(C₁-C₄)alkyl, für jeweils gegebenenfalls einfach durch Halogen, Cyano, Phenyl oder Pyridyl substituiertes (C₁-C₄)Alkyl oder (C₁-C₄)Alkoxy, wobei Phenyl bzw. Pyridyl jeweils gegebenenfalls einfach oder zweifach, gleich oder verschieden durch Trifluormethyl, Cyano, Fluor, Chlor, Brom oder Trifluormethoxy substituiert sein kann, oder
R⁸ für Phenyl, Thiazolyl, Pyrazolyl, Pyridyl oder einen 3- bis 6-gliedrigen gesättigten Heterocyclus steht, enthaltend 1-2 Heteroatome aus der Reihe N,S,O, wobei Phenyl, Pyridyl bzw. der Heterocyclus jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
R¹³ für jeweils gegebenenfalls einfach durch Halogen, Cyano, Phenyl oder Pyridyl substituiertes (C₁-C₄)Alkyl oder (C₃-C₆)Cycloalkyl, wobei Phenyl bzw. Pyridyl jeweils gegebenenfalls einfach oder zweifach, gleich oder verschieden durch Trifluormethyl, Cyano, Fluor, Chlor oder Trifluormethoxy substituiert sein kann, oder
R¹³ für Phenyl, Pyridyl, oder einen 3- bis 6-gliedrigen gesättigten Heterocyclus steht, enthaltend 1-2 Heteroatome aus der Reihe N,S,O, wobei Phenyl, Pyridyl, bzw. der Heterocyclus jeweils gegebenenfalls einfach oder zweifach, gleich oder verschieden substituiert sein kann, und wobei die Substituenten unabhängig voneinander ausgewählt sein können aus C₁C₄-Alkyl, C₁-C₄-Halogenalkyl, Halogen oder Cyano,
R¹¹, R¹² unabhängig voneinander für Wasserstoff oder für R¹³ stehen,
n für 0, 1 oder 2 steht.

4. Verbindungen der Formel (I) gemäß Anspruch 1, in welcher
A¹ für Stickstoff oder =C-R⁴ steht,
R¹ für Methyl, Ethyl, n-Propyl, i-Propyl, cyclo-Propyl, n-Butyl, i-Butyl, tert.-Butyl, cycloButyl, Fluormethyl, Difluormethyl, Trifluormethyl, Fluorethyl, Difluorethyl, Trifluorethyl, Tetrafluorethyl oder Pentafluorethyl steht,
R² weiterhin für eine Gruppe ausgewählt aus G1, G2, G3, G4, G18 oder G19 steht,
R³ für Wasserstoff, Cyano, Halogen, (C₁C₄)Alkyl, (C₁C₄)Halogenalkyl, (C₁-C₄)Halogenalkoxy, (C₁C₄)Alkylthio, (C₁C₄)Alkylsulfinyl, (C₁-₄)Alkylsulfonyl, (C₁-C₄)Halogenalkylthio, (C₁-C₄)Halogenalkylsulfinyl, (C₁-C₄)Halogenalkylsulfonyl oder NHCO-(C₁-C₄)alkyl ((C₁-C₄)Alkylcarbonylamino) steht,
R⁴ für Wasserstoff, Fluor, Chlor, Brom oder Cyano steht,
X für ein heteroaromatisches 9-gliedriges oder 12-gliedriges annelliertes bicyclisches oder tricyclisches Ringsystem aus der Reihe H1, H2, H3, H4, H5, H7, H8, H10, H11, H12, H13, H15, H16, H17, H18, H19 oder H20 steht,
R⁵ für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylcarbonyl, (C₁-C₄)Halogenalkylcarbonyl, (C₁-C₄)Alkylaminocarbonyl, Di-(C₁-C₄)alkyl-aminocarbonyl, (C₁-C₄)Alkylsulfonylamino, (C₁-C₄)Alkylaminosulfonyl oder Di-(C₁-C₄)alkylaminosulfonyl steht,
R⁶ für Wasserstoff steht,
R⁷ für (C₁-C₄)Alkyl oder (C₁-C₄)Alkoxy-(C₁-C₄)alkyl steht,
R⁸ für Wasserstoff, (C₁-C₄)Alkyl, (C₁-C₄)Halogenalkyl, (C₁-C₄)Alkoxy-(C₁-C₄)alkyl, (C₁-C₄)Alkylthio-(C₁-C₄)alkyl, (C₁-C₄)Alkylsulfonyl-(C₁-C₄)alkyl, (C₃-C₆)Cycloalkyl, (C₃-C₆)Cycloalkyl-(C₁-C₄)alkyl oder (C₁-C₄)Alkoxy, oder
R⁸ für Phenyl oder Pyridyl steht, wobei Phenyl bzw. Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano,
R¹³ für (C₁-C₄)Alkyl oder (C₃-C₆)Cycloalkyl oder
R¹³ für Phenyl oder Pyridyl steht, wobei Phenyl oder Pyridyl jeweils gegebenenfalls einfach oder zweifach, gleich oder verschieden substituiert sein kann, und wobei die Substituenten unabhängig voneinander ausgewählt sein können aus C₁-C₄-Alkyl, C₁-C₄-Halogenalkyl, Halogen oder Cyano, oder für Oxetanyl,
R¹¹, R¹² unabhängig voneinander für Wasserstoff oder für R¹³ stehen,
n für 0, 1 oder 2 steht.

5. Verbindungen der Formel (I) gemäß Anspruch 1, in welcher
A¹ für Stickstoff steht,
R¹ für Methyl, Ethyl, n-Propyl, i-Propyl oder cyclo-Propyl steht,
R² weiterhin für eine Gruppe ausgewählt aus G3 steht, wobei R¹¹ für Wasserstoff steht und R¹² für Methyl, Ethyl, iso-Propyl, Cyclopropyl oder tert-Butyl steht,
R³ für Wasserstoff steht,
X für ein heteroaromatisches 9-gliedriges oder 12-gliedriges annelliertes bicyclisches oder tricyclisches Ringsystem aus der Reihe H1, H2, H5, H15, H16, H17 oder H19 steht,
R⁵ für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl steht,
R⁶ für Wasserstoff steht,
R⁷ für Methyl, Ethyl, i-Propyl, Methoxymethyl oder Methoxyethyl steht,
n für 0, 1 oder 2 steht.

6. Verbindungen der Formel (I) gemäß Anspruch 1, in welcher
A¹ Stickstoff steht,
R¹ für Ethyl steht,
R² weiterhin für eine Gruppe ausgewählt aus G3 steht, wobei R¹¹ für Wasserstoff steht und R¹² für Methyl steht,
R³ für Wasserstoff steht,
X für ein heteroaromatisches 9-gliedriges oder 12-gliedriges annelliertes bicyclisches oder tricyclisches Ringsystem aus der Reihe H1, H2, H5, H15, H16, H17 oder H19 steht,
R⁵ für Trifluormethyl steht,
R⁶ für Wasserstoff steht,
R⁷ für Methyl steht,
n für 0, 1 oder 2 steht.

7. Verbindungen der Formel (I) gemäß Anspruch 1, in welcher
A¹, R¹, R³, R⁵, R⁶, R⁷, X und n die oben angegebenen Bedeutungen haben und
R² für einen Rest aus der folgender Reihe steht:

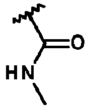

8. Verbindungen der Formel (I) gemäß Anspruch 1, in welcher
A¹, R¹, R², R³, R⁵, R⁶, R⁷ und n haben die oben angegebenen Bedeutungen haben und
X für einen Rest aus folgender Reihe steht:

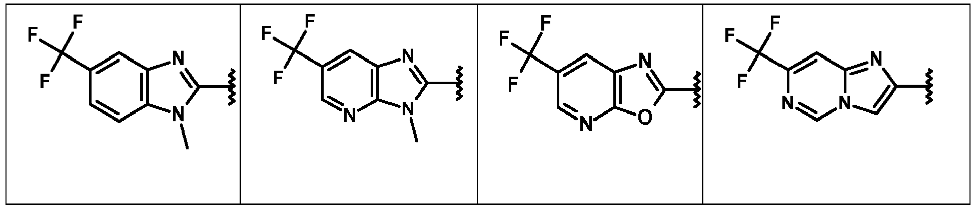

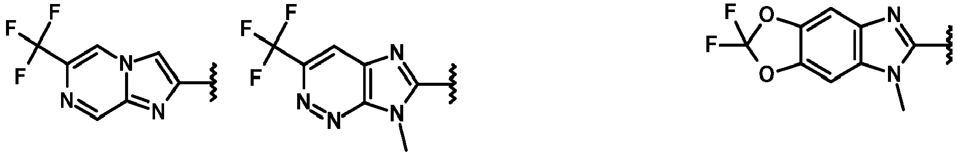

9. Verbindungen der Formel (I) gemäß Anspruch 1, wobei die Substituenten R¹, R², R³, A¹, X und n die in der folgenden Tabelle angegebenen Bedeutungen haben und wobei die Bindung von X und R² zum Restmolekül mit einer Wellenline gekennzeichnet ist:
| **Bsp.** | **R¹** | **n** | **A¹** | **R³** | **X** | **R²** |
|---|---|---|---|---|---|---|
| Bsp- I-34 | -CH₂CH₃ | 2 | N | H | 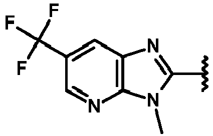 | 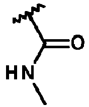 |
| Bsp. I-38 | -CH₂CH₃ | 2 | N | H | 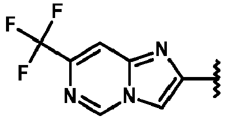 | 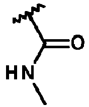 |
| Bsp. I-39 | -CH₂CH₃ | 2 | N | H | 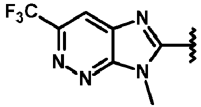 | 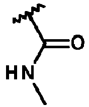 |

10. Agrochemische Formulierung enthaltend Verbindungen der Formel (I) gemäß Anspruch 1, sowie Streckmittel und/oder oberflächenaktive Substanzen.

11. Agrochemische Formulierung gemäß Anspruch 10 zusätzlich enthaltend einen weiteren agrochemischen Wirkstoff.

12. Verfahren zur Bekämpfung von tierischen Schädlingen **dadurch gekennzeichnet, dass** man eine Verbindung der Formel (I) gemäß Anspruch 1 oder eine agrochemische Formulierung gemäß einem der Ansprüche 10 oder 11 auf die tierischen Schädlinge und/oder ihren Lebensraum einwirken lässt.

13. Verwendung von Verbindungen der Formel (I) gemäß Anspruch 1 oder von agrochemischen Formulierungen gemäß einem der Ansprüche 10 oder 11 zur Bekämpfung von tierischen Schädlingen.

14. Verbindungen der Formel (LXXIX) 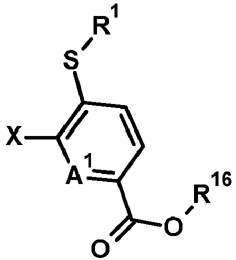 in welcher
X, A¹ und R¹ die oben beschriebenen Bedeutungen haben und R¹⁶ steht für Methyl, Ethyl oder Propyl.

15. Verbindungen der Formel (LXXX) 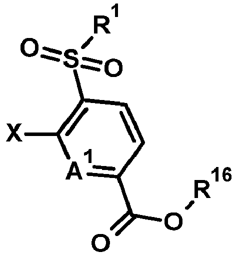 in welcher
X, A¹ und R¹ die oben beschriebenen Bedeutungen haben und R¹⁶ steht für Methyl, Ethyl oder Propyl.
